# EUROPÄISCHE PATENTANMELDUNG

(11) **EP 2 036 983 A1**
(43) Veröffentlichungstag der Anmeldung: **18.03.2009**
(21) Anmeldenummer: 07116174.9
(22) Anmeldetag: 12.09.2007
(51) Int. Cl.: C12N 15/82

(54) **Pflanzen, die erhöhte Mengen an Glucosaminglycanen synthetisieren**

(71) Anmelder: Bayer CropScience AG, 40789 Monheim (DE)
(72) Erfinder: Frohberg, Claus, 14532, Kleinmachnow (DE); Essigmann, Bernd, 12157, Berlin (DE)
(74) Vertreter: Quanz, Martin

(57) **Zusammenfassung**

Die vorliegende Erfindung betrifft Pflanzenzellen und Pflanzen, die eine erhöhte Menge an Glucosaminglycanen synthetisieren, sowie Verfahren zur Herstellung solcher Pflanzen, als auch Verfahren zur Herstellung von Glucosaminglycanen mit Hilfe dieser Pflanzenzellen oder Pflanzen. Erfindungsgemäße Pflanzenzellen oder genetisch modifizierte Pflanzen weisen dabei die Aktivität einer Glucosaminglycansynthase und zusätzlich eine erhöhte Aktivität einer Glucosamin-6-Phosphat-Acetyltransferase und eine erhöhte Aktivität einer UDP-N-acety-Glucosamin Pyrophosphorylase im Vergleich zu Wildtyp Pflanzenzellen bzw. Wildtyp Pflanzen auf. Weiterhin betrifft die vorliegende Erfindung Zusammensetzungen, enthaltend Pflanzenzellen mit gesteigerter Glucosaminglycansynthese.

## Beschreibung

Die vorliegende Erfindung betrifft Pflanzenzellen und Pflanzen, die eine erhöhte Menge an Glucosaminglycanen synthetisieren, sowie Verfahren zur Herstellung solcher Pflanzen, als auch Verfahren zur Herstellung von Glucosaminglycanen mit Hilfe dieser Pflanzenzellen oder Pflanzen. Erfindungsgemäße Pflanzenzellen oder genetisch modifizierte Pflanzen weisen dabei die Aktivität einer Glucosaminglycansynthase und zusätzlich eine erhöhte Aktivität einer Glucosamin-6-Phosphat-Acetyltransferase und eine erhöhte Aktivität einer UDP-N-acety-Glucosamin Pyrophosphorylase im Vergleich zu Wildtyp Pflanzenzellen bzw. Wildtyp Pflanzen auf. Weiterhin betrifft die vorliegende Erfindung Zusammensetzungen, enthaltend Pflanzenzellen mit gesteigerter Glucosaminglycansynthese.

Proteoglycane, eine Klasse von Glycoproteinen, sind u.a. ein wesentlicher Bestandteil des Knorpels und weisen an ein Kernprotein gebundene Glucosaminglycane, die sich aus repetitiven Disaccharideinheiten zusammensetzten, auf. Die repetitiven Disaccharideinheiten sind wiederum über eine charakteristische Kohlenhydratbindungssequenz an das Kernprotein kovalent gebunden. Je nach Zusammensetzung der Disaccharideinheiten unterscheidet man u.a. die Glucosaminglycane Heparan-/Heparinsulfat, Keratansulfat und Chondroitin-/Dermatansulfat, deren Disaccharideinheiten jeweils ein Molekül aufweisen, das Glucosamin oder ein Derivat des Glucosamins darstellt.
Hyaluronan, ein weiteres Glucosaminglycan weist ebenfalls ein Derivat des Glucosamins (N-acetyl-Glucosamin) als eines der Bestandteile seiner Disaccharideinheit auf, liegt jedoch natürlicherweise nicht an Proteine gebunden vor. Mit Ausnahme des Hyaluronans stellen die genannten Glucosaminglycane natürlicherweise sulfatierete Polymere dar. Bei diesen Substanzen sind Sulfatgruppen an verschiedenen Atomen oder Substituenten der Disaccharideinheiten eingeführt, so dass es sich bei den jeweils genannten Substanzen nicht um einheitliche Polymere, sondern um Polymergruppen handelt, die unter dem jeweiligen Oberbegriff zusammengefasst werden. Die einzelnen Moleküle der betreffenden Polymergruppen können sich dabei sowohl durch den Grad der Sulfatierung, als auch durch die Position der Monomere, die Sulfatgruppen aufweisen, unterscheiden.

Hyaluronan ist ein in der Natur vorkommendes, unverzweigtes, lineares Mucopolysaccharid (Glucosaminglycan), welches aus alternierenden Molekülen von Glucuronsäure (GlcA) und N-acetyl-Glucosamin (GlcNAc) zusammengesetzt ist. Der Grundbaustein von Hyaluronan besteht aus dem Disaccharid Glucuronsäure-beta-1,3-N-acetyl-Glucosamin. Diese Wiederholungseinheit ist im Hyaluronan durch beta-1,4-Verknüpfungen miteinander verbunden.
Im Bereich der Pharmazie wird häufig der Begriff Hyaluronsäure verwendet. Da Hyaluronan meist als Polyanion und nicht als freie Säure vorliegt, wird im Folgenden der Begriff Hyaluronan bevorzugt verwendet, wobei beide Molekülformen von der jeweiligen Bezeichnung als umfasst gelten.

Hyaluronan besitzt außergewöhnliche physico-chemische Eigenschaften, wie z.B. Eigenschaften von Polyelektrolyten, viskoelastische Eigenschaften, eine hohe Wasserbindekapazität, Eigenschaften der Gelbildung, die, neben weiteren Eigenschaften von Hyaluronan, in einem Übersichtsartikel von Lapcik et al. (1998, Chemical Reviews 98(8), 2663-2684) beschrieben sind.
Hyaluronan ist Bestandteil von extrazellulärem Bindegewebe und Körperflüssigkeiten von Vertebraten. Beim Menschen wird Hyaluronsäure von der Zellmembran aller Körperzellen, vor allem der mesenchymalen Zellen, synthetisiert und kommt ubiquitär im Körper vor, mit einer besonders hohen Konzentration in den Bindegeweben, der extrazellulären Matrix, der Nabelschnur, der Gelenkflüssigkeit, dem Knorpelgewebe, der Haut und dem Glaskörper des Auges (Bernhard Gebauer, 1998, Inaugural-Dissertation, Virchow-Klinikum Medizinische Fakultät Charité der Humboldt Universität zu Berlin; Fraser et al., 1997, Journal of Internal Medicine 242, 27-33). Kürzlich konnte Hyaluronan auch in tierischen nicht-Vertebraten Organismen (Mollusken) nachgewiesen werden (Volpi und Maccari, 2003, Biochimie 85, 619-625).
Weiterhin synthetisieren einige human pathogene gram-positive Bakterien (Gruppe A und C *Streptococcus*) und gram-negative Bakterien (*Pasteurella*) Hyaluronan als Exopolysaccharide, die diese Bakterien vor dem Zugriff des Immunsystems ihres Wirtes bewahren, da Hyaluronan eine nicht immunogene Substanz darstellt..
Viren, welche einzellige und teilweise als Endosymbionten in *Paramecium* Spezies vorkommende Grünalgen der Gattung *Chlorella* infizieren, vermitteln den einzelligen Grünalgen nach Virusinfektion die Fähigkeit, Hyaluronan zu synthetisieren (Graves et al., 1999, Virology 257, 15-23). Jedoch ist die Fähigkeit zur Hyaluronsynthese kein Merkmal, dass den betreffenden Algen zuzuordnen ist. Die Fähigkeit der Algen, Hyaluronan zu synthetisieren wird durch eine Infektion eines Virus, dessen Genom eine Hyaluronansynthase codierende Nucleinsäuresequenz aufweist, vermittelt (DeAngelis, 1997, Science 278, 1800-1803).

Die Katalyse der Hyaluronansynthese wird durch ein einziges, Membran integriertes oder Membran assoziiertes Enzym, der Hyaluronansynthase bewerkstelligt.
Der Mechanismus des Transfers von synthetisierten Hyaluronanmolekülen über die Cytoplasmamembran hinweg in das die Zelle umgebende Medium, ist noch nicht vollständig geklärt. Frühere Hypothesen, gingen davon aus, dass der Transport über die Zellmembran hinweg von der Hyaluronansynthase selbst bewerkstelligt würde. Neuere Ergebnisse deuten jedoch darauf hin, dass der Transport von Hyaluronanmolekülen über die Cytoplasmamembran durch einen energieabhängigen Transport mittels dafür zuständiger Transportproteine stattfindet. So wurden durch Mutagenese *Streptococcus* Stämme erzeugt, bei welchen die Synthese eines aktiven Transportproteins inhibiert war. Diese Stämme synthetisierten weniger Hyaluronan als entsprechende Wildtyp Bakterienstämme (Ouskova et al., 2004, Glycobiology 14(10), 931-938). In humanen Fibroblastenzellen konnte mit Hilfe von auf bekannte Transportproteine spezifisch inhibierend wirkenden Agentien gezeigt werden, dass sowohl die Menge an produziertem Hyaluronan, als auch die Aktivität von Hyaluronansynthasen reduziert werden kann (Prehm und Schumacher, 2004, Biochemical Pharmacology 68, 1401-1410). Ob und wenn überhaupt, in welcher Menge Transportproteine, die Hyaluronan transportieren können, in Pflanzen vorliegen, ist nicht bekannt.

Die außergewöhnlichen Eigenschaften des Hyaluronans eröffnen eine Vielfalt an Möglichkeiten für die Anwendung auf verschiedensten Gebieten, wie z.B. der Pharmazie, der Kosmetikindustrie, in der Lebens- und Futtermittel Herstellung, bei technischen Anwendungen (z.B. als Schmierstoffe) etc. Die wichtigsten Anwendungen, bei denen Hyaluronan zurzeit verwendet wird, liegen im medizinischen und kosmetischen Bereich (siehe z.B. Lapcik et al., 1998, Chemical Reviews 98(8), 2663-2684, Goa und Benfield, 1994, Drugs 47(3), 536-566).
Im medizinischen Bereich werden Hyaluronan enthaltende Produkte derzeit zur intraartikulären Arthrosebehandlung sowie bei den Ophthalmika, die bei Operationen am Auge zum Einsatz kommen, verwendet. Auch für die Behandlung von Gelenkerkrankungen bei Rennpferden wird Hyaluronan eingesetzt. Daneben ist Hyaluronan Bestandteil einiger Rhinologica, die z.B. in Form von Augentropfen und Nasalia der Befeuchtung trockener Schleimhäute dienen. Hyaluronan enthaltende Injektionslösungen werden als Analgetika und Antirheumatica verwendet. Hyaluronan oder derivatisiertes Hyaluronan enthaltende Auflagen werden bei der Wundheilung eingesetzt. Als Dermatika werden Hyaluronan enthaltende Gelimplantate zur Korrektur von Hautdeformationen in der plastischen Chirugie verwendet.
Für pharmakologische Anwendungen wird Hyaluronan mit einem hohen Molekulargewicht bevorzugt.
In der Schönheitsmedizin zählen Hyaluronanpräparate zu den geeigneten Hautfüllmaterialien. Durch Einspritzen von Hyaluronan erreicht man für eine begrenzte Zeit die Glättung von Falten oder ein vergrößertes Lippenvolumen.
In kosmetischen Produkten, insbesondere in Hautcremes und Lotionen findet Hyaluronan wegen seiner hohen Wasserbindungskapazität häufig Anwendung als Feuchtigkeitsspender.

Weiterhin werden Hyaluronan enthaltende Präparate als so genannte Nahrungsergänzungsmittel (Nutraceuticals) vertrieben, die auch bei Tieren (z.B. Hunde, Pferde) zur Vorbeugung und Linderung von Arthrose angewandt werden.

Für gewerbliche Zwecke verwendetes Hyaluronan wird zurzeit aus tierischen Geweben (Hahnenkämmen) isoliert oder fermentativ mittels Bakterienkulturen hergestellt.

Ein Verfahren zur Isolierung von Hyaluronan aus Hahnenkämmen oder alternativ aus Nabelschnüren ist z.B. beschrieben in US 4,141,973. Neben Hyaluronan kommen in tierischen Geweben (z.B. Hahnenkämme, Nabelschnüre) noch weitere, mit Hyaluronan verwandte Mucopolysaccharide, wie Chondrotinsulfat, Dermatansulfat, Keratansulfat, Heparansulfat und Heparin vor. Tierische Organismen weisen weiterhin Proteine (Hyaladherine) auf, die spezifisch an Hyaluronan binden und für verschiedenste Funktionen im Organismus, wie z.B. dem Abbau von Hyaluronan in der Leber, der Funktion von Hyaluronan als Leitstruktur für die Zellwanderung, die Regulierung der Endocytose, die Verankerung von Hyaluronan an der Zelloberfläche oder die Ausbildung von Hyaluronan Netzwerken notwendig sind (Turley, 1991, Adv Drug Delivery Rev 7, 257 ff.; Laurent und Fraser, 1992, FASEB J. 6, 183 ff.; Stamenkovic und Aruffo, 1993, Methods Enzymol. 245, 195 ff; Knudson und Knudson, 1993, FASEB 7, 1233 ff.).

Die zur bakteriellen Produktion von Hyaluronan verwendeten *Streptococcus* Stämme gehören ausschließlich zu den human pathogenen Bakterien. Diese Bakterien produzieren auch beim Kultivieren (pyrogene) Exotoxine und Haemolysine (Streptolysin, insbesondere alpha- und beta-Haemolysin; Kilian, M.: Streptococcus and Enterococcus. In: Medical Microbiology, Greenwood, D.; Slack, RCA; Peutherer, J.F. (Eds.). Kapitel 16. Churchill Livingstone, Edinburgh, UK: pp. 174-188, 2002, ISBN 0443070776), die ins Kulturmedium abgegeben werden. Dieses erschwert die Reinigung und Isolierung des Hyaluronans, welches mit Hilfe von *Streptococcus* Stämmen hergestellt wird. Besonders für phamazeutische Anwendungen stellt das Vorliegen von Exotoxinen und Haemolysinen in den Präparaten ein Problem dar.
US 4,801,539 beschreibt die Herstellung von Hyaluronan durch Fermentation eines mutagenisierten Bakterienstammes (*Streptococcus zooedemicus*). Der verwendete mutagenisierte Bakterienstamm synthetisiert kein beta-Haemolysin mehr. Es wurde dabei eine Ausbeute von 3,6 g Hyaluronan pro Liter Kultur erreicht.
EP 0694616 beschreibt ein Verfahren zur Kultivierung von *Streptococcus zooedemicus* oder *Streptococcus equi,* worin unter den verwendeten Kulturbedingungen kein Streptolysin, aber erhöhte Mengen an Hyaluronan synthetisiert werden. Es wurde dabei eine Ausbeute von 3,5 g Hyaluronan pro Liter Kultur erreicht.

*Streptococcus* Stämme geben während der Kultur das Enzym Hyaluronidase in das Kulturmedium ab, was dazu führt, dass auch bei diesem Produktionssystem das Molekulargewicht während der Aufreinigung reduziert wird. Die Verwendung von Hyaluronidase negativen *Streptococcus* Stämmen oder von Produktionsverfahren, bei welchen die Produktion von Hyaluronidase während der Kultur inhibiert wird, zur Produktion von Hyaluronan, sind in US 4,782,046 beschrieben. Es wurde dabei eine Ausbeute von bis zu 2,5 g Hyaluronan pro Liter Kultur erreicht, wobei ein maximales mittleres Molekulargewicht von 3,8x10⁶ Da bei einer Molekulargewichtsverteilung von 2,4x10⁶ bis 4,0x10⁶ erhalten wurde.
US 20030175902 und WO 03 054163 beschreiben die Herstellung von Hyaluronan mit Hilfe der heterologen Expression einer Hyaluronansynthase aus *Streptococcus equisimilis* in *Bacillus subtilis.* Um die Produktion von ausreichenden Mengen an Hyaluronan zu erlangen ist neben der heterologen Expression einer Hyaluronansynthase auch die gleichzeitige Expression einer UDP-Glucose-Dehydrogenase in den *Bacillus* Zellen notwendig. Die absolute Menge an Hyaluronan, die bei der Produktion mit Hilfe von *Bacillus subtilis* erhalten wird, ist in US 20030175902 und WO 03 054163 nicht angegeben. Es wurde ein maximales mittleres Molekulargewicht von ca. 4,2x10⁶ Da erhalten. Dieses mittlere Molekulargewicht wurde jedoch nur für den rekombinanten *Bacillus* Stamm erhalten, bei welchem ein Gen codierend das Hyaluronansynthase Gen aus *Streptococcus equisimilis* und das Gen codierend die UDP-Glucose-Dehydrogenase aus *Bacillus subtilis* unter Kontrolle des *amy*Q Promotors in das *Bacillus subtilis* Genom integriert war und gleichzeitig das *Bacillus subtilis* endogene *cxp*Y Gen (codierend eine P450 Cytochromoxidase) inaktiviert war. Chien und Lee (2007, Biotechnol. Prog. Online publication, ASAP Article 10.1021/bp070036w, S8756-7938(07)00036-7) beschreiben verschiedene rekombinante *Baciullus subtilis* Stämme. Ein Stamm, der mit einer Nucleinsäuresequenz codierend eine Hyaluronansynthase und einer Nucleinsäuresequenz codierend eine UDP-Glucose Dehydrogenase transformiert war, synthetisierte maximal 1,14 g/l Hyaluronan. Ein Stamm, der zusätzlich zu den gerade genannten Nucleinsäuresequenzen mit einer Nucleinsäuresequenz codierend *Vitreoscilla* Haemoglobin transformiert war, synthetisierte 1,8 g/l Hyaluronan.

WO 06 032538 beschreibt transgene Pflanzen, die mit Nucleinsäuremolekülen, codierend Hyaluronansynthasen transformiert wurden. Die Synthese von Hyaluronan in den betreffenden Pflanzen konnte eindeutig nachgewiesen werden.

WO 05 012529 beschreibt die Herstellung von transgenen Tabakpflanzen, die mit Nucleinsäuremolekülen, codierend Hyaluronansynthasen aus *Chlorella* infizierenden Viren transformiert wurden. In WO 05 012529 wurden zum einen Nucleinsäuresequenzen, codierend Hyaluronansynthase des Chlorellavirus CVH1-Stammes und zum anderen des Chlorellavirus CVKA1-Stammes für die Transformation von Tabakpflanzen verwendet. Die Synthese von Hyaluronan konnte nur für eine Pflanze, die nach Transformation mit einer Nucleinsäuresequenz, codierend eine Hyaluronansynthase, die aus dem Chlorellavirus CVKA1-Stamm isoliert wurde, nachgewiesen werden. Für Tabakpflanzen, die mit einer Nucleinsäuresequenz, codierend eine Hyaluronansynthase, die aus dem Chlorellavirus CVH1-Stamm isoliert wurde, konnte keine Hyaluronansynthese in den entsprechenden transgenen Pflanzen nachgewiesen werden. Die Menge an Hyaluronan, die von der einzigen in WO 05 012529 erhaltenen, Hyaluronan produzierenden transgenen Tabakpflanze synthetisiert wurde, ist mit ca. 4,2 µg Hyaluronan pro ml Messvolumen angegeben was unter Berücksichtigung der Beschreibung zur Durchführung des betreffenden Experiments etwa einer Menge von maximal 12 µg an produziertem Hyaluronan pro Gramm Frischgewicht an Pflanzenmaterial entspricht.

WO 2007 039314 beschreibt transgene Pflanzen, die die Aktivität einer Hyaluronansynthase und zusätzlich eine erhöhte Aktivität einer Glutamin:Fructose-6-Phosphat Amidotransferase (GFAT) aufweisen. Diese Pflanzen synthetisieren eine erhöhte Menge an Hyaluronan im Vergleich zu Pflanzen, die nur die Aktivität einer Hyaluronansynthase aufweisen. Die maximale Menge an Hyaluronan, die von diesen Tabakpflanzen synthetisiert wird, beträgt ca. 0,03% pro Gramm Frischgewicht an für die Messung eingesetztem Pflanzenmaterial (siehe FIG. 5 in WO 2007 039316).

WO 2007 039316 beschreibt transgene Pflanzen, die die Aktivität einer Hyaluronansynthase und zusätzlich eine erhöhte Aktivität einer Glutamin:Fructose-6-Phosphat Amidotransferase (GFAT) und eine erhöhte Aktivität einer UDP-Glucose Dehydrogenase (UDP-Glc-DH) im Vergleich zu Wildtyp Pflanzen aufweisen. Diese Pflanzen synthetisieren eine erhöhte Menge an Hyaluronan im Vergleich zu Pflanzen, die die Aktivität einer Hyaluronansynthase und gleichzeitig die Aktivität eines Proteins mit der Aktivität einer GFAT aufweisen. Die maximale Menge an Hyaluronan, die von diesen Tabakpflanzen synthetisiert wird, beträgt 0,2% pro Gramm Frischgewicht an für die Messung eingesetztem Pflanzenmaterial (siehe FIG. 6 in WO 2007 039316).

Weiterhin enthält WO 2007 039316 eine Auflistung von Proteinen, die in Pflanzenzellen exprimiert werden könnten, um die Menge an synthetisiertem Hyaluronan in Pflanzenzellen weiter zu erhöhen. Bei den in WO 2007 039316 vorgeschlagenen Proteinen handelt es sich um eine beliebige Liste an Enzymen, die in unterschiedlichsten Organismen an der Synthese von UDP-GlcNAc beteiligt sind Die in WO 2007 039316 aufgelisteten Proteine weisen unterschiedliche enzymatische Funktionen auf. WO 2007 039316 gibt keinen Hinweis darauf, ob und welche der aufgelisteten Enzyme bei deren Expression in transgenen Pflanzen tatsächlich eine Steigerung des Hyaluronangehaltes bewirken könnten.

Die Synthese der Disaccharidkette des Chondroitins/Dermatans ([beta-1,4)]-[Glucuronsäure-beta-1,4-N-acetyl-Galactosamin]ₙ) wird von einer Chondroitinsynthase ausgehend, von UDP-Glucuronsäure (UDP-GlcA) und UDP-N-acetyl-Galactosamin (UDP-GalNAc), einem Epimer des UDP-N-acetyl-Glucosamins (UDP-GlcNAc), katalyisiert (Kitagawa et al., 2001, J Biol Chem 276(42), 38721-38726). Die Glucuronsäuremoleküle des Chondroitins können durch eine Epimerase in Iduronsäure umgewandelt werden. Liegen mehr als 10% der Glucuronsäuremoleküle als Iduronsäure vor, so bezeichnet man das Polymer als Dermatan. Die Einführung der Sulfatgruppen an unterschiedlichen Positionen der Disaccharidkette des Chondroitins bzw. des Dermatans wird anschließend von weiteren Enzymen katalysiert, wobei Chondroitin-/Dermatansulfat entsteht. Der Grad der Sulfatierung kann sich dabei von Molekül zu Molekül unterscheiden.

Chondroitinsulfat, wird seit einiger Zeit als potentieller Wirkstoff zur Behandlung von Osteoarthritis in Erwägung gezogen (Clegg et al., 2006, The New England Journal of Medicine 354(8), 795-808).

Die Synthese der Disaccharidkette des Heparins/Heparans (Heparosans) ([alpha-1,4]-[Glucuronsäure-beta-1,4-Glucosamin]ₙ oder [alpha-1,4]-[Iduronsäure-alpha-1,4-Glucosamin]ₙ) wird von einer Heparin-/Heparosansynthase ausgehend von UDP-GlcA und UDP-GlcNAc katalysiert (DeAngelis und White, 2004, J. Bacteriology 186(24), 8529-8532). Die Glucuronsäuremoleküle des Heparosans können durch eine Epimerase in Iduronsäure umgewandelt werden. Die Einführung der Sulfatgruppen an unterschiedlichen Positionen der Disaccharidkette des Heparosans wird anschließend von weiteren Enzymen katalysiert, wobei Heparin- oder Heparansulfat entsteht. Heparinsulfat weißt eine deutlich höhere Substitution mit Sulfatgruppen auf, als Heparansulfat. Heparinsulfat weist etwa 90% Iduronsäuremoleküle auf, während beim Heparansulfat der Anteil an Glucuronsäuremolekülen überwiegt (Gallagher et al., 1992, Int. J. Biochem 24, 553-560). Wie beim Chondroitin-/Dermatansulfat kann auch beim Heparin-/Heparansulfat der Grad der Sulfatierung von Molekül zu Molekül unterschiedlich sein.
Heparinsulfat wird u.a. als Antikoagulans z.B. zur Vorbeugung und Behandlung von Thrombosen eingesetzt.

Chondroitin-/Dermatansulfat und Heparin-/Heparansulfat werden zurzeit mittels Isolierung aus tierischen Geweben hergestellt. Chondroitinsulfat wird überwiegend aus Rinder- oder Haifischknorpel, Heparin-/Heparansulfat aus Schweinedärmen oder Rinderlungen isoliert. Da die Disaccharidketten von Chondroitin-/Dermatansulfat bzw. Heparin-/Heparansulfat kein einheitliches Sulfatierungsmuster aufweisen, ist es schwierig, ein einheitliches, spezifisches Produkt zu erhalten. Es handelt sich daher immer um eine Mischung aus unterschiedlich stark sulfatierten Molekülen.

Wie bereits beschrieben, werden Glucosaminglycane, wie z.B. Chondroitinsulfat oder Heparin-/Heparansulfat zurzeit aus tierischen Geweben isoliert. Diese Gewebe enthalten neben den jeweils gewünschten Substanzen, noch weitere Glucosaminglycane. Die Trennung der einzelnen Glucosaminglycane ist dabei, wenn überhaupt vollständig möglich, schwierig und aufwendig. Weiterhin stellt die potentielle Belastung von tierischen Geweben mit pathogenen Mikroorganismen und/oder von anderen Substanzen, wie z.B. dem BSE oder dem Vogelgrippe Erreger, die beim Menschen Krankheiten hervorrufen können, ein Problem für die Verwendung von aus tierischem Gewebe isolierten Glucosaminglycanen dar. Die Verwendung von medizinischen Präparaten, die mit tierischen Proteinen verunreinigt sind, kann bei Patienten zu unerwünschten immunologischen Reaktionen des Körpers führen (siehe für Hyaluronanpräparate z.B. US 4,141,973), insbesondere dann, wenn der Patient eine Allergie gegen tierische Proteine aufweist.
Weiterhin sind Substanzen, die aus tierischen Rohstoffen hergestellt wurden für einige Lebensweisen, wie z.B. dem Veganismus oder für die koschere Speisezubereitung, nicht akzeptabel.
Ein weiteres Problem bei der Isolierung von Glucosaminglycanen aus tierischen Geweben besteht darin, dass das Molekulargewicht der Glucosaminglycane häufig während der Aufreinigung verringert wird, weil tierische Gewebe auch Glucosaminglycan abbauende Enzyme aufweisen.
Die Mengen (Ausbeuten) an Glucosaminglycanen, die aus tierischen Geweben in ausreichender Qualität und Reinheit gewonnen werden können, sind gering (z.B. Hyaluronan aus Hahnenkamm: 0,079% w/w, EP 0144019, US 4,782,046), was dazu führt, dass große Mengen an tierischen Geweben verarbeitet werden müssen.
Die Produktion von Glucosaminglycanen mit Hilfe der Fermentation von Bakterien ist mit hohen Kosten verbunden, da die Bakterien in geschlossenen, sterilen Behältnissen, unter aufwendigen, kontrollierten Kulturbedingungen (siehe z.B. für Hyaluronan US 4,897,349) fermentiert werden müssen. Weiterhin ist die Menge an Glucosaminglycanen, die mittels Fermentation von Bakterien Stämmen produziert werden kann, beschränkt durch die jeweils bestehenden Produktionsanlagen. Dabei ist auch zu berücksichtigen, dass Fermenter wegen physikalischer Gesetzmäßigkeiten nicht für übermäßig große Kulturvolumen gebaut werden können. Insbesondere ist hier die für eine effiziente Produktion gleichmäßige Durchmischung von durch von außen zugeführten Substanzen (z.B. essentielle Nährstoffquellen für Bakterien, Reagenzien zur pH Regulierung, Sauerstoff) mit dem Kulturmedium zu erwähnen, die in großen Fermentern, wenn überhaupt, nur mit großem technischen Aufwand gewährleistet werden kann.

Pflanzen produzieren Glucosaminglycane wie z.B. Hyaluronan, Heparan-/Heparinsulfat, Keratansulfat oder Chondroitin-/Dermatansulfat natürlicherweise nicht. Natürlich vorkommende Pflanzen selbst weisen in ihrem Genom keine Nucleinsäuren auf, die Proteine codieren, welche die Synthese von Glucosaminglycanen katalysieren und, obwohl eine Vielzahl an pflanzlichen Kohlenhydraten beschrieben und charakterisiert wurden, konnten bisher keine der genannten Glucosaminglycane in nicht infizierten, natürlich vorkommenden Pflanzenzellen nachgewiesen werden (Graves et al., 1999, Virology 257, 15-23).

WO 98 35047 (US 6,444,878) beschreibt für die Synthese von GlcNAc in Pflanzenzellen einen Stoffwechselweg, wobei Glucosamin, welches durch mehrere aufeinander folgende enzymatisch katalysierte Reaktionsschritte unter Bildung der Metabolite GlcNAc, N-acetyl-Glucosamin-6-Phosphat, N-acetyl-Glucosamin-1-Phosphat zu UDP-GlcNAc umgesetzt wird. Glucosamin-6-Phosphat ist in höheren Konzentrationen für Pflanzenzellen toxisch (WO 98 35047).
Ein für Pflanzen alternativ beschriebener Stoffwechselweg beinhaltet die Umsetzung von Fructose-6-Phosphat und Glutamin zu Glucosamin-6-Phosphat, welches anschließend durch mehrere aufeinander folgende enzymatisch katalysierte Reaktionsschritte unter Bildung der Metabolite Glucosamin-1-Phosphat und N-acetyl-Glucosamin-1-Phosphat zu UDP-GlcNAc umgesetzt wird (Mayer et al., 1968, Plant Physiol. 43, 1097-1107).

Es ist zurzeit weiterhin nicht eindeutig geklärt, welche Proteinaktivitäten im pflanzlichen Stoffwechselweg zur Synthese von UDP-GlcNAc verändert werden müssen, damit Pflanzen erhöhte Mengen an Glucosaminglycanen synthetisieren.

Der vorliegenden Erfindung liegt somit die Aufgabe zu Grunde, alternative Mittel und Verfahren für die Herstellung von effizienten Mengen an Glucosaminglycanen in Pflanzen zur Verfügung zu stellen.

Diese Aufgabe wird durch die in den Ansprüchen bezeichneten Ausführungsformen gelöst.

Es wurde überraschenderweise gefunden, dass genetisch modifizierte Pflanzenzellen oder genetisch modifizierte Pflanzen, die ein Nucleinsäuremolekül, codierend eine Glucosaminglycansynthase aufweisen und die zusätzlich ein fremdes Nucleinsäuremolekül, codierend ein Protein mit der Aktivität einer Glucosamin-6-Phosphat-Acetyltransferase und ein fremdes Nucleinsäuremolekül, codierend ein Protein mit der Aktivität einer monofunktionalen UDP-N-acetyl-Glucosamin Pyrophosphorylase aufweisen, signifikant höhere Mengen an Glucosaminglycan produzieren, als Pflanzenzellen oder Pflanzen, die (nur) die Aktivität einer Glucosaminglycansynthase aufweisen.

Somit betrifft die vorliegende Erfindung genetisch modifizierte Pflanzenzellen oder genetisch modifizierte Pflanzen, die ein fremdes Nucleinsäuremolekül, codierend eine Glucosaminglycansynthase aufweisen, dadurch gekennzeichnet, dass besagte genetisch modifizierte Pflanzenzellen oder genetisch modifizierte Pflanzen zusätzlich ein fremdes Nucleinsäuremolekül, codierend ein Protein mit der Aktivität einer Glucosamin-6-Phosphat-Acetyltransferase und ein fremdes Nucleinsäuremolekül, codierend ein Protein mit der Aktivität einer monofunktionalen UDP-N-acetyl-Glucosamin Pyrophosphorylase aufweisen.

Die Produktion von Glucosaminglycanen mit Hilfe der Fermentation von Bakterien Stämmen ist mit hohen Kosten verbunden, da die Bakterien in geschlossenen, sterilen Behältnissen, unter aufwendigen, kontrollierten Kulturbedingungen (siehe z.B. US 4,897,349) fermentiert werden müssen. Weiterhin ist die Menge an Glucosaminglycanen, die mittels Fermentation von Bakterien Stämmen produziert werden kann, beschränkt durch die jeweils bestehenden Produktionsanlagen. Der hohe Preis von z.B. derzeitig käuflich erwerbbaren Hyaluronan hat zur Folge, dass dieses Glucosaminglycan trotz seiner besonderen Eigenschaften (z.B. Viskoelatizität, hohe Wasserbindungskapazität) nicht für technische Anwendungen zur Verfügung steht.
Erfindungsgemäße Pflanzenzellen und erfindungsgemäße Pflanzen bieten daher gegenüber bekannten Mitteln zur Herstellung von Glucosaminglycanen den Vorteil, dass sie erhöhte Mengen an Glucosaminglycanen (z.B. Hyaluronan) synthetisieren, im Vergleich zu Pflanzenzellen, oder Pflanzen, die nur die Aktivität einer Glucosaminglycansynthase aufweisen.

Die genetische Modifikation erfindungsgemäßer genetisch modifizierter Pflanzenzellen oder erfindungsgemäßer genetisch modifizierter Pflanzen kann dabei jede genetische Modifikation sein, die eine Integration eines fremden Nucleinsäuremoleküls, codierend eine Glucosaminsynthase und eine Integration eines fremden Nucleinsäuremoleküls, codierend ein Protein mit der Aktivität einer Glucosamin-6-Phosphat-Acetyltransferase und eine Integration eines fremden Nucleinsäuremoleküls, codierend ein Protein mit der Aktivität einer monofunktionalen UDP-N-acetyl-Glucosamin Pyrophosphorylase in eine Pflanzenzelle oder eine Pflanze bewirkt.

Unter dem Begriff "Glucosaminglycansynthase" soll im Zusammenhang mit der vorliegenden Erfindung ein Protein verstanden werden, das ausgehend von den Substraten UDP-Glucuronsäure (UDP-GlcA) und UDP-N-acetyl-Aldohexsosamin (UDP-AldohexNAc) Glucosaminglycan synthetisiert. Die Katalyse der Glucosaminglycane folgt dabei folgendem allgemeinen Reaktionsschema:

nUDP-GlcA + nUDP-AldohexNAc → [GlcA-GlcNAc]ₙ + 2 nUDP

Bevorzugte handelt es sich bei dem in obiger Reaktionsgleichung dargestellten UDP-N-acetyl-Aldohexsosamin um UDP-N-acetyl-Glucosamin oder UDP-N-acetyl-Galactosamin.

In einer bevorzugten Ausführungsform betrifft die vorliegende Erfindung erfindungsgemäße Pflanzenzellen oder erfindungsgemäße Pflanzen, wobei das fremde Nucleinsäuremolekül codierend eine Glucosaminglycansynthase eine Hyaluronansynthase, oder eine Chondroitinsynthase oder eine Heparin-/Heparosansynthase codiert.

Unter dem Begriff "Hyaluronansynthase" (EC 2.4.1.212) soll im Zusammenhang mit der vorliegenden Erfindung ein Protein verstanden werden, das ausgehend von den Substraten UDP-Glucuronsäure (UDP-GlcA) und UDP-N-acetyl-Glucosamin (UDP-GlcNAc) Hyaluronan synthetisiert. Die Katalyse der Hyaluronansynthese folgt dabei folgendem Reaktionsschema:

nUDP-GlcA + nUDP-GlcNAc → beta-1,4-[GlcA-beta-1,3-GlcNAc]ₙ + 2 nUDP

Die bisher untersuchten Hyaluronansynthasen können in zwei Gruppen eingeteilt werden: Hyaluronansynthasen der Klasse I und Hyaluronansynthasen der Klasse II (DeAngelis, 1999, CMLS, Cellular and Molecular Life Sciences 56, 670-682). Eine weitere Unterscheidung der Hyaluronansynthasen aus Vertebraten erfolgt an Hand der identifizierten Isoenzyme. Die verschiedenen Isoenzyme werden in der Reihenfolge ihrer Identifizierung mit arabischen Nummern bezeichnet (z.B. hsHAS1, hsHAS2, hsHAS3).

Nucleinsäuremoleküle und korrespondierende Proteinsequenzen, codierend Hyaluronansynthasen sind u.a. für folgende Organismen beschrieben: Kaninchen (*Oryctolagus cuniculus*) ocHas2 (EMBL AB055978.1, US 20030235893), ocHas3 (EMBL AB055979.1, US 20030235893); Pavian (*Papio anubis*) paHas1 (EMBL AY463695.1); Frosch (*Xenopus laevis*) xlHas1 (EMBL M22249.1, US 20030235893), xlHas2 (DG42) (EMBL AF168465.1), xlHas3 (EMBL AY302252.1); Mensch (*Homo sapiens*) hsHAS1 (EMBL D84424.1, US 20030235893), hsHAS2 (EMBL U54804.1, US 20030235893), hsHAS3 (EMBL AF232772.1, US 20030235893); Maus (*Mus musculus*), mmHas1 (EMBL D82964.1, US 20030235893), mmHAS2 (EMBL U52524.2, US 20030235893), mmHas3 (EMBL U86408.2, US 20030235893); Rind (*Bos taurus*) btHas2 (EMBL AJ004951.1, US 20030235893); Huhn (*Gallus gallus*) ggHas2 (EMBL AF106940.1, US 20030235893); Ratte (*Rattus norvegicus*) rnHas 1 (EMBL AB097568.1, Itano et al., 2004, J. Biol. Chem. 279(18) 18679-18678), rnHas2 (EMBL AF008201.1); rnHas 3 (NCBI NM_172319.1, Itano et al., 2004, J. Biol. Chem. 279(18) 18679-18678), Pferd (*Equus caballus*) ecHAS2 (EMBL AY056582.1, GI:23428486), Schwein (*Sus scrofa*) sscHAS2 (NCBI NM_214053.1, GI:47522921), sscHas 3 (EMBLAB159675), Zebrafisch (*Danio rerio*) brHas1 (EMBL AY437407), brHas2 (EMBL AF190742.1) brHas3 (EMBL AF190743.1); *Pasteurella multocida* pmHas (EMBL AF036004.2); *Streptococcus pyogenes* spHas (EMBL, L20853.1, L21187.1, US 6,455,304, US 20030235893); *Streptococcus equis* seHas (EMBL AF347022.1, AY173078.1), *Streptococcus uberis* suHasA (EMBL AJ242946.2, US 20030235893), *Streptococcus equisimilis* seqHas (EMBL AF023876.1, US 20030235893); *Sulfolobus solfataricus* ssHAS (US 20030235893), *Sulfolobus tokodaii* stHas (AP000988.1), *Paramecium bursaria Chlorella* Virus 1, cvHAS (EMBL U42580.3, PB42580, US 20030235893).

In einer vorzugsweisen Ausführungsform betrifft die vorliegende Erfindung erfindungsgemäße genetisch modifizierte Pflanzenzellen oder erfindungsgemäße genetisch modifizierte Pflanzen, wobei das Glucosaminglycansynthase codierende fremde Nucleinsäuremolekül dadurch gekennzeichnet ist, dass eine Hyaluronansynthase codiert. Betreffend ein fremdes Nucleinsäuremolekül codierend eine Hyaluronansynthase handelt es sich vorzugsweise um ein fremdes Nucleinsäuremolekül, codierend eine virale Hyaluronansynthase. Bevorzugt codiert das Hyaluronansynthase codierende fremde Nucleinsäuremolekül eine Hyaluronansynthase eines Virus, der Algen infiziert.
Bezüglich eines Virus, der Algen infiziert, codiert das Hyaluronansynthase codierende fremde Nucleinsäuremolekül vorzugsweise eine Hyaluronansynthase eines *Chlorella* infizierenden Virus, besonders bevorzugt eine Hyaluronansynthase eines *Paramecium bursaria Chlorella* Virus 1 und insbesondere bevorzugt eine Hyaluronansynthase eines *Paramecium bursaria Chlorella* Virus eines H1 Stammes.
Bevorzugt ist das Hyaluronansynthase codierende fremde Nucleinsäuremolekül dadurch gekennzeichnet, dass es eine Hyaluronansynthase codiert, dessen Aminosäuresequenz mit der unter SEQ ID NO 2 dargestellten Aminosäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% aufweist. In einer besonders bevorzugten Ausführungsform ist das Hyaluronansynthase codierende fremde Nucleinsäuremolekül dadurch gekennzeichnet, dass es eine Hyaluronansynthase codiert, die die unter SEQ ID No 2 dargestellte Aminosäuresequenz aufweist.
In einer weiteren Ausführungsform weist das fremde Nucleinsäuremolekül, codierend eine Hyaluronansynthase mit der unter SEQ ID NO 1 oder SEQ ID NO 3 dargestellten Nucleinsäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% auf. In einer besonders bevorzugten Ausführungsform ist das Hyaluronansynthase codierende fremde Nucleinsäuremolekül dadurch gekennzeichnet, dass es die unter SEQ ID No 3 dargestellte Nucleinsäuresequenz aufweist oder dass die Sequenz des fremden Nucleinsäuremoleküls von der unter SEQ ID No 1 oder 3 dargestellten Nucleinsäuresequenz aufgrund der Degeneration des genetisches Codes abweicht.

Das Plasmid IC 341-222, enthaltend ein synthetisches Nucleinsäuremolekül, das eine *Paramecium bursaria Chlorella* Virus Hyaluronansynthase codiert, wurde am 25.08.2004 unter der Nummer DSM16664 nach dem Budapester Vertrag hinterlegt bei der Deutschen Sammlung von Mikroorganismen und Zellkulturen GmbH, Mascheroder Weg 1b, 38124 Braunschweig, Deutschland. Die in SEQ ID NO 2 dargestellte Aminosäuresequenz kann von der codierenden Region der in Plasmid IC 341-222 integrierten Nucleinsäuresequenz abgeleitet werden und codiert für eine *Paramecium bursaria Chlorella* Virus Hyaluronansynthase.

Die vorliegende Erfindung betrifft daher auch erfindungsgemäße genetisch modifizierte Pflanzenzellen oder erfindungsgemäße genetisch modifizierte Pflanzen, wobei das Hyaluronansynthase codierende Nucleinsäuremolekül dadurch gekennzeichnet ist, dass es ein Protein codiert, dessen Aminosäuresequenz von der codierenden Region der im Plasmid DSM16664 inserierten Nucleinsäuresequenz abgeleitet werden kann oder dass es ein Protein codiert, dessen Aminosäuresequenz mit der Aminosäuresequenz, die von der codierenden Region der im Plasmid DSM16664 inserierten Nucleinsäuresequenz abgeleitet werden kann, eine Identität von mindestens 70%, vorzugsweise von mindestens 80%, bevorzugt von mindestens 90%, insbesondere bevorzugt von mindestens 95% und im speziellen bevorzugt von mindestens 98% aufweist.
Die vorliegende Erfindung betrifft auch erfindungsgemäße genetisch modifizierte Pflanzenzellen oder erfindungsgemäße genetisch modifizierte Pflanzen, wobei das Hyaluronansynthase codierende fremde Nucleinsäuremolekül dadurch gekennzeichnet ist, dass es die in Plasmid DSM16664 integrierte, Hyaluronansynthase codierende Nucleinsäuresequenz darstellt oder dass sie mit der in Plasmid DSM16664 integrierten Nucleinsäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% aufweist.

Unter dem Begriff "Chondroitinsynthase" (EC 2.4.1.175, EC 1.4.1.226) soll im Zusammenhang mit der vorliegenden Erfindung ein Protein oder ein Proteinkomplex, bestehend aus zwei Proteinen verstanden werden, der ausgehend von den Substraten UDP-Glucuronsäure (UDP-GlcA) und UDP-N-acetyl-Glalactosamin (UDP-GaINAc) Chondroitin synthetisiert. Die Katalyse der Chondroitinsynthese folgt dabei folgendem Reaktionsschema:

nUDP-GlcA + nUDP-GalNAc → beta-1,4-[GlcA-beta-1,3-GalNAc]ₙ + 2 nUDP

Die Verlängerung des an ein Proteoglycan gebundenen Chondroitinmoleküls wird in einigen Organismen durch einen Chondroitinsynthase-Enzymkomplex katalysiert, der aus zwei verschiedenen Proteinen besteht. Eines der beiden Proteine, N-Acetylgalactosaminyltransferase II (EC 2.4.1.175), fügt dabei N-acetyl-Galactosamin-Monomere mittels einer beta-1,4-Verknüpfung, das zweite Protein, N-Acetylgalactosaminyl-Proteoglycan 3-beta-Glucuronosyltransferase (EC 2.4.1.226) fügt Glucuronat Monomere mittels einer beta-1,3-Verknüpfung an das Chondroitinmolekül an. Dem Fachmann sind jedoch auch bifunktionale Proteine bekannt, wobei ein einziges Protein sowohl N-acetyl-Galactosamin-Monomere, als auch Glucuronat Monomere an das Chondroitinmolekül anfügt.

In einer weiteren vorzugsweisen Ausführungsform betrifft die vorliegende Erfindung erfindungsgemäße genetisch modifizierte Pflanzenzellen oder erfindungsgemäße genetisch modifizierte Pflanzen, wobei das Glucosaminglycansynthase codierende fremde Nucleinsäuremolekül dadurch gekennzeichnet ist, dass es eine Chondroitinsynthase codiert.
Eine bevorzugte Ausführungsform der vorliegenden Erfindung betrifft erfindungsgemäße Pflanzenzellen oder erfindungsgemäße Pflanzen, wobei das fremde Nucleinsäuremolekül, codierend eine Chondroitinsynthase eine bifunktionale Chondroitinsynthase codiert, die sowohl N-acetyl-Galactosamin-Monomere, als auch Glucuronat-Monomere an das Chondroitinmolekül anfügt.

Unter dem Begriff "bifunktionale Chondroitinsynthase" soll im Zusammenhang mit der vorliegenden Erfindung ein Protein verstanden werden, bei dem die Aktivität einer N-Acetylgalactosaminyltransferase II (EC 2.4.1.175) und die Aktivität einer Acetylgalactosaminyl-Proteoglycan 3-beta-Glucuronosyltransferase (EC 2.4.1.226) auf einem Molekül vorliegt.

Nucleinsäuremoleküle und davon abgeleitete Aminosäuresequenzen, codierend monofunktionale Chondroitinsynthasen sind z.B. aus Bakterien (z.B. *Escherichia coli,* US2003109693, EP 1283259) beschrieben.
Nucleinsäuremoleküle und davon abgeleitete Aminosäuresequenzen, codierend bifunktionale Chondroitinsynthasen sind z.B. aus Säugetieren (z.B. *Homo sapiens,* WO 03 012099, US 2005048604, US 2006052335, NCBI acc. No: BC046247.1, BC023531.2; Kitagawa et al., 2001, J. Biol. Chem. 276(42), 38721-38726) oder *Pasteurella multicoda* (US 2003104601, EMBL acc. No: AF195517, DeAngelis und Padgett-McCue, 2000, J. Biol. Chem. 275(31), 24124-24129) beschrieben.

Betreffend ein fremdes Nucleinsäuremolekül codierend eine Chondroitinsynthase handelt es sich vorzugsweise um ein fremdes Nucleinsäuremolekül, codierend eine bakterielle Chondroitinsynthase, bevorzugt codierend eine Chondroitinsynthase aus *Pasteurella,* besonders bevorzugt codierend eine Chondroitinsynthase aus *Pasteurella multocida.*
Bevorzugt ist das Chondroitinsynthase codierende fremde Nucleinsäuremolekül dadurch gekennzeichnet, dass es eine Chondroitinsynthase codiert, dessen Aminosäuresequenz mit der unter SEQ ID NO 5 dargestellten Aminosäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% aufweist. In einer besonders bevorzugten Ausführungsform ist das Chondroitinsynthase codierende fremde Nucleinsäuremolekül dadurch gekennzeichnet, dass es eine Chondroitinsynthase codiert, die die unter SEQ ID No 5 dargestellte Aminosäuresequenz aufweist.

In einer weiteren Ausführungsform weist das fremde Nucleinsäuremolekül, codierend eine Chondroitinsynthase mit der unter SEQ ID NO 4 dargestellten Nucleinsäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% auf. In einer besonders bevorzugten Ausführungsform ist das Chondroitinsynthase codierende Nucleinsäuremolekül dadurch gekennzeichnet, dass es die unter SEQ ID No 4 dargestellte Nucleinsäuresequenz aufweist oder dass die Sequenz des fremden Nucleinsäuremoleküls von der unter SEQ ID No 4 dargestellten Nucleinsäuresequenz aufgrund der Degeneration des genetisches Codes abweicht.

Unter dem Begriff "Heparin-/Heparosansynthase" oder "Heparosansynthase" (EC 2.4.1.224, EC 2.4.1.225) soll im Zusammenhang mit der vorliegenden Erfindung ein Protein oder ein Enzymkomplex, bestehend aus zwei Proteinen verstanden werden, der ausgehend von den Substraten UDP-Glucuronsäure (UDP-GlcA) und UDP-N-acetyl-Glucosamin (UDP-GlcNAc) Heparin/Heparan synthetisiert. Die Katalyse der Heparin-/Heparansynthese folgt dabei folgendem Reaktionsschema:

nUDP-GlcA + nUDP-GlcNAc → alpha-1,4-[GlcA-beta-1,4-GlcNAc]ₙ + 2 nUDP

Nucleinsäuremoleküle und davon abgeleitete Aminosäuresequenzen, codierend eine Heparin-/Heparosansynthase sind z.B. aus Bakterien (*Pasteurella multocida* EMBL acc. Nos: AF425591, AF439804, AY292199, AY292200, US 20030099967, *Escherichia coli* EMBL acc. No: X77617.1) oder Mensch (NCBI acc. Nos: BC001174.1, NM_207122.1) beschrieben

Die Verlängerung des an ein Proteoglycan gebundenen Heparin-/Heparosanmoleküls wird in einigen Organismen durch einen Heparin-/Heparosansynthase-Enzymkomplex katalysiert, der aus zwei verschiedenen Proteinen besteht. Eines der beiden Proteine, Glucuronosyl-N-Aceytlglucosaminyl-Proteoglycan 4-alpha-N-Glucosaminyltransferase (EC 2.4.1.224), fügt dabei N-acetyl-Glucosamin-Monomere mittels einer beta-1,4-Verknüpfung, das zweite Protein, N-acetylglucosaminyl-Proteoglycan 4-beta-Glucoronosyltransferase (EC 2.4.1.225) fügt Glucuronat-Monomere mittels einer beta-1,3-Verknüpfung an das Heparin-/Heparanmolekül an. Dem Fachmann sind jedoch auch bifunktionale Proteine bekannt, wobei ein einziges Protein sowohl N-acetyl-Glucosamin-Monomere, als auch Glucuronat Monomere an das Heparin-/Heparosanmolekül anfügt. Solche bifunktionalen Heparin-/Heparosansynthasen sind z.B. aus Mensch (Busse und Kusche-Gullberg, 2003, J. Biol. Chem. 278(42), 41333-41337) oder aus *Pasteurella* (DeAngelis und White, 2004, J. Bacteriology 186(24), 8529-8532) beschrieben. Bifunktionale Proteine mit der Aktivität einer Heparin-/Heparosansynthase weisen sowohl die Aktivität eines unter EC Nummer 2.4.1.224, als auch die Aktivität eines unter EC Nummer 2.4.1.225 klassifizierten Enzyms auf.

In einer weiteren vorzugsweisen Ausführungsform betrifft die vorliegende Erfindung erfindungsgemäße genetisch modifizierte Pflanzenzellen oder erfindungsgemäße genetisch modifizierte Pflanzen, wobei das Glucosaminglycansynthase codierende fremde Nucleinsäuremolekül dadurch gekennzeichnet ist, dass es eine Heparin-/Heparosansynthase codiert.
Eine bevorzugte Ausführungsform der vorliegenden Erfindung betrifft erfindungsgemäße Pflanzenzellen oder erfindungsgemäße Pflanzen, wobei das fremde Nucleinsäuremolekül, codierend eine Heparin-/Heparosansynthase eine bifunktionale Heparin-/Heparansynthase codiert, die sowohl N-acetyl-Glucosamin-Monomere, als auch Glucuronat-Monomere an das Heparin-/Heparanmolekül anfügt. Betreffend ein fremdes Nucleinsäuremolekül codierend eine Heparin-/Heparosansynthase handelt es sich vorzugsweise um ein fremdes Nucleinsäuremolekül, codierend eine bakterielle Heparin-/Heparosansynthase, bevorzugt codierend eine Heparin-/Heparosansynthase aus *Pasteurella,* besonders bevorzugt codierend eine Heparin-/Heparosansynthase aus *Pasteurella multocida.*

Bevorzugt ist das Heparin-/Heparosansynthase codierende fremde Nucleinsäuremolekül dadurch gekennzeichnet, dass es eine Heparin-/Heparosansynthase codiert, dessen Aminosäuresequenz mit der unter SEQ ID NO 7 dargestellten Aminosäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% aufweist. In einer besonders bevorzugten Ausführungsform ist das Heparin-/Heparosansynthase codierende fremde Nucleinsäuremolekül dadurch gekennzeichnet, dass es eine Heparin-/Heparosansynthase codiert, die die unter SEQ ID No 7 dargestellte Aminosäuresequenz aufweist.
In einer weiteren Ausführungsform weist das fremde Nucleinsäuremolekül, codierend eine Heparin-/Heparosansynthase mit der unter SEQ ID NO 6 dargestellten Nucleinsäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% auf. In einer besonders bevorzugten Ausführungsform ist das Heparin-/Heparosansynthase codierende Nucleinsäuremolekül dadurch gekennzeichnet, dass es die unter SEQ ID No 6 dargestellte Nucleinsäuresequenz aufweist oder dass die Sequenz des fremden Nucleinsäuremoleküls von der unter SEQ ID No 6 dargestellten Nucleinsäuresequenz aufgrund der Degeneration des genetisches Codes abweicht.

Unter dem Begriff "Glucosamin-Phosphat-N-Acetyltransferase (acetyl-CoA:D-Glucosamin-Phosphat N-Acetyltransferase oder GlcN-P Acetyltransferase)" (EC 2.3.1.4) soll im Zusammenhang mit der vorliegenden Erfindung ein Protein verstanden werden, das ausgehend von den Substraten D-Glucosamin-Phosphat (GlcN-P) und acetyl-CoA (AcCoA) N-acetyl-D-Glucosamin-Phosphat (GlcNAc-P) synthetisiert. Die Katalyse der Synthese von N-acetyl-Glucosamin-6-Phosphat folgt dabei folgendem Reaktionsschema:

GlcN-P + AcCoA → GlcNAc-P + CoASH

In der dargestellten Reaktionsgleichung kann es sich bei dem Substrat GlcN-P sowohl um Glucosamin-1-Phosphat (GlcN-1-P), als auch um Glucosamin-6-Phosphat (GlcN-6-P) handeln.

In den betreffenden Stoffwechselwegen zur Synthese von UDP-N-acetyl-Glucosamin besteht zwischen den untersuchten bakteriellen und eukaryontischen Organismen ein wesentlicher Unterschied darin, dass unterschiedliche Zwischenprodukte des betreffenden Stoffwechselweges als Substrat für die Acetylierungsreaktion verwendet werden. In bakteriellen Organismen erfolgt die Acetylierung von GlcN-1-P durch ein Protein mit der Aktivität einer GlcN-1-P Acetyltransferase (EC 2.3.1.157) (Gehring et al., 1996, Biochemistry 35, 579-585), wohingegen in eukaryontischen Tieren oder Pilzen GlcN-6-P durch eine Protein mit der Aktivität einer Glucosamin-6-Phosphat-Acetyltransferase (EC 2.3.1.4) katalysiert wird (Milewski et al., 2006, Yeast 23, 1-14, published online in Wiley InterScience, DOI: 10.1002./yea.1337). Es werden daher sowohl verschiedene Substrate, als auch verschiedene Proteine für die Synthese von UDP-GlcNAc in den verschiedenen Organismen verwendet.
Es wurde überraschenderweise gefunden, dass es, im Gegensatz zu der Offenbarung im Stand der Technik (WO 2007 023682), nicht möglich ist, die Menge an in Pflanzenzellen synthetisierten Glucosaminglycanen zu steigern, indem ein beliebiges Nucleinsäuremolekül codierend ein Protein mit der Aktivität einer GlcN-P Acetyltransferase in Pflanzenzellen eingeführt wird. Es wurde vielmehr gefunden, dass die Einführung eines fremden Nucleinsäuremoleküls, codierend ein Protein mit der Aktivität einer GlcN-P Acetyltransferase, das GlcN-1-P acetyliert (z.B. *glmu* aus *Escherichia coli*), nicht zur Erhöhung der Menge an von Pflanzenzellen oder Pflanzen synthetisierten Glucosaminglycanen führt. Es ist daher wesentlich für erfindungsgemäße Pflanzenzellen oder erfindungsgemäße Pflanzen, dass das fremde Nucleinsäuremolekül, ein Protein mit der Aktivität einer GlcN-P Acetyltransferase codiert, welches GlcN-6-P als Substrat für die Acetylierungsreaktion verwendet und daher ein Protein mit der Aktivität einer GlcN-6-P Acetyltransferase darstellt (EC 2.3.1.4). Fremde Nucleinsäuremoleküle, codierend Proteine mit der Aktivität einer GlcN-1-P Acetyltransferase (EC 2.3.1.157), die GlcN-1-P für die Acetylierungsreaktion verwenden, sind hingegen nicht zur Herstellung erfindungsgemäßer Pflanzenzellen bzw. erfindungsgemäßer Pflanzen geeignet. Es wurde weiterhin festgestellt, dass Pflanzenzellen oder Pflanzen, die ein fremdes Nucleinsäuremolekül, codierend eine Glucosamin-6-Phosphat Mutase (GlcN-6-P Mutase), welche eine Isomerisierung von GlcN-6-P zu GlcN-1-P katalysiert, aufweisen, keine signifikant erhöhten Mengen an Glucosaminglycan synthetisieren.

Unter dem Begriff "Glucosamin-6-Phosphat-Acetyltransferase (acetyl-CoA:D-Glucosamin-6-Phosphat N-Acetyltransferase oder GlcN-6-P Acetyltransferase)" (EC 2.3.1.4) soll im Zusammenhang mit der vorliegenden Erfindung ein Protein verstanden werden, das ausgehend von den Substraten D-Glucosamin-6-Phosphat (GlcN-6-P) und acetyl-CoA (AcCoA) N-acetyl-D-Glucosamin-6-Phosphat (GlcNAc-6-P) synthetisiert. Die Katalyse der Synthese von N-acetyl-Glucosamin-6-Phosphat folgt dabei folgendem Reaktionsschema:

GlcN-6-P + AcCoA → GlcNAc-6-P + CoASH

I

Die funktionelle Form eines Proteins mit der Aktivität einer GlcN-6-P Acetyltransferase stellt ein Homodimer dar. Die Tertiärstruktur des Monomers weist eine zentrale Kernregion auf. Diese Kernregion besteht aus einer beta-Faltblattstruktur mit fünf antiparallelen Strängen (beta-Stränge 1 - 5), die von vier alpha-Helices und einem sechsten beta-Strang (beta-6-Strang) umgeben sind. Während der Homodimerbildung besteht eine Interaktion eines beta-6-Stranges einer Untereinheit mit dem betreffenden beta-6-Strang der betreffenden anderen Untereinheit.
Die unter SEQ ID No 9 dargestellte Aminosäuresequenz (EMBL acc No: AB017626.1) codiert ein Protein mit der Aktivität einer GlcN-6-P Acetyltransferase aus *Saccharomyces cerevisiae.* In der unter SEQ ID No 9 dargestellten Aminosäuresequenz stellen die Aminosäuren 7 - 11 den beta-1-Strang, Aminosäuren 13 - 26 den alpha-1-Strang, Aminosäuren 37 - 47 den alpha-2-Strang, Aminosäuren 62 - 69 den beta-2-Strang, Aminosäuren 74 - 86 den beta-3-Strang, Aminosäuren 92 - 103 den beta-4-Strang, Aminosäuren 111 - 125 den alpha-3-Strang, Aminosäuren 130 - 136 den beta-5-Strang, Aminosäuren 139 - 146 den alpha-3-Strang und Aminosäuren 154 - 159 den beta-6-Strang dar. Die im beta-4-Strang vorliegenden Aminosäuren Glu (Position 98), Asp (Position 99) und Ile (Position 100) in der unter SEQ ID No 9 dargestellten Sequenz interagieren mit dem Substart AcCoA, polarisieren dessen Carbonyl-Bindung und stabilisieren die negative Ladung des Sauerstoffatoms des AcCoA im tetraedrischen Reaktionszwischenprodukt bestehend aus AcCoA und GlcN-6-P und GlcN-6-P acetyltransferase. Die Aminosäure Tyr (Position 143) in der unter SEQ ID No 9 dargestellten Sequenz stabilisiert das Thiolat Anion des abzuspaltenden CoA Moleküls. Diese Interaktionen während der Katalyse der Reaktion werden durch die Aminosäure Leu (Position 133) in der unter SEQ ID No 9 dargestellten Sequenz unterstützt. GlcN-6-P wird während der Reaktionskatalyse in einer zwischen den Monomeren des Homodimers ausgebildeten Tasche, an deren Ausbildung Aminosäurereste des beta-6-Stranges beteiligt sind, gebunden. Während der Katalyse der Reaktion erhöht die Aminsäure Asp (Position 134) in der unter SEQ ID No 9 dargestellten Sequenz die Nucleophilität der Aminogruppe des GlcN-6-P (Milewski et al., 2006, published online in Wiley InterScience, www.interscience.wiley.com, DOI:10.1002/yea.1337). Weitere Aminosäuren eines Proteins mit der Aktivität einer GlcN-6-P Acetyltransferase die an der Katalyse der betreffenden Reaktion beteiligt sind, sind bei Peneff et al. (2004, J. Biological Chemistry 276(19), 16328-16334, Fig. 1) beschrieben.
Die hier exemplarisch für die Aminosäuresequenz von *Saccharomyces cerevisiae* an der Katalyse der Reaktion beteiligten Aminosäuren können auch in Aminosäuresequenzen codierend Proteine mit der Aktivität einer GlcN-6-P Acetyltransferase aus anderen Organismen identifiziert werden. Beispielsweise sind dieses die Aminosäuren Glu88, Asp80, Ile90, Asp124 und Tyr133 in der Aminosäuresequenz codierend ein Protein mit der Aktivität einer GlcN-6-P Acetyltransferase aus *Candida albicans* (EMBL acc. No: AB017627.1).

Nucleinsäuremoleküle und korrespondierende Proteinsequenzen, codierend Proteine mit der Aktivität einer GlcN-6-P Acetyltransferase sind u.a. für folgende Organismen beschrieben: *Saccharomyces cerevisiae* (EMBL acc No: AB017626.1), *Schizosaccharomyces pombe* (EMBL acc. No: AB017629.1), *Candida albicans* (EMBL acc No: AB017627.1), *Aspergillus oryzae* (EMBL CDS acc. No: BAE62756.1), *Caenorhabditis elegans* (NCBI acc No: NM_073253.4, EMBL CDS acc. No: BAA63497.1, CAA044531.1), *Drosophila melanogaster* (EMBL CDS acc. Nos: AAL13916.1), *Xenopus traopicalis* (EMBL acc. No: CR760021.2), *Mus musculus* (EMBL CDS acc. No: BAE39886.1), *Homo sapiens* (EMBL CDS acc. No: BAC03482.1), *Pongo pygmaeus* (EMBL CDS acc. No: CR858996.1), *Acanthamoeba polyphaga* Mimivirus (EMBL CDS acc. No: AAV50586.1). Obwohl, wie bereits beschrieben, die an der Katalyse der Reaktion beteiligten Aminosäurereste von aus unterschiedlichen Organismen stammenden Proteinen mit der Aktivität einer GlcN-6-P Acetyltransferase konserviert sind, weisen ihre Sequenzen teilweise sehr geringe Identitäten zueinander auf. So weist die Aminosäuresequenz, codierend ein Protein mit der Aktivität einer GlcN-6-P Acetyltransferase aus *Saccharomyces cerevisiae* (EMBL acc No: AB017626.1) zu der entsprechenden Sequenz aus *Candida albicans* (EMBL acc No: AB017627.1) nur eine Identität von 44% und zu der aus *Schizosaccharomyces pombe* (EMBL acc. No: AB017629.1) sogar nur eine Identität von 25% auf (Milewski et al., 2006, published online in Wiley InterScience, www.interscience.wiley.com, DOI:10.1002/yea.1337). Trotz der geringen Identität der betreffenden Aminosäuresequenzen zueinander, eigenen sich alle genannten Sequenzen codierend ein Protein mit der Aktivität einer GlcN-6-P-Acetyltransferase zur Herstellung erfindungsgemäßer Pflanzenzellen oder erfindungsgemäßer Pflanzen.

Erfindungsgemäß kann das fremde Nucleinsäuremolekül codierend ein Protein mit der enzymatischen Aktivität einer GlcN-6-P acetyltransferase aus einem beliebigen Organismus stammen, bevorzugt stammt besagtes Nucleinsäuremolekül aus Pilzen, Tieren oder Pflanzen besonders bevorzugt aus Pilzen und insbesondere bevorzugt aus *Saccharomyces cerevisiae.*
Bevorzugt ist das GlcN-6-P acetyltransferase codierende fremde Nucleinsäuremolekül dadurch gekennzeichnet, dass es eine GlcN-6-P acetyltransferase codiert, dessen Aminosäuresequenz mit der unter SEQ ID NO 9 dargestellten Aminosäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% aufweist. In einer besonders bevorzugten Ausführungsform ist das fremde Nucleinsäuremolekül codierend ein Protein mit der Aktivität einer GlcN-6-P acetyltransferase dadurch gekennzeichnet, dass es ein Protein mit der Aktivität einer GlcN-6-P acetyltransferase codiert, die die unter SEQ ID No 9 dargestellte Aminosäuresequenz aufweist.
In einer weiteren Ausführungsform weist das fremde Nucleinsäuremolekül, codierend ein Protein mit der Aktivität einer GlcN-6-P acetyltransferase mit der unter SEQ ID NO 8 dargestellten Nucleinsäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% auf. In einer besonders bevorzugten Ausführungsform ist das GlcN-6-P acetyltransferase codierende Nucleinsäuremolekül dadurch gekennzeichnet, dass es die unter SEQ ID No 8 dargestellte Nucleinsäuresequenz aufweist oder dass die Sequenz des fremden Nucleinsäuremoleküls von der unter SEQ ID No 8 dargestellten Nucleinsäuresequenz aufgrund der Degeneration des genetisches Codes abweicht.

Unter dem Begriff "UDP-GlcNAc Pyrophosphorylase (2-acetamido-2-deoxy-d-Glucose-1-Phosphat Uridyltransferase) (EC 2.7.7.23)" soll im Zusammenhang mit der vorliegenden Erfindung ein Protein verstanden werden, das ausgehend von den Substraten Uridintriphosphate (UTP) und N-acetyl-D-Glucosamin 1-Phosphat (GlcNAc-1-P) UDP-N-acetyl-Glucosamin (UDP-GlcNAc) synthetisiert. Die Katalyse der Synthese von UDP-GlcNAc folgt dabei folgendem Reaktionsschema:

UTP + GlcNAc-1-P ⇆ P-P + UDP-GlcNAc

Prokaryontische Proteine mit der Aktivität einer UDP-GlcNAc Pyrophosphorylase stellen in der Regel bifunktionale Proteine dar, die zusätzlich zu der oben dargestellten Reaktion (EC 2.7.7.23) die Funktion einer Glucosamin-1-Phosphat Acetyltransferase (GlcN-1-P Acetyltransferase, EC 2.3.1.157) aufweisen, also eine N-Acetylierung von Glucosamin-1-Phosphat (GlcN-1-P) zu N-acetyl-Glucosamin-1-Phosphat (GlcNAc-1-P) katalysieren (GlcN-1-P + AcCoA → GlcNAc-1-P + CoASH) (Gehring et al., 1996, Biochemistry 35, 579-585). Eukaryontische Proteine mit der Aktivität einer UDP-GlcNAc Pyrophosphorylase hingegen stellen monofunktionale Proteine dar, die lediglich die oben beschriebe Reaktion (UTP + GlcNAc-1-P ⇆ P-P + UDP-GlcNAc) katalysieren (Mio et al., 1998, J. Biol. Chem. 273 (23), 14392-14397).

Unter dem Begriff "monofunktionale Proteine mit der Aktivität einer UDP-GlcNAc Pyrophosphorylase" soll im Zusammengang mit der vorliegenden Erfindung ein Protein verstanden werden, dass die oben für ein Protein mit der Aktivität einer UDP-GlcNAc Pyrophosphorylase dargestellte Reaktion (UTP + GlcNAc-1-P ⇆ P-P + UDP-GlcNAc) katalysiert. Ein monofunktionales Protein mit der Aktivität einer UDP-GlcNAc Pyrophosphorylase weist keine (zusätzliche) Aktivität auf, die eine Acetylierung von GlcN-1-P zu GlcNAc-1-P katalysiert. Monofunktionale Proteine mit der Aktivität einer GlcNAc Pyrophosphorylase stellen daher ein unter der EC Nummer 2.7.7.23 klassifiziertes Protein dar, wohingegen bifunktionale Proteine mit der Aktivität einer UDP-GlcNAc Pyrophosphorylase sowohl die Aktivität eines unter EC Nummer 2.7.7.23, als auch die Aktivität eines unter EC Nummer 2.3.1.157 klassifizierten Enzyms aufweisen.

Es wurde im Gegensatz zu der Offenbarung im Stand der Technik (z.B. WO 2007 023682) überraschenderweise gefunden, dass erfindungsgemäße Pflanzenzellen oder erfindungsgemäße Pflanzen, die ein fremdes Nucleinsäuremolekül, codierend ein bifunktionales Protein mit der Aktivität einer UDP-GlcNAc Pyrophosphorylase und der Aktivität einer GlcN-1-P Acetyltransferase (z.B. *glm*U aus E. *coli,* EC 2.7.7.23 und EC 2.3.1.157) aufweisen, keine erhöhten Mengen an Glucosaminglycanen synthetisieren. Es ist daher wesentlich für erfindungsgemäße Pflanzenzellen oder erfindungsgemäße Pflanzen, dass das fremde Nucleinsäuremolekül, codierend eine UDP-GlcNAc Pyrophosphorylase ein monofunktionales Protein mit der Aktivität einer UDP-GlcNAc Pyrophosphorylase (EC 2.7.7.23) codiert. Das fremde Nucleinsäuremolekül, codierend eine UDP-GlcNAc Pyrophosphorylase soll daher kein Protein codieren, dass zu der gerade genannten Aktivität einer UDP-GlcNAc Pyrophosphorylase eine zusätzliche Aktivität einer GlcN-1-P Acetyltransferase (EC 2.3.1.157) aufweist. Es handelt sich daher bevorzugt um ein fremdes Nucleinsäuremolekül eukaryontischen Ursprungs.
Weiterhin wurde überraschenderweise gefunden, dass im Gegensatz zu der Offenbarung im Stand der Technik (z.B. WO 2007 023682) die Expression einer Phosphoacetylglucosamin Mutase (GlcNAc-P Mutase, EC 5.4.2.3) zusätzlich zu der Expression eines Proteins mit der Aktivität einer GlcN-6-P Acetyltransferase und der Expression eines Proteins mit der Aktivität einer UDP-GlcNAc Pyrophosphorylase nicht zur weiteren Erhöhung der Menge an Glucosaminglycanen in Pflanzenzellen bzw. Pflanzen führt.

Aminosäuresequenzen, codierend monofunktionale Proteine mit der Aktivität einer UDP-GlcNAc Pyrophosphorylase weisen zwischen den Proteinen hoch konservierte Aminosäurereste auf. Aminosäuresequenzen codierend eukaryontische Proteine mit der Aktivität einer UDP-GlcNAc Pyrophosphorylase weisen jeweils drei zwischen den Proteinen konservierte Domänen auf. Die Konsensussequenz der ersten Domäne lautet GlyGlyGlnXxxThrArgLeuGlyXxxXxxXxxProLysGly (Aminosäuren 111-124 in der unter SEQ ID No 11 dargestellten Sequenz), die der zweiten Domäne lautet Pro(Asp oder Asn)GlyAsn(Gly oder Ala)GlyXxxXxxXxxAla (Aminosäuren 219-228 in der unter SEQ ID No 11 dargestellten Sequenz) und die der dritten Domäne lautet LysXxxGluXxxPheXxxPheAspXxxPhe (Aminosäuren 377-386 in der unter SEQ ID No 11 dargestellten Sequenz), wobei Xxx für eine beliebige Aminosäure steht. Prokaryontische Proteine mit der Aktivität einer UDP-GlcNAc Pyrophosphorylase (z.B. glmU aus *Escherichia coli,* EMBL acc. No: EAY46949.1) weisen eine konservierte Domäne (GlyXxxGlyThr(Arg oder Ser)XxxXxxXxxXxxProLys) auf, die der ersten Domäne von entsprechenden Proteinen aus Eukaryonten ähnlich ist. Für die Domänen zwei und drei der Eukaryontischen Proteine finden sich keine entsprechenden Domänen in den prokaryontischen Proteinen. (Mok und Edwards, 2000, J. Biol. Chem. 280(47), 39363-39372)

Die Aminosäuren Gly (Position 112), Gly (Position 114), Thr (Position 115), Arg (Position 116), Pro (Position 122) und Lys (Position 123) in der unter SEQ ID No 11 dargestellten Aminosäuresequenz sind in den Primärsequenzen codierend Proteine mit der Aktivität einer UDP-GlcNAc Pyrophosphorylase konserviert. Ein Austausch der Aminosäuren Gly (Position 112), Arg (Position 116), oder Lys (Position 123) in der unter SEQ ID No 11 dargestellten Aminosäuresequenz führt zu nahezu inaktiven Proteinen. Ein Austausch der Aminosäuren Gly (Position 114), Thr (Position 115) oder Pro (Position 122) in der unter SEQ ID No 11 dargestellten Aminosäuresequenz bewirkt hingegen nur eine Reduktion der Aktivität des betreffenden Proteins. Die Aminosäuren Gly (Position 112), Arg (Position 116) bzw. Lys (Position 123) in der unter SEQ ID No 11 dargestellten Aminosäuresequenz stellen daher Aminosäuren dar, die in Proteinen mit der Aktivität einer UDP-GlcNAc Pyrophosphorylase katalytische Funktion aufweisen (Mio et al., 1998, J. Biol. Chem. 273(23), 14392-14397).

In der Aminosäuresequenz codierend ein Protein mit der Aktivität einer UDP-GlcNAc Pyrophosphorylase aus *Giardia intestinales* (EMBL acc. No: AAM54702.1) entspricht die Aminosäure Gly (Position 108) der Aminosäure Gly (Position 112) der unter SEQ ID No 11 dargestellten Sequenz. Der Austausch der Aminosäure Gly (Position 108) in der Aminosäuresequenz codierend ein Protein mit der Aktivität einer UDP-GlcNAc Pyrophosphorylase aus *Giardia intestinales* durch die Aminosäure Ala führt ebenfalls zu einer fast vollständigen Reduktion der Aktivität des Proteins (Mok und Edwards, 2005, J. Biol. Chem. 280(47), 39363-39372). Der Austausch der zu der Aminosäure Gly (Position 112) der unter SEQ ID No 11 dargestellten Sequenz korrespondierenden Aminosäure Gly (Position 111) in der Aminosäuresequenz codierend ein Protein mit der Aktivität einer UDP-GlcNAc Pyrophosphorylase aus *Homo sapiens* (EMBL acc. No: BAA31202.1) führt ebenfalls zur fast vollständigen Reduktion der Aktivität (Wang-Gillam et al., 2000, J. Biol. Chem. 275(2), 1433-1438). Ein Austausch der Aminosäure Gly (Position 222) in dem Protein codierend eine UDP-GlcNAc Pyrophosphorylase aus *Homo sapiens* (EMBL acc. No: BAA31202) und der korrespondierenden Aminosäure Gly (Position 210) von einem korrespondierenden Protein aus *Giardia intestinales* (EMBL acc. No: AAM54702.1) führt in beiden Fällen ebenfalls zu einem annähernd vollständigem Verlust der Aktivität, was darauf hinweist, dass die genannten Aminosäuren ebenfalls an der Katalyse beteiligte Aminosäuren darstellen (Mok und Edwards, 2005, J. Biol. Chem. 280(47), 39363-39372). Ein Austausch der Aminosäure Gly (Position 224) in dem Protein codierend eine UDP-GlcNAc Pyrophosphorylase aus *Homo sapiens* (EMBL acc. No: BAA31202) führte zu einem beträchtlichen aber nicht ganz vollständigen Verlust der Aktivität des Proteins und ein Austausch der Aminosäure Pro (Position 222) hatte nur eine geringfügige Reduktion der Aktivität zur Folge. Daraus wurde gefolgert, dass die Aminosäuren Gly (Position 221 und Gly (Position 223) der unter SEQ ID No 11 dargestellten Sequenz an der Erkennung des UTP und die in den jeweiligen Primärsequenzen konservierten Aminosäuren Gly (Position 111) und Gly (Position 112) der unter SEQ ID No 11 dargestellten Sequenz an der Bindung des GlcNAc-1-P beteiligt sind (Wang-Gillam et al., 2000, J. Biol. Chem. 275(2), 1433-1438).

Nucleinsäuremoleküle und korrespondierende Proteinsequenzen, codierend Proteine mit monofunktionaler Aktivität einer UDP-GlcNAc Pyrophosphorylase, die die oben genannten Eigenschaften aufweisen, sind u.a. für folgende Organismen beschrieben: *Giardia intestinales* (EMBL acc. No: AAM54702.1), *Saccharomyces cerevisiae* (EMBL acc. No: X79380.1, NCBI Protein ID: accession No: CAA557927), *Candida albicans* (NCBI acc. No: XM_715480.1), *Pichia stipitis* (NCBI acc. No: XM_001385151.1), *Mus musculus* (NCBI acc. No: NM_133806.4), *Canis lupus* (NCBI acc. No: XM_844774.1); *Bos taurus* (NCBI acc. No: NM_001046404.1), *Xenopus tropicalis* (NM_001011142.1), *Xenopus laevis* (NCBI acc. No: BC077836.1), *Arabidopsis thaliana* (NCBI acc. No: NM_102845.4), *Danio rerio* (NCBI acc. No: NM_212621.1), *Homo sapiens* (NCBI acc. No: NM_003115.3, EMBL acc.No.: BAA31202.1).

Erfindungsgemäß kann das fremde Nucleinsäuremolekül codierend ein Protein mit der enzymatischen Aktivität einer UDP-GlcNAc Pyrophosphorylase aus einem beliebigen eukaryontischen Organismus stammen, bevorzugt stammt besagtes Nucleinsäuremolekül aus Pilzen, Tieren oder Pflanzen, besonders bevorzugt aus Pilzen, insbesondere bevorzugt aus *Saccharomyces cerevisiae.*
Bevorzugt ist das ein Protein mit der Aktivität einer UDP-GlcNAc Pyrophosphorylase codierende fremde Nucleinsäuremolekül dadurch gekennzeichnet, dass es eine UDP-GlcNAc Pyrophosphorylase codiert, dessen Aminosäuresequenz mit der unter SEQ ID NO 11 dargestellten Aminosäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% aufweist. In einer besonders bevorzugten Ausführungsform weist das fremde Nucleinsäuremolekül codierend ein Protein mit der Aktivität einer UDP-GlcNAc Pyrophosphorylase dadurch gekennzeichnet, dass es ein Protein mit der Aktivität einer UDP-GlcNAc Pyrophosphorylase codiert, die die unter SEQ ID No 11 dargestellte Aminosäuresequenz aufweist.
In einer weiteren Ausführungsform weist das fremde Nucleinsäuremolekül, codierend ein Protein mit der Aktivität einer UDP-GlcNAc Pyrophosphorylase mit der unter SEQ ID NO 10 dargestellten Nucleinsäuresequenz eine Identität von mindestens 70%, vorzugsweise von mindestens 80%, bevorzugt von mindestens 90%, insbesondere bevorzugt von mindestens 95% und im speziellen bevorzugt von mindestens 98% auf. In einer besonders bevorzugten Ausführungsform ist das UDP-GlcNAc Pyrophosphorylase codierende Nucleinsäuremolekül dadurch gekennzeichnet, dass es die unter SEQ ID No 10 dargestellte Nucleinsäuresequenz aufweist oder dass die Sequenz des fremden Nucleinsäuremoleküls von der unter SEQ ID No 10 dargestellten Nucleinsäuresequenz aufgrund der Degeneration des genetisches Codes abweicht.

Unter dem Begriff "fremdes Nucleinsäuremolekül" soll im Zusammenhang mit der vorliegenden Erfindung ein solches Molekül verstanden werden, das entweder natürlicherweise in entsprechenden Wildtyp-Pflanzenzellen nicht vorkommt, oder das in der konkreten räumlichen Anordnung nicht natürlicherweise in Wildtyp-Pflanzenzellen vorkommt oder das an einem Ort im Genom der Wildtyp-Pflanzenzelle lokalisiert ist, an dem es natürlicherweise nicht vorkommt.
Bevorzugt ist das fremde Nucleinsäuremolekül ein rekombinantes Molekül, das aus verschiedenen Elementen besteht, deren Kombination oder spezifische räumliche Anordnung natürlicherweise in pflanzlichen Zellen nicht auftritt.

Unter dem Begriff "rekombinantes Nucleinsäuremolekül" soll im Zusammenhang mit der vorliegenden Erfindung ein Nucleinsäuremolekül verstanden werden, welches unterschiedliche Nucleinsäuremoleküle aufweist, die natürlicherweise nicht in einer Kombination vorliegen, wie sie in einem rekombinanten Nucleinsäuremolekül vorliegen. So können rekombinante Nucleinsäuremoleküle z.B., neben Nucleinsäuremolekülen, die eine Glucosaminglycansynthase und/oder ein Protein mit der Aktivität einer GlcN-6-P Acetyltransferase und/oder ein Protein mit der Aktivität einer UDP-GlcNAc Pyrophosphorylase codieren, zusätzliche Nucleinsäuresequenzen aufweisen, welche natürlicherweise nicht in Kombination mit den genannten Nucleinsäuremolekülen vorliegen. Die genannten zusätzlichen Nucleinsäuresequenzen, die auf einem rekombinanten Nucleinsäuremolekül in Kombination mit einem eine Glucosaminglycansynthase und/oder einem ein Protein mit der Aktivität einer GlcN-6-P Acetyltransferase und/oder einem ein Protein mit der Aktivität einer UDP-GlcNAc Pyrophosphorylase codierenden Nucleinsäuremolekül vorliegen, können dabei beliebige Sequenzen sein. Sie können z.B. genomische pflanzliche Nucleinsäuresequenzen darstellen. Bevorzugt handelt es sich bei genannten zusätzlichen Nucleinsäuresequenzen um regulatorische Sequenzen (Promotoren, Terminationssignale, Enhancer), besonders bevorzugt um regulatorische Sequenzen, die in pflanzlichem Gewebe aktiv sind, insbesondere bevorzugt um gewebespezifische regulatorische Sequenzen die in pflanzlichem Gewebe aktiv sind. Methoden zur Erzeugung rekombinanter Nucleinsäuremoleküle sind dem Fachmann bekannt und umfassen gentechnische Methoden wie z.B. die Verbindung von Nucleinsäuremolekülen durch Ligation, genetische Rekombination oder die Neusynthese von Nucleinsäuremolekülen (siehe z.B. Sambrok et al., Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons; 5th edition ( 2002),ISBN: 0471250929).

Eine bevorzugte Ausführungsform der vorliegenden Erfindung betrifft erfindungsgemäße Pflanzenzellen oder erfindungsgemäße Pflanzen, worin die fremden Nucleinsäuremoleküle stabil in das Genom der Pflanzenzelle bzw. Pflanze integriert sind.

Genetisch modifizierte Pflanzenzellen und genetisch modifizierte Pflanzen, die ein in ihr Genom (stabil) integriertes fremdes Nucleinsäuremolekül oder mehrere in ihr Genom integrierte fremde Nucleinsäuremoleküle aufweisen, die eine Glucosaminglycansynthase und/oder ein Protein mit der Aktivität einer GlcN-6-P Acetyltransferase und/oder ein Protein mit der Aktivität einer UDP-GlcNAc Pyrophosphorylase codieren, lassen sich von Wildtyp-Pflanzenzellen bzw. Wildtyp-Pflanzen unter anderem dadurch unterscheiden, dass sie ein fremdes Nucleinsäuremolekül enthalten, das natürlicherweise in Wildtyp-Pflanzenzellen bzw. Wildtyp-Pflanzen nicht vorkommt oder dadurch, dass ein solches Molekül an einem Ort im Genom der erfindungsgemäßen genetisch modifizierten Pflanzenzelle oder im Genom der erfindungsgemäßen genetisch modifizierten Pflanze integriert vorliegt, an dem es bei Wildtyp-Pflanzenzellen bzw. Wildtyp-Pflanzen nicht vorkommt, d.h. in einer anderen genomischen Umgebung. Ferner lassen sich derartige erfindungsgemäße genetisch modifizierte Pflanzenzellen und erfindungsgemäße genetisch modifizierte Pflanzen von nicht genetisch modifizierten Wildtyp-Pflanzenzellen bzw. nicht genetisch modifizierten Wildtyp-Pflanzen dadurch unterscheiden, dass sie mindestens eine Kopie des fremden Nucleinsäuremoleküls integriert in ihr Genom enthalten, gegebenenfalls zusätzlich zu natürlicherweise in den Wildtyp-Pflanzenzellen bzw. Wildtyp-Pflanzen vorkommenden Kopien eines solchen Moleküls. Handelt es sich bei dem (den) in die erfindungsgemäßen genetisch modifizierten Pflanzenzellen oder erfindungsgemäßen genetisch modifizierten Pflanzen eingeführten fremden Nucleinsäuremolekül(en) um zusätzliche Kopien zu bereits natürlicherweise in den Wildtyp-Pflanzenzellen bzw. Wildtyp-Pflanzen vorkommenden Molekülen, so lassen sich die erfindungsgemäßen genetisch modifizierten Pflanzenzellen und die erfindungsgemäßen genetisch modifizierten Pflanzen von Wildtyp-Pflanzenzellen bzw. Wildtyp-Pflanzen insbesondere dadurch unterscheiden, dass diese zusätzliche(n) Kopie(n) an Orten im Genom lokalisiert ist (sind), an denen sie bei Wildtyp-Pflanzenzellen bzw. Wildtyp-Pflanzen nicht vorkommt (vorkommen).
Nachgewiesen werden kann die Integration eines Nucleinsäuremoleküls in das Genom einer Pflanzenzelle oder einer Pflanze durch genetische und/oder molekularbiologische Methoden. Eine stabile Integration eines Nucleinsäuremoleküls in das Genom einer Pflanzenzelle oder in das Genom einer Pflanze zeichnet sich dadurch aus, dass das stabil integrierte Nucleinsäuremolekül bei der Nachkommenschaft, der besagtes Nucleinsäuremolekül vererbt wurde, in derselben genomischen Umgebung vorliegt wie in der Elterngeneration. Das Vorliegen einer stabilen Integration einer Nucleinsäuresequenz im Genom einer Pflanzenzelle oder im Genom einer Pflanze kann mit dem Fachmann bekannten Methoden, u.a. mit Hilfe der Southern-Blot Analyse der RFLP-Analyse (Restriction Fragment Length Polymorphism) (Nam et al., 1989, The Plant Cell 1, 699-705; Leister and Dean, 1993, The Plant Journal 4 (4), 745-750), auf PCR basierenden Methoden, wie z.B. die Analyse von amplifizierten Fragment Längenunterschieden (Amplified Fragment Length Polymorphism, AFLP) (Castiglioni et al., 1998, Genetics 149, 2039-2056; Meksem et al., 2001, Molecular Genetics and Genomics 265, 207-214; Meyer et al., 1998, Molecular and General Genetics 259, 150-160) oder der Verwendung von mit Restriktionsendonucleasen geschnittenen amplifizierten Fragmenten (Cleaved Amplified Polymorphic Sequences, CAPS) (Konieczny und Ausubel, 1993, The Plant Journal 4, 403-410; Jarvis et al., 1994, Plant Molecular Biology 24, 685-687; Bachem et al., 1996, The Plant Journal 9 (5), 745-753) nachgewiesen werden.

Der Begriff "Wildtyp-Pflanzenzelle" bedeutet im Zusammenhang mit der vorliegenden Erfindung, dass es sich um Pflanzenzellen handelt, die als Ausgangsmaterial für die Herstellung der erfindungsgemäßen genetisch modifizierten Pflanzenzellen dienten, d.h. deren genetische Information, abgesehen von den eingeführten genetischen Modifikationen, die zu einer Integration eines Nucleinsäuremoleküls, codierend eine Glucosaminglycansynthase und/oder ein Protein mit der Aktivität einer GlcN-6-P Acetyltransferase und/oder ein Protein mit der Aktivität einer UDP-GlcNAc Pyrophosphorylase führen, der einer erfindungsgemäßen genetisch modifizierten Pflanzenzelle entspricht.

Der Begriff "Wildtyp-Pflanze" bedeutet im Zusammenhang mit der vorliegenden Erfindung, dass es sich um Pflanzen handelt, die als Ausgangsmaterial für die Herstellung der erfindungsgemäßen genetisch modifizierten Pflanzen dienten, d.h. deren genetische Information, abgesehen von den eingeführten genetischen Modifikationen, die zu einer Integration eines Nucleinsäuremoleküls codierend die eine Glucosaminglycansynthase und/oder ein Protein mit der Aktivität einer GlcN-6-P Acetyltransferase und/oder ein Protein mit der Aktivität einer UDP-GlcNAc Pyrophosphorylase führen, der einer erfindungsgemäßen genetisch modifizierten Pflanze entspricht.

Unter dem Begriff "Genom" soll im Zusammenhang mit der vorliegenden Erfindung die Gesamtheit des in einer pflanzlichen Zelle vorliegenden Erbmaterials verstanden werden. Dem Fachmann ist bekannt, dass neben dem Zellkern auch andere Kompartimente (z.B. Plastiden, Mitochondrien) Erbmaterial enthalten.

Für die (stabile) Integration von Nucleinsäuremolekülen in eine pflanzliche Wirtszelle steht eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten (Übersicht in "Transgenic Plants", Leandro ed., Humana Press 2004, ISBN 1-59259-827-7).

Die Verwendung der Agrobakterien-vermittelten Transformation von Pflanzenzellen ist intensiv untersucht und ausreichend in EP 120516; Hoekema, IN: The Binary Plant Vector System Offsetdrukkerij Kanters B.V. Alblasserdam (1985), Chapter V; Fraley et al., Crit. Rev. Plant Sci. 4, 1-46 und bei An et al. EMBO J. 4, (1985), 277-287 beschrieben worden. Für die Transformation von Kartoffel, siehe z.B. Rocha-Sosa et al., EMBO J. 8, (1989), 29-33), für die Transformation von Tomatenpflanzen z.B. US 5,565,347.

Auch die Transformation monokotyler Pflanzen mittels auf *Agrobacterium* Transformation basierender Vektoren wurde beschrieben (Chan et al., Plant Mol. Biol. 22, (1993),491-506; Hiei et al., Plant J. 6, (1994) 271-282; Deng et al, Science in China 33, (1990), 28-34; Wilmink et al., Plant Cell Reports 11, (1992), 76-80; May et al., Bio/Technology 13, (1995), 486-492; Conner und Domisse, Int. J. Plant Sci. 153 (1992), 550-555; Ritchie et al, Transgenic Res. 2, (1993), 252-265). Ein alternatives System zur Transformation von monokotylen Pflanzen ist die Transformation mittels des biolistischen Ansatzes (Wan und Lemaux, Plant Physiol. 104, (1994), 37-48; Vasil et al., Bio/Technology 11 (1993), 1553-1558; Ritala et al., Plant Mol. Biol. 24, (1994), 317-325; Spencer et al., Theor. Appl. Genet. 79, (1990), 625-631), die Protoplastentransformation, die Elektroporation von partiell permeabilisierten Zellen oder die Einbringung von DNA mittels Glasfasern. Insbesondere die Transformation von Mais wird in der Literatur mehrfach beschrieben (vgl. z. B. WO 95 06128, EP0513849, EP0465875, EP0292435; Fromm et al., Biotechnology 8, (1990), 833-844; Gordon-Kamm et al., Plant Cell 2, (1990), 603-618; Koziel et al., Biotechnology 11 (1993), 194-200; Moroc et al., Theor. Appl. Genet. 80, (1990), 721-726). Die Transformation von anderen Gräsern, wie z.B. der Rutenhirse (switchgrass, *Panicum virgatum,* Somleva et al., 2002, Crop Science 42:2080-2087; Richards et al., 2001, Plant Cell Reporters 20, 48-54) von Zuckerrohr (Bower und Birch, 1992, Plant Journal 2(3), 409-416; Bower et al., 1996, Molecular Breeding 2, 239-249; Arencibia et al., 1998, Transgenic Research 7, 213-222) oder Hirse (Casas et al., 1993, PNAS 90, 11212-11216; US 6,369,298) ist ebenfalls beschrieben.
Auch die erfolgreiche Transformation von Getreidearten wurde bereits beschrieben, z.B. für Gerste (Wan und Lemaux, s.o.; Ritala et al., s.o.; Krens et al., Nature 296, (1982), 72-74) und für Weizen (Nehra et al., Plant J. 5, (1994), 285-297; Becker et al., 1994, Plant Journal 5, 299-307). Alle vorstehenden Methoden sind im Rahmen der vorliegenden Erfindung geeignet.

Erfindungsgemäße genetisch modifizierte Pflanzenzellen oder erfindungsgemäße genetisch modifizierte Pflanzen bieten gegenüber dem Stand der Technik den Vorteil, dass sie höhere Mengen an Glucosaminglycan (z.B. Hyaluronan) produzieren als Pflanzen, die nur die Aktivität einer Glucosaminglycansynthase aufweisen. Dieses ermöglicht die Glucosaminglycan Produktion mit geringem Aufwand, da die Isolierung von Glucosaminglycan aus Pflanzen mit höherem Glucosaminglycangehalt weniger aufwendig und kostengünstiger ist. Weiterhin sind geringere Anbauflächen notwendig, um mit erfindungsgemäßen genetisch modifizierten Pflanzen Glucosaminglycan zu produzieren im Vergleich zu im Stand der Technik beschriebenen Pflanzen. Dieses führt zu der Möglichkeit, Glucosaminglycan in ausreichender Menge auch für technische Anwendungen zur Verfügung zu stellen, bei welchen es wegen der geringen Verfügbarkeit und des hohen Preises zurzeit keine Verwendung findet. Pflanzlichen Organismen der Gattung *Chlorella,* die mit einem Virus infiziert sind, sind nicht geeignet für die Produktion von größeren Mengen von Glucosaminglycan (Hyaluronan). Virus infizierte Algen weisen für die Produktion von Glucosaminglycan (Hyaluronan) den Nachteil auf, dass sie die Gene, die für die Glucosaminglycansynthase notwendig sind, nicht stabil in ihr Genom integriert haben (Van Etten und Meints, 1999, Annu. Rev. Microbiol. 53, 447-494), so dass für die Produktion von Glucosaminglycan (Hyaluronan) immer wieder eine erneute Virusinfektion erfolgen muss. Daher ist es nicht möglich, einzelne *Chlorella* Zellen zu isolieren, die kontinuierlich die gewünschte Qualität und Quantität an Glucosaminglycan (Hyaluronan) synthetisieren. Weiterhin findet die Produktion von Glucosaminglycan (Hyaluronan) in Virus infizierten *Chlorella* Algen nur für eine begrenzte Zeit statt und die Algen werden durch von dem Virus bewirkte Lyse bereits etwa 8 Stunden nach der Infektion abgetötet (Van Etten et al., 2002, Arch Virol 147, 1479-1516). Demgegenüber bietet die vorliegende Erfindung den Vorteil, dass erfindungsgemäße genetisch modifizierte Pflanzenzellen und erfindungsgemäße genetisch modifizierte Pflanzen unbegrenzt vegetativ oder sexuell vermehrt werden können und dass sie kontinuierlich Glucosaminglycan (Hyaluronan) produzieren.
Die in WO 05 012529 beschriebenen transgenen Pflanzen, die ein Nucleinsäuremolekül, codierend eine Hyaluronansynthase, aufweisen, synthetisieren eine relativ geringe Menge an Glucosaminglycan (Hyaluronan). Demgegenüber bietet die vorliegende Erfindung den Vorteil, dass erfindungsgemäße genetisch modifizierte Pflanzenzellen und erfindungsgemäße genetisch modifizierte Pflanzen deutlich höhere Mengen an Glucosaminglycan synthetisieren.

Gegenstand der vorliegenden Erfindung sind daher auch erfindungsgemäße genetisch modifizierte Pflanzenzellen oder erfindungsgemäße genetisch modifizierte Pflanzen, die Glucosaminglycan synthetisieren.
In einer bevorzugten Ausführungsform synthetisieren erfindungsgemäße Pflanzenzellen oder erfindungsgemäße Pflanzen Glucosaminglycane ausgesucht aus der Gruppe aus Chondroitin, Heparin/Heparosan oder Hyaluronan.

Zur Bestimmung des Glucosaminglycangehaltes bezüglich des Frischgeweichtes in erfindungsgemäßen genetisch modifizierten Pflanzen wird bevorzugt das gesamte oberirdische Material der Pflanze, d.h. alle Pflanzenteile außer der Wurzel verwendet.
Erfindungsgemäße genetisch modifizierte Pflanzenzellen oder erfindungsgemäße genetisch modifizierte Pflanzen, die Glucosaminglycan synthetisieren, lassen sich dadurch nachweisen, dass man das von ihnen synthetisierte Glucosaminglycan isoliert und dessen Struktur nachweist. Da pflanzliches Gewebe den Vorteil aufweist, dass es keine Glucosaminglycane enthält, kann zum Nachweis des Vorliegens von Glucosaminglycanen in erfindungsgemäßen genetisch modifizierten Pflanzenzellen oder erfindungsgemäßen genetisch modifizierten Pflanzen eine einfache und schnelle Isolierungsmethode verwendet werden. Da pflanzliches Gewebe weiterhin den Vorteil aufweist, dass es keine Glucosaminglycan abbauenden Enzyme enthält, kann zum Nachweis des Vorliegens von Glucosaminglycanen in erfindungsgemäßen genetisch modifizierten Pflanzenzellen oder erfindungsgemäßen genetisch modifizierten Pflanzen eine einfache und schnelle Isolierungsmethode verwendet werden. Dazu wird zu dem zu untersuchenden Pflanzengewebe Wasser hinzu gegeben, bevor das Pflanzengewebe mechanisch (z.B. unter zu Hilfenahme einer Kugelmühle, eines Warring Blendors, eines Entsafters etc.) zerkleinert wird. Anschließend kann bei Bedarf erneut Wasser zu der Suspension hinzu gegeben werden, bevor Zelltrümmer und Wasser unlösliche Bestandteile durch Zentrifugation abgetrennt werden. Der Nachweis des Vorliegens von Glucosaminglycanen (z.B. Hyaluronan) kann anschließend im nach Zentrifugation erhaltenen Überstand z.B. mittels eines spezifisch an das betreffende Glucosaminglycan (z.B. Hyaluronan) bindenden Proteins erfolgen.
Solche, auf immunologischen Reagenzien beruhende Test Kits (ELISA) für verschiedene Glucosaminglycane sind dem Fachmann bekannt und käuflich erwerbbar (z.B. Test Kit für Heparin: Lifespan Technologies, 2401 Foothill Drive, Salt Lake City, UT 84109-1405, Prodkt Nr.: K-21 00).
Ein Verfahren zum Nachweis von Hyaluronan mit Hilfe eines spezifisch an Hyaluronan bindenden Proteins ist z.B. in US 5,019,498 beschrieben. Testkits zur Durchführung der in US 5,019,498 beschrieben Methode, sind käuflich erwerbbar (z.B. das Hyaluronic Acid (HA) Test Kit der Firma Corgenix, Inc., Colorado, USA, Prod. Nr. 029-001; siehe auch Allgemeine Methoden Punkt 4).
Chondroitin kann beispielsweise mit Hilfe von immunologischen Methoden nachgewiesen werden (Mizuguchi et al., 2003, Nature 423,443-448).
Weiterhin kann der Nachweis des Vorliegens von Glucosaminglycanen im Überstand der Zentrifugation auch mit anderen Analysemethoden, wie z.B. der IR-, NMR- oder Massen-Spektroskopie, erfolgen.

Da beobachtet wurde, dass Glucosaminglycane über die Entwicklungszeit erfindungsgemäßer Pflanzen in pflanzlichem Gewebe akkumulieren, soll die Menge an Glucosaminglycan bezüglich des Frischgewichtes in erfindungsgemäßen genetisch modifizierten Pflanzen besonders bevorzugt zum Zeitpunkt der Ernte oder wenige (ein bis zwei) Tage vor der Ernte betreffender Pflanzen bestimmt werden.

In einer bevorzugten Ausführungsform betrifft die vorliegende Erfindung erfindungsgemäße genetisch modifizierte Pflanzenzellen oder erfindungsgemäße genetisch modifizierte Pflanzen, dadurch gekennzeichnet, dass sie eine erhöhte Menge an Glucosaminglycan produzieren im Vergleich zu genetisch modifizierten Pflanzenzellen bzw. im Vergleich zu genetisch modifizierten Pflanzen, die nur ein fremdes Nucleinsäuremolekül, codierend eine Glucosaminglycansynthase aufweisen oder im Vergleich zu genetisch modifizierten Pflanzenzellen bzw. im Vergleich zu genetisch modifizierten Pflanzen, die ein fremdes Nucleinsäuremolekül, codierend eine Glucosaminglycansynthase aufweisen und keine fremden Nucleinsäuremoleküle, codierend Proteine mit der Aktivität einer UDP-GlcNAc Acetyltransferase und keine fremden Nucleinsäuremoleküle, codierend Proteine mit der Aktivität einer UDP-GlcNAc Pyrophosphorylase aufweisen.

Vorzugsweise ist die Menge an produziertem Glucosaminglycan bezüglich des Frischgewichtes des Pflanzematerials in erfindungsgemäßen genetisch modifizierten Pflanzenzellen bzw. in erfindungsgemäßen genetisch modifizierten Pflanzen um mindestens das 1,2 fache, bevorzugt um mindestens das 1,4 fache, besonders bevorzugt um mindestens das 1,6 fache und insbesondere bevorzugt um mindestens 1,8 fache erhöht, im Vergleich zu entsprechenden genetisch modifizierten Pflanzenzellen bzw. im Vergleich zu entsprechenden genetisch modifizierten Pflanzen, die (nur) die Aktivität einer Glucosaminglycansynthase aufweisen. Zur Feststellung der Erhöhung des Glucosaminglycangehaltes bezüglich des Frischgewichtes des Pflanzematerials in erfindungsgemäßen genetisch modifizierten Pflanzenzellen bzw. in erfindungsgemäßen genetisch modifizierten Pflanzen ist vorzugsweise ein Vergleich zwischen erfindungsgemäßen genetisch modifizierten Pflanzenzellen bzw. erfindungsgemäßen genetisch modifizierten Pflanzen mit entsprechenden Pflanzenzellen bzw. Pflanzen, die (nur) die Aktivität Glucosaminglycansynthase aufweisen heranzuziehen, wobei äquivalentes Material (z.B. Blatt, Knolle) von Pflanzenzellen bzw. Pflanzen miteinander verglichen werden soll, die Pflanzenzellen bzw. Pflanzen, von denen diese Material entnommen wird, unter gleichen Bedingungen kultiviert wurden und wobei der Glucosaminglycangehalt von Pflanzenmaterial miteinander verglichen werden soll, das ein vergleichbares Alter (Entwicklungsstadium) aufweist. Es sollen z.B. nicht junge Blätter einer Pflanze mit alten Blättern einer anderen Pflanze verglichen werden. Gleiches ist für die Bestimmung des Glucosaminglycangehaltes von gesamten oberirdischen Teilen von Pflanzen anzuwenden. Die zu vergleichenden Pflanzen sollen unter vergleichbaren Bedingungen kultiviert worden sein und das gleiche Entwicklungsstadium aufweisen.

In einer vorzugsweisen Ausführungsform betrifft die vorliegende Erfindung erfindungsgemäße Pflanzenzellen oder erfindungsgemäße Pflanzen, die mindestens 160 µg bevorzugt mindestens 180 µg besonders bevorzugt mindestens 200 µg, insbesondere bevorzugt mindestens 225 µg und speziell bevorzugt mindestens 250 µg Glucosaminglycan pro g Frischgewicht (FG) Pflanzenmaterial synthetisieren.
In einer weiteren Ausführungsform synthetisieren erfindungsgemäße Pflanzenzellen oder erfindungsgemäße Pflanzen maximal 450 µg bevorzugt maximal 400 µg besonders bevorzugt maximal 300 µg, insbesondere bevorzugt maximal 280 µg und speziell bevorzugt maximal 260 µg Glucosaminglycan pro g Frischgewicht (FG) Pflanzenmaterial.

In einer weiteren Ausführungsform der vorliegenden Erfindung handelt es sich bei erfindungsgemäßen genetisch modifizierten Pflanzenzellen oder erfindungsgemäßen genetisch modifizierten Pflanzen um Pflanzenzellen einer grünen Landpflanze bzw. um grüne Landpflanzen, die Glucosaminglycane synthetisieren.

Der Begriff "grüne Landpflanze (Embryophyta)" ist im Zusammenhang mit der vorliegenden Erfindung so zu verstehen, wie er in Strasburger, "Lehrbuch der Botanik", 34. Aufl., Spektrum Akad. Verl., 1999, (ISBN 3-8274-0779-6) definiert ist.

Eine bevorzugte Ausführungsform der vorliegenden Erfindung betrifft erfindungsgemäße genetisch modifizierte Pflanzenzellen von mehrzelligen Pflanzen oder erfindungsgemäße genetisch modifizierte Pflanzen, die mehrzellige Organismen darstellen. Es handelt sich bei dieser Ausführungsform daher um Pflanzenzellen oder Pflanzen die nicht von einzelligen Pflanzen (Protisten) stammen oder keine Protisten sind.

Bei den erfindungsgemäßen genetisch modifizierten Pflanzenzellen oder erfindungsgemäßen genetisch modifizierten Pflanzen kann es sich prinzipiell um Pflanzenzellen bzw. um Pflanzen jeder beliebigen Pflanzenspezies handeln, d.h. sowohl um monokotyle als auch dikotyle Pflanzen. Vorzugsweise handelt es sich um Nutzpflanzen, d.h. Pflanzen, die vom Menschen kultiviert werden für Zwecke der Ernährung von Mensch und Tier oder für die Produktion von Biomasse bzw. die Herstellung von Substanzen für technische, industrielle Zwecke (z.B. Mais, Reis, Weizen, Luzerne, Roggen, Hafer, Gerste, Maniok, Kartoffel, Tomate, Rutenhirse (*Panicum virgatum*), Sago, Mungbohne, Erbse, Sorghum, Möhre, Aubergine, Rettich, Raps, Sojabohne, Erdnuss, Gurke, Kürbis, Melone, Lauch, Knoblauch, Kohl, Spinat, Süßkartoffel, Spargel, Zucchini, Salat, Artischocke, Süßmais, Pastinak, Schwarzwurzel, Topinamubur, Banane, Zuckerrübe, Zuckerrohr, Rote Bete, Brokkoli, Kohl, Zwiebel, gelbe Rübe, Löwenzahn, Erdbeere, Apfel, Aprikose, Pflaume, Pfirsich, Weintrauben, Blumenkohl, Sellerie, Paprika, Steckrübe, Rhabarber). Besonders bevorzugt handelt es sich um Mais, Zuckerrohr, Süßkartoffel oder Zuckerhirse, insbesondere bevorzugt um Tomaten- oder Kartoffelpflanzen.

In einer weiteren bevorzugten Ausführungsform betrifft die vorliegende Erfindung erfindungsgemäße genetisch modifizierte Pflanzenzellen oder erfindungsgemäße genetisch modifizierte Pflanzen, wobei die fremden Nucleinsäuremoleküle, codierend Proteine, dadurch gekennzeichnet sind, dass die Codons des fremden Nucleinsäuremoleküls verändert sind, im Vergleich zu den Codons des Nucleinsäuremoleküls, welches das betreffende Protein des Ursprungsorganismus codiert. Besonders bevorzugt sind die Codons der fremden Nucleinsäuremoleküle dahingehend verändert, dass sie an die Häufigkeit der Verwendung der Codons der Pflanzenzelle oder der Pflanze, in dessen Genom sie integriert sind oder werden, angepasst sind.

Aminosäuren können auf Grund der Degeneration des genetischen Codes durch ein oder mehrere Codons codiert werden. Unterschiedliche Organismen verwenden die jeweils für eine Aminosäure codierenden Codons mit unterschiedlicher Häufigkeit. Das Anpassen der Codons einer codierenden Nucleinsäuresequenz an die Häufigkeit ihrer Verwendung in der Pflanzenzelle oder in der Pflanze, in dessen Genom die zu exprimierende Sequenz integriert werden soll, kann zu einer erhöhten Menge an translatiertem Protein und/oder zur Stabilität der betreffenden mRNA in den betreffenden Pflanzenzellen oder Pflanzen beitragen. Der Fachmann kann die Häufigkeit der Verwendung von Codons in betreffenden Pflanzenzellen oder Pflanzen ermitteln, indem er möglichst viele codierende Nucleinsäuresequenzen des betreffenden Organismus dahingehend untersucht, wie häufig bestimmte Codons für die Codierung einer bestimmten Aminosäure verwendet werden. Die Häufigkeit der Verwendung von Codons bestimmter Organismen ist dem Fachmann geläufig und kann mit Hilfe von Computerprogrammen einfach und schnell durchgeführt werden. Entsprechende Computerprogramme sind öffentlich zugänglich und werden u.a. im Internet frei zur Verfügung gestellt (z.B. http://gcua.schoedl.de/; http://www.kazusa.or.jp/codon/; http://www.entelechon.com/eng/cutanalysis.html).
Das Anpassen der Codons einer codierenden Nucleinsäuresequenz an die Häufigkeit ihrer Verwendung in der Pflanzenzelle oder in der Pflanze, in dessen Genom die zu exprimierende Sequenz integriert werden soll, kann durch *in vitro* Mutagenese oder bevorzugt durch Neusynthese der Gensequenz erfolgen. Methoden zur Neusynthese von Nucleinsäuresequenzen sind dem Fachmann bekannt. Eine Neusynthese kann z.B. dadurch erfolgen, dass zunächst einzelne Nucleinsäureoligonucleotide synthetisiert werden, diese mit zu ihnen komplementären Oligonucleotiden hybridisiert werden, so dass sie einen DNA-Doppelstrang ausbilden, bevor die einzelnen doppelsträngigen Oligonucleotide so miteinander ligiert werden, dass die gewünschte Nucleinsäuresequenz erhalten wird. Die Neusynthese von Nucleinsäuresequenzen inklusive der Anpassung der Häufigkeit der Verwendung der Codons an einen bestimmten Zielorganismus kann auch als Auftrag an Firmen, die dieses als Dienstleistung anbieten, vergeben werden (z.B. Entelechon GmbH, Regensburg, Germany).

Der Begriff "Identität" bedeutet im Zusammenhang mit der vorliegenden Erfindung eine Sequenzidentität über die gesamte Länge der codierenden Region eines Nucleinsäuremoleküls oder die gesamte Länge einer Aminosäuresequenz, die ein Protein codiert, von mindestens 60%, insbesondere eine Identität von mindestens 70%, vorzugsweise von mindestens 80%, besonders bevorzugt von mindestens 90%, insbesondere bevorzugt von mindestens 95% und im speziellen bevorzugt von mindestens 98%. Unter dem Begriff "Identität" soll im Zusammenhang mit der vorliegenden Erfindung die Anzahl der übereinstimmenden Aminosäuren/Nucleotide (Identität) mit anderen Proteinen/Nucleinsäuren, ausgedrückt in Prozent verstanden werden. Bevorzugt wird die Identität betreffend ein Protein mit der Aktivität einer Hyaluronansynthase durch Vergleiche der unter SEQ ID NO 2 angegebenen Aminosäuresequenz bzw. die Identität betreffend ein Nucleinsäuremolekül codierend ein Protein mit der Aktivität einer Hyaluronansynthase durch Vergleiche der unter SEQ ID NO 1 oder SEQ ID No 3 angegebenen Nucleinsäuresequenz, die Identität betreffend ein Protein mit der Aktivität einer Chondroitinsynthase durch Vergleiche der unter SEQ ID NO 5 angegebenen Aminosäuresequenz bzw. die Identität betreffend ein Nucleinsäuremolekül codierend ein Protein mit der Aktivität einer Chondroitinsynthase durch Vergleiche der unter SEQ ID NO 4 dargestellten Nucleinsäuresequenz, die Identität betreffend ein Protein mit der Aktivität einer Heparin-/Heparosansynthase durch Vergleiche der unter SEQ ID NO 7 angegebenen Aminosäuresequenz bzw. die Identität betreffend ein Nucleinsäuremolekül codierend ein Protein mit der Aktivität einer Heparin-/Heparosansynthase durch Vergleiche der unter SEQ ID NO 6 dargestellten Nucleinsäuresequenz, die Identität betreffend ein Protein mit der Aktivität einer GlcNAc-6-P Acetyltransferase durch Vergleiche der unter SEQ ID NO 9 angegebenen Aminosäuresequenz bzw. die Identität betreffend ein Nucleinsäuremolekül codierend ein Protein mit der Aktivität einer GlcNAc-6-P Acetyltransferase durch Vergleiche der unter SEQ ID NO 8 dargestellten Nucleinsäuresequenz, die Identität betreffend ein Protein mit der Aktivität einer UDP-GlcNAc Pyrophosphorylase durch Vergleiche der unter SEQ ID NO 11 angegebenen Aminosäuresequenz bzw. die Identität betreffend ein Nucleinsäuremolekül codierend ein Protein mit der Aktivität einer UDP-GlcNAc Pyrophosphorylase durch Vergleiche der unter SEQ ID NO 10 dargestellten Nucleinsäuresequenz zu anderen Proteinen/Nucleinsäuren mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., Nucleic Acids Research 22 (1994), 4673-4680) ermittelt. ClustalW wird öffentlich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Genetique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK), heruntergeladen werden.
Vorzugsweise wird das ClustalW Computerprogramm der Version 1.8 benutzt, um die Identität zwischen im Rahmen der vorliegenden Erfindung beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.
Vorzugsweise wird das ClustalW Computerprogramm der Version 1.8 benutzt, um die Identität zwischen z.B. der Nucleotidsequenz der im Rahmen der vorliegenden Erfindung beschriebenen Nucleinsäuremoleküle und der Nucleotidsequenz von anderen Nucleinsäuremolekülen zu bestimmen. Dabei sind folgende Parameter einzustellen:
KTUPLE=2, TOPDIAGS=4, PAIRGAP=5, DNAMATRIX:IUB, GAPOPEN=10, GAPEXT=5, MAXDIV=40, TRANSITIONS: unweighted.

Identität bedeutet ferner, dass funktionelle und/oder strukturelle Äquivalenz zwischen den betreffenden Nucleinsäuremolekülen oder den durch sie codierten Proteinen, besteht. Bei den Nucleinsäuremolekülen, die homolog zu den oben beschriebenen Molekülen sind und Derivate dieser Moleküle darstellen, handelt es sich in der Regel um Variationen dieser Moleküle, die Modifikationen darstellen, die dieselbe biologische Funktion ausüben. Es kann sich dabei sowohl um natürlicherweise auftretende Variationen handeln, beispielsweise um Sequenzen aus anderen Spezies oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten. Eine spezielle Form von Derivaten stellen z.B. Nucleinsäuremoleküle dar, die auf Grund der Degeneration des genetischen Codes von im Rahmen der vorliegenden Erfindung beschriebenen Nucleinsäuremolekülen abweichen.

Von verschiedenen Nucleinsäuremoleküle-Derivaten codierte Proteine weisen jeweils bestimmte gemeinsame Charakteristika auf.
Dazu können z.B. biologische Aktivität, Substratspezifität, Molekulargewicht, immunologische Reaktivität, Konformation etc. gehören.

Ein weiterer Gegenstand der vorliegenden Erfindung betrifft erfindungsgemäße genetisch modifizierte Pflanzenzellen oder erfindungsgemäße genetisch modifizierte Pflanzen, dadurch gekennzeichnet, dass die in das Genom der Pflanzenzelle oder der Pflanze integrierten fremden Nucleinsäuremoleküle, codierend eine Glucosaminglycansynthase und codierend ein Protein mit der Aktivität einer GlcNAc-6-P Acetyltransferase und/oder codierend ein Protein mit der Aktivität einer UDP-GlcNAc Acetyltransferase mit regulatorischen Elementen verknüpft sind, die die Transkription in Pflanzenzellen initiieren (Promotoren). Es kann sich um homologe oder heterologe Promotoren handeln. Bei den Promotoren kann es sich um konstitutive, gewebespezifische, entwicklungsspezifische oder um durch äußere Einflüsse (z.B. nach Applikation chemischer Substanzen, durch Einwirkung abiotischer Faktoren wie Hitze und/oder Kälte, Trockenheit, Krankheitsbefall etc.) regulierte Promotoren handeln. Dabei können Nucleinsäuremoleküle, codierend eine Glucosaminglycansynthase oder ein Protein mit der Aktivität einer GlcNAc-6-P Acetyltransferase oder ein Protein mit der Aktivität einer UDP-GlcNAc Pyrophosphorylase, die in das Genom einer erfindungsgemäßen genetisch modifizierten Pflanzenzelle oder einer erfindungsgemäßen genetisch modifizierten Pflanze integriert sind, mit jeweils demselben Promotor verknüpft sein, oder es können unterschiedliche Promotoren mit den einzelnen Sequenzen verknüpft sein. Es können dabei zwei oder drei verschiedene Promotoren in jeder beliebigen Kombination jeweils verknüpft mit einem betreffenden fremden Nucleinsäuremolekül codierend eine Glucosaminglycansynthase oder ein Protein mit der Aktivität einer GlcNAc-6-P Acetyltransferase oder ein Protein mit der Aktivität einer UDP-GlcNAc Pyrophosphorylase in einer erfindungsgemäßen genetisch modifizierten Pflanzezelle oder einer erfindungsgemäßen genetisch modifizierten Pflanze vorliegen.

Eine bevorzugte Ausführungsform der vorliegenden Erfindung betrifft erfindungsgemäße genetisch modifizierte Pflanzenzellen oder erfindungsgemäße genetisch modifizierte Pflanzen, wobei mindestens ein fremdes Nucleinsäuremolekül, besonders bevorzugt mindestens zwei fremde Nucleinsäuremoleküle, insbesondere bevorzugt drei fremde Nucleinsäuremoleküle ausgewählt aus der Gruppe von Nucleinsäuremolekülen codierend eine Glucosaminglycansynthase oder ein Protein mit der Aktivität einer GlcNAc-6-P Acetyltransferase oder ein Protein mit der Aktivität einer UDP-GlcNAc Pyrophosphorylase mit einem gewebespezifische Promotoren verknüpft ist (sind). Bevorzugte gewebespezifische Promotoren stellen Promotoren dar, die die Initiation der Transkription spezifisch in pflanzlichen Knollen-, Frucht-oder Samenzellen oder Blättern initiieren.

Generell kommt für die Expression von Nucleinsäuremolekülen, die eine Glucosaminglycansynthase oder ein Protein mit der Aktivität einer GlcNAc-6-P Acetyltransferase oder ein Protein mit der Aktivität einer UDP-GlcNAc Pyrophosphorylase codieren jeder in pflanzlichen Zellen aktive Promotor in Frage.
Der Promotor kann dabei so gewählt sein, dass die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt. Sowohl in Bezug auf die Pflanze als auch in Bezug auf das zu exprimierende Nucleinsäuremolekül kann der Promotor homolog oder heterolog sein.
Geeignete Promotoren sind z.B. der Promotor der 35S RNA des Cauliflower Mosaic Virus, der Ubiquitin-Promotor aus Mais (Christensen und Quail, 1996, Transgenic Research 5(3), 213-218), der Kafirin Promotor aus Hirse (DeRose et al., 1996, Plant Molecular Biology 32 1029-1035; Mishra et al., 2007, Molecular Biology Reports online: 2 February 2007, DOI: 10.1007/s11033-007-9056-8) oder der *Cestrum* YLCV (Yellow Leaf Curling Virus; WO 01 73087; Stavolone et al., 2003, Plant Mol. Biol. 53, 703-713) für eine konstitutive Expression, der Patatingen-Promotor B33 (Rocha-Sosa et al., EMBO J. 8 (1989), 23-29) für eine knollenspezifische Expression in Kartoffeln oder ein fruchtspezifischer Promotor für Tomate wie z.B. der Polygalacturonase Promotor aus Tomate (Montgomery et al., 1993, Plant Cell 5, 1049-1062) oder der E8 Promotor aus Tomate (Metha et al., 2002, Nature Biotechnol. 20(6), 613-618) oder der ACC Oxidase Promotor aus Pfirsich (Moon und Callahan, 2004, J. Experimental Botany 55 (402), 1519-1528) oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451) oder für eine endospermspezifische Expression der HMWG-Promotor aus Weizen, der USP-Promotor, der Phaseolinpromotor, Promotoren von Zein-Genen aus Mais (Pedersen et al., Cell 29 (1982), 1015-1026; Quatroccio et al., Plant Mol. Biol. 15 (1990), 81-93), Glutelin-Promotor (Leisy et al., Plant Mol. Biol. 14 (1990), 41-50; Zheng et al., Plant J. 4 (1993), 357-366; Yoshihara et al., FEBS Lett. 383 (1996), 213-218) oder Shrunken-1 Promotor (Werr et al., EMBO J. 4 (1985), 1373-1380). Es können jedoch auch Promotoren verwendet werden, die nur zu einem durch äußere Einflüsse determinierten Zeitpunkt aktiviert werden (siehe beispielsweise WO 9307279). Von besonderem Interesse können hierbei Promotoren von heat-shock Proteinen sein, die eine einfache Induktion erlauben. Ferner können samenspezifische Promotoren verwendet werden, wie z.B. der USP-Promoter aus *Vicia faba,* der eine samenspezifische Expression in *Vicia faba* und anderen Pflanzen gewährleistet (Fiedler et al., Plant Mol. Biol. 22 (1993), 669-679; Bäumlein et al., Mol. Gen. Genet. 225 (1991), 459-467).
Auch die Verwendung von Promotoren, die im Genom von Algen infizierenden Viren vorkommen, sind für die Expression von Nucleinsäuresequenzen in Pflanzen geeignet (Mitra et al., 1994, Biochem. Biophys Res Commun 204(1), 187-194; Mitra und Higgins, 1994, Plant Mol Biol 26(1), 85-93, Van Etten et al., 2002, Arch Virol 147, 1479-1516)

Unter dem Begriff "gewebespezifisch" soll im Zusammenhang mit der vorliegenden Erfindung die überwiegende Beschränkung einer Ausprägung (z.B. die Initiation der Transkription) auf ein bestimmtes Gewebe verstanden werden.

Unter den Begriffen "Knollen-, Frucht-, oder Samenzelle" sollen im Zusammenhang mit der vorliegenden Erfindung alle Zellen verstanden werden, die in einer Knolle, Frucht bzw. in einem Samen enthalten sind.

Unter dem Begriff "homologer Promotor" soll im Zusammenhang mit der vorliegenden Erfindung ein Promotor verstanden werden, der natürlicherweise in Pflanzenzellen oder Pflanzen vorkommt, die für die Herstellung erfindungsgemäßer genetisch modifizierter Pflanzenzellen bzw. erfindungsgemäßer genetisch modifizierter Pflanzen verwendet wurden (Homolog bezüglich der Pflanzenzelle oder Pflanze) oder ein Promotor verstanden werden, der die Regulation der Expression eines Gens in dem Organismus reguliert, aus dem die Sequenz isoliert wurde (homolog bezüglich des zu exprimierenden Nucleinsäuremoleküls).

Unter dem Begriff "heterologer Promotor" soll im Zusammenhang mit der vorliegenden Erfindung ein Promotor verstanden werden, der natürlicherweise nicht in Pflanzenzellen oder Pflanzen vorkommt, die für die Herstellung erfindungsgemäßer genetisch modifizierter Pflanzenzellen bzw. erfindungsgemäßer genetisch modifizierter Pflanzen verwendet wurden (heterolog bezüglich der Pflanzenzelle oder Pflanze) oder ein Promotor verstanden werden, der natürlicherweise in dem Organismus, aus dem eine zu exprimierende Nucleinsäuresequenz isoliert wurde, nicht zur Regulation der Expression besagter Nucleinsäuresequenz vorkommt (heterolog bezüglich des zu exprimierenden Gens).

Ferner kann eine Terminationssequenz (Polyandenylierungssignal) vorhanden sein, die der Addition eines Poly-A-Schwanzes an das mRNA Transkript des Nucleinsäuremoleküls dient. Dem Poly-A-Schwanz wird eine Funktion bei der Stabilisierung der Transkripte beigemessen. Derartige Elemente sind in der Literatur beschrieben (vgl. Gielen et al., EMBO J. 8 (1989), 23-29) und beliebig austauschbar.

Es können auch Intronsequenzen zwischen dem Promotor und der codierenden Region oder in dem fremden Nucleinsäuremolekül, codierend ein Protein, vorhanden sein. Solche Intronsequenzen können zur Stabilität der Expression und zu einer erhöhten Expression in Pflanzen führen (Callis et al., 1987, Genes Devel. 1, 1183-1200; Luehrsen, and Walbot, 1991, Mol. Gen. Genet. 225, 81-93; Rethmeier et al., 1997; Plant Journal 12(4), 895-899; Rose and Beliakoff, 2000, Plant Physiol. 122 (2), 535-542; Vasil et al., 1989, Plant Physiol. 91, 1575-1579; XU et al., 2003, Science in China Series C Vol.46 No.6, 561-569). Geeignete Intronsequenzen sind beispielsweise das erste Intron des sh1-Gens aus Mais, das erste Intron des Poly-Ubiquitin Gens 1 aus Mais, das erste Intron des EPSPS Gens aus Reis oder eines der beiden ersten Introns des PAT1 Gens aus *Arabidopsis.*

Ein weiterer Gegenstand der vorliegenden Erfindung betrifft Pflanzen, enthaltend erfindungsgemäße genetisch modifizierte Pflanzenzellen. Derartige Pflanzen können durch Regeneration aus erfindungsgemäßen genetisch modifizierten Pflanzenzellen erzeugt werden.

In einer weiteren Ausführungsform betrifft die vorliegende Erfindung erntebare Pflanzenteile erfindungsgemäßer genetisch modifizierter Pflanzen, wie Früchte, Speicherwurzeln, Wurzeln, Blüten, Knospen, Sprosse, Blätter oder Stämme, vorzugsweise Samen, Früchte oder Knollen, wobei diese erntebaren Teile erfindungsgemäße genetisch modifizierte Pflanzenzellen enthalten.

In einer vorzugsweisen Ausführungsform der vorliegenden Erfindung handelt es sich bei den erfindungsgemäßen erntebaren Pflanzenteilen um verarbeitbare oder konsumierbare Teile von erfindungsgemäßen genetisch modifizierten Pflanzen, enthaltend erfindungsgemäße genetisch modifizierte Pflanzenzellen.

Unter dem Begriff "verarbeitbare Teile" sollen im Zusammenhang mit der vorliegenden Erfindung Pflanzenteile verstanden werden, die Verwendung in der Herstellung von Nahrungs- oder Futtermitteln finden, die als Rohstoffquelle für industrielle Prozesse, als Rohstoffquelle für die Herstellung pharmazeutischer oder als Rohstoffquelle für die Herstellung kosmetischer Produkte eingesetzt werden.

Unter dem Begriff "konsumierbare Teile" sollen im Zusammenhang mit der vorliegenden Erfindung Pflanzenteile verstanden werden, die dem Menschen als Nahrungsmittel dienen oder als Tierfutter verwendet werden.

Die vorliegende Erfindung betrifft auch Vermehrungsmaterial erfindungsgemäßer genetisch modifizierter Pflanzen, enthaltend eine erfindungsgemäße genetisch modifizierte Pflanzenzelle.

Der Begriff "Vermehrungsmaterial" umfasst dabei jene Bestandteile der Pflanze, die geeignet sind zur Erzeugung von Nachkommen auf vegetativem oder sexuellem Weg. Für die vegetative Vermehrung eignen sich beispielsweise Stecklinge, Calluskulturen, Rhizome oder Knollen, aber z.B. auch Protoplasten und Zellkulturen. Mittels sexueller Vorgänge entstandenes Vermehrungsmaterial umfasst beispielsweise Körner, Früchte, Samen, Sämlinge, ,etc. Vorzugsweise handelt es sich bei dem Vermehrungsmaterial um Knollen, Früchte oder Samen.

Bevorzugt betrifft die vorliegende Erfindung erfindungsgemäßes Vermehrungsmaterial oder erfindungsgemäße erntebare Teile von Pflanzen, enthaltend Glucosaminglycan (z.B. Hyaluronan). Besonders bevorzugt handelt es sich bei dem erfindungsgemäßen Vermehrungsmaterial oder erfindungsgemäßen erntebaren Teilen von Pflanzen um erfindungsgemäßes Vermehrungsmaterial oder erfindungsgemäße erntebare Teile von Pflanzen, die Glucosaminglycan synthetisieren. Vorzugsweise handelt es sich bei besagtem Glucosaminglycan um Chondroitin, Heparin/Heparan oder Hyaluronan, insbesondere bevorzugt um Hyaluronan.

Der Begriff "Kartoffelpflanze" oder "Kartoffel" meint im Zusammenhang mit der vorliegenden Erfindung Pflanzenspezies der Gattung *Solanum,* besonders Knollen produzierende Spezies der Gattung *Solanum* und insbesondere *Solanum tuberosum.*

Der Begriff "Tomatenpflanze" oder "Tomate" meint im Zusammenhang mit der vorliegenden Erfindung Pflanzenspezies der Gattung *Lycopersicon,* besonders *Lycopersicon esculentum.*

Ein weiterer Vorteil der vorliegenden Erfindung liegt darin, dass erntebare Teile, Vermehrungsmaterial, verarbeitbare Teile oder konsumierbare Teile von erfindungsgemäßen genetisch modifizierten Pflanzen mehr Glucosaminglycan (z.B. Hyaluronan) enthalten im Vergleich zu Pflanzen, die nur ein fremdes Nucleinsäuremolekül, codierend eine Glucosaminsynthase aufweisen. Erfindungsgemäße genetisch modifizierte Pflanzen sind daher nicht nur besonders geeignet zur Verwendung als Rohmaterial, aus dem Glucosaminglycan (z.B. Hyaluronan) isoliert werden kann, sondern auch als direkte Verwendung als Nahrungs-/Futtermittel oder zur Herstellung von Nahrungs-/Futtermitteln, welche einen vorbeugenden oder therapeutischen Charakter aufweisen (z.B. zur Vorbeugung gegen Osteoarthritis, US 6,607,745). Da erfindungsgemäße genetisch modifizierte Pflanzen einen höheren Glucosaminglycangehalt aufweisen als Pflanzen, die nur ein fremdes Nucleinsäuremolekül, codierend eine Glucosaminglycansynthase aufweisen, müssen geringere Mengen an erntebaren Teilen, Vermehrungsmaterial, verarbeitbaren Teilen oder konsumierbaren Teilen von erfindungsgemäßen genetisch modifizierten Pflanzen eingesetzt werden, um solche Nahrungs-/Futtermittel herzustellen. Werden konsumierbare Teile von erfindungsgemäßen genetisch modifizierten Pflanzen z.B. direkt als so genanntes Nahrungsergänzungsmittel ("Nutraceutical") konsumiert, so ist es möglich einen positiven Effekt bereits durch den Verzehr geringerer Mengen an Substanz zu erreichen. Dieses kann u.a. bei der Herstellung von Tierfutter eine besondere Bedeutung erlangen, da Tierfutter mit einem zu hohen Gehalt an pflanzlichen Bestandteilen, für verschiedenste Tierarten nicht als Futtermittel geeignet ist.
Durch die hohe Wasserbindungskapazität von Glucosaminglycanen, insbesondere von Hyaluronan weisen erntebare Teile, Vermehrungsmaterial, verarbeitbare Teile oder konsumierbare Teile von erfindungsgemäßen genetisch modifizierten Pflanzen weiterhin den Vorteil auf, dass bei der Herstellung von verfestigten Nahrungs-/Futtermitteln weniger Dickungsmittel eingesetzt werden müssen. So kann z.B. bei der Herstellung von Gelee weniger Zucker verwendet werden, was einen zusätzlichen positiven Gesundheitseffekt mit sich bringt. Bei der Herstellung von Nahrungs-/Futtermitteln, bei welchen es notwendig ist, dem pflanzlichen Rohstoff Wasser zu entziehen, besteht der Vorteil bei der Verwendung von erntebaren Teilen, Vermehrungsmaterial, verarbeitbaren Teilen oder konsumierbaren Teilen von erfindungsgemäßen genetisch modifizierten Pflanzen darin, dass dem betreffenden Pflanzenmaterial weniger Wasser entzogen werden muss, was zu geringern Produktionskosten führt und durch schonendere Herstellungsverfahren (z.B. geringere und/oder kürzere Wärmezufuhr) einen erhöhten Nährwert der betreffenden Nahrungs-/Futtermittel gewährleistet. So muss z.B. bei der Herstellung von Tomaten Ketchup weniger Energie zugeführt werden, um die gewünschte Konsistenz zu erreichen.

Ein weiterer Gegenstand der vorliegenden Erfindung betrifft ein Verfahren zur Herstellung einer Pflanze, worin
a) eine Pflanzenzelle, genetisch modifiziert wird, wobei die genetische Modifikation die folgenden Schritte i bis iii umfasst
   i) Einführung eines fremden Nucleinsäuremoleküls, codierend eine Glucosaminglycansynthase in eine Pflanzenzelle
   ii) Einführung eines fremden Nucleinsäuremoleküls, codierend eine Glucosamin-6-Phosphat-Acetyltransferase in eine Pflanzenzelle
   iii) Einführung eines fremden Nucleinsäuremoleküls, codierend eine UDP-N-acety-Glucosamin Pyrophosphorylase in eine Pflanzenzelle
   wobei die Schritte i bis iii in beliebiger Reihenfolge, einzeln oder beliebige Kombinationen der Schritte i bis iii gleichzeitig durchgeführt werden können
b) aus Pflanzenzellen von Schritt a)i und/oder a)ii und/oder a)iii eine Pflanze regeneriert wird;
c) gegebenenfalls weitere Pflanzen mit Hilfe der Pflanzen nach Schritt b) erzeugt werden,
wobei gegebenenfalls aus Pflanzen gemäß den Schritten b) oder c) Pflanzenzellen isoliert werden und die Verfahrensschritte a) bis c) solange wiederholt werden, bis eine Pflanze erzeugt wurde, die ein fremdes Nucleinsäuremolekül, codierend eine Glucosaminglycansynthase und ein fremdes Nucleinsäuremolekül codierend ein Protein mit der Aktivität einer Glucosamin-6-Phosphat-Acetyltransferase und ein fremdes Nucleinsäuremolekül codierend ein Protein mit der Aktivität einer UDP-GlcNAc Pyrophosphorylase aufweist.

Eine bevorzugte Ausführungsform des erfindungsgemäßen Verfahrens zur Herstellung einer Pflanze betrifft ein Verfahren zur Herstellung einer Pflanze, worin
a) eine Pflanzenzelle, genetisch modifiziert wird, wobei die genetische Modifikation die folgenden Schritte i bis iii in beliebiger Reihenfolge umfasst oder beliebige Kombinationen der folgenden Schritte i bis iii einzeln oder gleichzeitig durchgeführt werden
   i) Einführung eines fremden Nucleinsäuremoleküls, codierend eine Glucosaminglycansynthase in die Pflanzenzelle
   ii) Einführung eines fremden Nucleinsäuremoleküls, codierend eine Glucosamin-6-Phosphat-Acetyltransferase in eine Pflanzenzelle
   iii) Einführung eines fremden Nucleinsäuremoleküls, codierend eine UDP-GIcNAc Pyrophosphorylase in eine Pflanzenzelle
b) aus Pflanzenzellen enthaltend die genetische Modifikation gemäß den Schritten
   i) a) i
   ii) a) ii
   iii) a) iii
   iv) a) i und a) ii,
   v) a) i und a) iii,
   vi) a) ii und a) iii, oder
   vii) a) i und a) ii und a) iii
   eine Pflanze regeneriert wird
c) in Pflanzenzellen von Pflanzen gemäß Schritt
   i) b) i eine genetische Modifikation gemäß Schritt a) ii,
   ii) b) i eine genetische Modifikation gemäß Schritt a) iii,
   iii) b) i eine genetische Modifikation gemäß Schritt a) ii und gleichzeitig eine genetische Modifikation gemäß Schritt a) iii,
   iv) b) ii eine genetische Modifikation gemäß Schritt a) i,
   v) b) ii eine genetische Modifikation gemäß Schritt a) iii,
   vi) b) ii eine genetische Modifikation gemäß Schritt a) i und gleichzeitig eine genetische Modifikation gemäß Schritt a) iii,
   vii) b) iii eine genetische Modifikation gemäß Schritt a) i,
   viii) b) iii eine genetische Modifikation gemäß Schritt a) ii,
   ix) b) iii eine genetische Modifikation gemäß Schritt a) i und gleichzeitig eine genetische Modifikation gemäß Schritt a) ii,
   x) b) iv eine genetische Modifikation gemäß Schritt a) iii,
   xi) b) v eine genetische Modifikation gemäß Schritt a) ii, oder
   xii) b) vi eine genetische Modifikation gemäß Schritt a) i
   eingeführt und eine Pflanze regeneriert wird
d) in Pflanzenzellen von Pflanzen gemäß Schritt
   i) c) i eine genetische Modifikation gemäß Schritt a) iii,
   ii) c) ii eine genetische Modifikation gemäß Schritt a) ii,
   iii) c) iv eine genetische Modifikation gemäß Schritt a) iii,
   iv) c) v eine genetische Modifikation gemäß Schritt a) ii,
   v) c) vii eine genetische Modifikation gemäß Schritt a) ii,
   vi) c) vii eine genetische Modifikation gemäß Schritt a) i, oder
   vii) c) ix eine genetische Modifikation gemäß Schritt a) ii
   eingeführt und eine Pflanze regeneriert wird
e) gegebenenfalls weitere Pflanzen mit Hilfe der Pflanzen nach einem der Schritte b) vii c) iii, c) vi, c) x, c) xi, c) xii oder nach einem der Schritte d) i bis d) vii erzeugt werden.

Bei Einführung von fremden Nucleinsäuremolekülen laut Schritt a) der erfindungsgemäßen Verfahren zur Herstellung einer Pflanze kann es sich um jegliche zur Verfügung stehende Technik handeln. Unterschiedliche Verfahren zur Transformation von Pflanzenzellen wurden bereits oben beschrieben und sind hier entsprechend anzuwenden. Werden die Verfahrensschritte gemäß Schritt a) der erfindungsgemäßen Verfahren zur Herstellung einer Pflanze nicht gleichzeitig, sondern hintereinander durchgeführt, so können für die einzelnen Transfromationsschritte gleiche oder unterschiedliche Techniken verwendet werden.

Die Regeneration der Pflanzen gemäß Schritt b) und gegebenenfalls Schritt c) und d) der erfindungsgemäßen Verfahren zur Herstellung einer Pflanze kann nach dem Fachmann bekannten Methoden erfolgen (z.B. beschrieben in "Plant Cell Culture Protocols", 1999, edt. by R.D. Hall, Humana Press, ISBN 0-89603-549-2).

Die Erzeugung weiterer Pflanzen (je nach Verfahren gemäß Schritt c) oder Schritt e)) der erfindungsgemäßen Verfahren zur Herstellung einer Pflanze kann z.B. erfolgen durch vegetative Vermehrung (beispielsweise über Stecklinge, Knollen oder über Calluskultur und Regeneration ganzer Pflanzen) oder durch sexuelle Vermehrung. Die sexuelle Vermehrung findet dabei vorzugsweise kontrolliert statt, d.h. es werden ausgewählte Pflanzen mit bestimmten Eigenschaften miteinander gekreuzt und vermehrt. Die Auswahl erfolgt dabei bevorzugt in der Weise, dass die weiteren Pflanzen (die je nach Verfahren gemäß Schritt c) oder Schritt e) erzeugt werden) die in den vorangegangenen Schritten eingeführten fremden Nucleinsäuremoleküle aufweisen.

In erfindungsgemäßen Verfahren zur Herstellung von Pflanzen können die genetischen Modifikationen zur Erzeugung der erfindungsgemäßen genetisch modifizierten Pflanzenzellen gleichzeitig oder in aufeinander folgenden Schritten und in jeder beliebigen Kombination erfolgen. Es kann sowohl von Wildtyp-Pflanzen bzw. Wildtyp-Pflanzenzellen ausgegangen werden, in die noch keine Einführung eines fremden Nucleinsäuremoleküls erfolgt ist oder es kann von bereits genetisch modifizierten Pflanzenzellen bzw. Pflanzen, in die bereits ein oder mehrere fremde Nucleinsäuremoleküle, eingeführt wurden, ausgegangen werden.

Bei der genetischen Modifikation des Einbringens der fremden Nucleinsäuremoleküle in die Pflanzezelle oder Pflanze, kann es sich in Schritt a) der erfindungsgemäßen Verfahren zur Herstellung einer Pflanze, um ein einzelnes Nucleinsäuremolekül oder um mehrere Nucleinsäuremoleküle handeln. So können die fremden Nucleinsäuremoleküle, codierend eine Glucosaminglycansynthase bzw. codierend ein Protein mit der enzymatischen Aktivität einer GlcNAc-6-P Acetyltransferase bzw. codierend ein Protein mit der enzymatischen Aktivität einer UDP-GlcNAc Pyrophosphorylase zusammen auf einem einzigen Nucleinsäuremolekül vorliegen oder es können zwei der genannten fremden Nucleinsäuremoleküle zusammen auf einem einzigen Nucleinsäuremolekül und das dritte fremde Nucleinsäuremolekül auf einem andern Nucleinsäuremolekül in jeder möglichen Kombination vorliegen, oder es können alle drei genannten fremden Nucleinsäuremoleküle jeweils auf einzelnen, getrennten Nucleinsäuremolekülen vorliegen.

Bevorzugte Eigenschaften von fremden Nucleinsäuremolekülen oder rekombinanten Nucleinsäuremolekülen wurden bereits oben im Zusammenhang mit erfindungsgemäßen Pflanzezellen bzw. erfindungsgemäßen Pflanzen beschrieben und sind zur Durchführung erfindungsgemäßer Verfahren zur Herstellung einer Pflanze entsprechend anwendbar.

In einer weiteren bevorzugten Ausführungsform werden erfindungsgemäße Verfahren zur Herstellung einer Pflanze, zur Herstellung erfindungemäßer genetisch modifizierter Pflanzen verwendet.

Pflanzen erhältlich nach erfindungsgemäßen Verfahren zur Herstellung einer Pflanze, die Hyaluronan synthetisiert, sind ebenfalls Gegenstand der vorliegenden Erfindung.

Ein weiterer Gegenstand der vorliegenden Erfindung betrifft ein Verfahren zur Herstellung von Glucosaminglycanen (z.B. Hyaluronan), umfassend den Schritt der Extraktion von Glucosaminglycanen aus erfindungsgemäßen genetisch modifizierten Pflanzenzellen, aus erfindungsgemäßen genetisch modifizierten Pflanzen, aus erfindungsgemäßem Vermehrungsmaterial, aus erfindungsgemäßen erntebaren Pflanzenteilen oder aus Pflanzen oder Teilen dieser Pflanzen, erhältlich nach einem erfindungsgemäßen Verfahren zur Herstellung von Pflanzen. Vorzugsweise umfasst ein solches Verfahren auch den Schritt des Erntens der kultivierten erfindungsgemäßen genetisch modifizierten Pflanzenzellen, der erfindungsgemäßen genetisch modifizierten Pflanzen, des erfindungsgemäßen Vermehrungsmaterials, der erfindungsgemäßen erntebaren Pflanzeteile, der erfindungsgemäßen verarbeitbaren Pflanzenteile vor der Extraktion des Glucosaminglycans (z.B. Hyaluronans) und besonders bevorzugt ferner den Schritt der Kultivierung erfindungsgemäßer genetisch modifizierten Pflanzenzellen oder erfindungsgemäßer genetisch modifizierten Pflanzen vor dem Ernten.

Im Gegensatz zu bakteriellen oder tierischen Geweben weisen pflanzliche Gewebe keine Glucosaminglycan abbauenden Enzyme (z.B. Hyaluronidasen) auf. Daher ist wie oben bereits beschrieben die Extraktion von Glucosaminglycanen aus pflanzlichen Geweben mit Hilfe relativ einfacher Verfahren möglich. Die oben beschriebenen wässrigen Extrakte aus pflanzlichen Zellen oder Geweben, enthaltend Glucosaminglycane, können bei Bedarf mit dem Fachmann bekannten Methoden, wie z.B. von mehrfach hintereinander folgenden Fällungen mit Ethanol, weiter aufgereinigt werden. Eine bevorzugte Methode zur Aufreinigung von Hyaluronan ist unter Allgemeine Methoden Punkt 3 beschrieben.

Die bereits beschriebenen Verfahren zur Extraktion von Glucosaminglycanen aus erfindungsgemäßen genetisch modifizierten Pflanzenzellen oder erfindungsgemäßen genetisch modifizierten Pflanzen sind auch für die Isolierung von Glucosaminglycanen (z.B. Hyaluronan) aus erfindungsgemäßem Vermehrungsmaterial, aus erfindungsgemäßen erntebaren Pflanzenteilen oder aus Pflanzen oder Teilen dieser Pflanzen, erhältlich nach einem erfindungsgemäßen Verfahren zur Herstellung von Pflanzen, die Hyaluronan synthetisieren, anwendbar.

Gegenstand der vorliegenden Erfindung ist auch die Verwendung erfindungsgemäßer genetisch modifizierter Pflanzenzellen, erfindungsgemäßer genetisch modifizierter Pflanzen, erfindungsgemäßen Vermehrungsmaterials, erfindungsgemäßen erntebaren Pflanzeteilen oder von Pflanzen, erhältlich nach einem erfindungsgemäßen Verfahren zur Herstellung einer Pflanze, zur Herstellung von Glucosaminglycanen.

Ein weiterer Gegenstand der vorliegenden Erfindung betrifft Zusammensetzungen, enthaltend erfindungsgemäße genetisch modifizierte Pflanzenzellen. Es ist dabei unerheblich, ob die Pflanzenzellen intakt oder nicht mehr intakt sind, weil sie z.B. durch Prozessierungsverfahren zerstört wurden. Bevorzugt handelt es sich bei den Zusammensetzungen um Nahrungs- oder Futtermittel, um pharmazeutische oder um kosmetische Produkte.

Ein bevorzugter Gegenstand der vorliegenden Erfindung betrifft Zusammensetzungen, enthaltend Bestandteile von erfindungsgemäßen genetisch modifizierten Pflanzenzellen, von erfindungsgemäßen genetisch modifizierten Pflanzen, von erfindungsgemäßem Vermehrungsmaterial, von erfindungsgemäßen erntebaren Pflanzenteilen oder von Pflanzen, erhältlich nach einem erfindungsgemäßen Verfahren und enthaltend rekombinante Nucleinsäuremoleküle, wobei die rekombinanten Nucleinsäuremoleküle dadurch gekennzeichnet sind, dass sie Nucleinsäuremoleküle umfassen, die eine Glucosaminsynthase und Proteine mit der enzymatischen Aktivität einer GIcNAc-6-P Acetyltransferase und Proteine mit der enzymatischen Aktivität einer UDP-GlcNAc Pyrophosphorylase codieren.

Eine stabile Integration von fremden Nucleinsäuremolekülen in das Genom einer Pflanzenzelle oder Pflanze führt dazu, dass die fremden Nucleinsäuremoleküle nach Integration in das Genom der Pflanzenzelle oder Pflanze von genomischen pflanzlichen Nucleinsäuresequenzen flankiert sind.
In einer bevorzugten Ausführungsform sind erfindungsgemäße Zusammensetzungen daher dadurch gekennzeichnet, dass die rekombinanten Nucleinsäuremoleküle, enthaltend in einer erfindungsgemäßen Zusammensetzung von genomischen pflanzlichen Nucleinsäuresequenzen flankiert sind.
Die genomischen pflanzlichen Nucleinsäuresequenzen können dabei beliebige Sequenzen sein, die im Genom der Pflanzenzelle oder Pflanze, die zur Herstellung der Zusammensetzung verwendet wurde, natürlicherweise vorliegen.

Bei den in erfindungsgemäßen Zusammensetzungen enthaltenden rekombinanten Nucleinsäuremolekülen kann es sich um einzelne oder verschiedene rekombinante Nucleinsäuremoleküle handeln, bei welchen Nucleinsäuremoleküle, die eine Glucosaminglycansynthase (z.B. Hyaluronansynthase) und Proteine mit der Aktivität einer GlcN-6-P Acetyltransferase und Proteine mit der Aktivität einer UDP-GIcNAc Pyrophosphorylase codieren, auf einem Nucleinsäuremolekül vorliegen, oder um solche, bei welchen die genannten Nucleinsäuremoleküle auf getrennten Nucleinsäuremolekülen vorliegen, handeln. Es können Nucleinsäuremoleküle, codierend eine Glucosaminglycansynthase (z.B. Hyaluronansynthase) bzw. codierend ein Protein mit der Aktivität einer GIcN-6-P Acetyltransferase bzw. codierend ein Protein mit der Aktivität einer UDP-GlcNAc Pyrophosphorylase zusammen auf einem einzigen rekombinanten Nucleinsäuremolekül vorliegen oder es können zwei der genannten Nucleinsäuremoleküle zusammen auf einem einzigen rekombinanten Nucleinsäuremolekül und das dritte Nucleinsäuremolekül auf einem anderen rekombinanten Nucleinsäuremolekül in jeder möglichen Kombination vorliegen, oder es können alle drei genannten Nucleinsäuremoleküle jeweils auf einzelnen, getrennten rekombinanten Nucleinsäuremolekülen vorliegen. Je nachdem, in welcher Weise die Nucleinsäuremoleküle, codierend eine Glucosaminglycansynthase (z.B. Hyaluronansynthase) bzw. codierend ein Protein mit der Aktivität einer GlcN-6-P Acetyltransferase bzw. codierend ein Protein mit der Aktivität einer UDP-GlcNAc Pyrophosphorylase in einer erfindungsgemäßen Zusammensetzung vorliegen, können sie von identischen oder von unterschiedlichen genomischen pflanzlichen Nucleinsäuresequenzen flankiert sein.

Dass erfindungsgemäße Zusammensetzungen rekombinante Nucleinsäuremoleküle enthalten, kann mit dem Fachmann bekannten Methoden, wie z.B. auf Hybridisierung basierenden Methoden oder bevorzugt mit auf PCR (Polymerase Chain Reaction) basierenden Methoden nachgewiesen werden.

Wie oben bereits ausgeführt können erfindungsgemäße genetisch modifizierte Pflanzenzellen, erfindungsgemäße genetisch modifizierte Pflanzen, erfindungsgemäßes Vermehrungsmaterial, erfindungsgemäße erntebare Pflanzeteile, oder Pflanzen, erhältlich nach einem erfindungsgemäßen Verfahren verwendet werden, um Nahrungs- oder Futtermittel herzustellen. Aber auch die Verwendung als Rohstoff für technische Anwendungen ist möglich, ohne dass Glucosaminglycane (z.B. Hyaluronan) isoliert werden müssen. So können z.B. erfindungsgemäße genetisch modifizierte Pflanzen bzw. Teile von erfindungsgemäßen genetisch modifizierten Pflanzen auf Ackerflächen aufgebracht werden, um eine erhöhte Wasserbindung des Bodens zu erreichen. Weiterhin ist eine Verwendung von erfindungsgemäßen genetisch modifizierten Pflanzen oder erfindungsgemäßen genetisch modifizierten Pflanzenzellen zur Herstellung von Trocknungsmitteln (z.B. für die Verwendung beim Versand von Gegenständen, die empfindlich auf Feuchtigkeit reagieren) oder als Absorber von Flüssigkeiten (z.B. in Babywindeln, oder zum Aufsaugen ausgelaufener wässriger Flüssigkeiten) möglich. Für solche Anwendungen können je nach Bedarf ganze erfindungsgemäße genetisch modifizierte Pflanzen, Teile von erfindungsgemäßen genetisch modifizierten Pflanzen oder zerkleinerte (z.B. zermahlene) erfindungsgemäße genetisch modifizierten Pflanzen oder erfindungsgemäße Pflanzenteile Verwendung finden. Insbesondere für Gebiete, in denen gemahlene Pflanzen oder Pflanzenteile zur Anwendung kommen, sind Pflanzenteile, die Glucosaminglycane (z.B. Hyaluronan) enthalten, jedoch selbst einen geringen Wasseranteil aufweisen. Es handelt es sich hierbei bevorzugt um Körner von Getreidepflanzen (Mais, Reis, Weizen, Roggen, Hafer, Gerste, Sago, oder Sorghum). Da erfindungsgemäße genetisch modifizierte Pflanzenzellen und erfindungsgemäße genetisch modifizierte Pflanzen einen höheren Gehalt an Glucosaminglycanen (z.B. Hyaluronan) aufweisen, als Pflanzen, die nur ein fremdes Nucleinsäuremolekül, codierend eine Glucosaminglycansynthase aufweisen, muss im Vergleich zu diesen für technische Anwendungen weniger Material eingesetzt werden, wenn erfindungsgemäße genetisch modifizierte Pflanzenzellen oder erfindungsgemäße genetisch modifizierte Pflanzen verwendet werden.

Weiterhin sind Gegenstand der vorliegenden Erfindung Verfahren zur Herstellung einer erfindungsgemäßen Zusammensetzung, worin erfindungsgemäße genetisch modifizierte Pflanzenzellen, erfindungsgemäße genetisch modifizierte Pflanzen, erfindungsgemäßes Vermehrungsmaterial, erfindungsgemäße erntebare Pflanzeteile oder Pflanzen, erhältlich nach einem erfindungsgemäßen Verfahren zur Herstellung einer Pflanze, verwendet werden. Bevorzugt handelt es sich bei den Verfahren zur Herstellung einer erfindungsgemäßen Zusammensetzung um Verfahren zur Herstellung von Nahrungs- oder Futtermitteln, Verfahren zur Herstellung eines pharmazeutischen oder Verfahren zur Herstellung zur Herstellung eines kosmetischen Produktes.

Verfahren zur Herstellung von Lebens- oder Futtermitteln sind dem Fachmann bekannt. Verfahren für die Verwendung von erfindungsgemäßen genetisch modifizierten Pflanzen oder erfindungsgemäßen Pflanzenteilen auf technischen Gebieten sind dem Fachmann ebenfalls bekannt und umfassen u.a., sind aber nicht ausschließlich beschränkt auf das Zerkleinern oder das Mahlen von erfindungsgemäßen genetisch modifizierten Pflanzen oder erfindungsgemäßen Pflanzenteilen. Einige Vorteile, die sich aus der Verwendung erfindungsgemäßer Gegenstände für die Herstellung von Nahrungs-/Futtermitteln oder für den Einsatz in technischen Gebieten ergeben, sind bereits oben beschrieben worden.

Besonders bevorzugt handelt es sich bei einem erfindungsgemäßen Verfahren zur Herstellung einer Zusammensetzung um ein Verfahren zur Herstellung einer Zusammensetzung, die Glucosaminglycan (z.B. Hyaluronan) enthält.

Zusammensetzungen erhältlich nach einem Verfahren zur Herstellung einer erfindungsgemäßen Zusammensetzung sind ebenfalls Gegenstand der vorliegenden Erfindung.

Die vorliegende Erfindung betrifft auch die Verwendung von erfindungsgemäßen genetisch modifizierten Pflanzenzellen, erfindungsgemäßen genetisch modifizierten Pflanzen, erfindungsgemäßem Vermehrungsmaterial, erfindungsgemäßen erntebaren Pflanzeteilen oder Pflanzen, erhältlich nach einem erfindungsgemäßen Verfahren zur Herstellung einer Pflanze, zur Herstellung einer erfindungsgemäßen Zusammensetzung. Bevorzugt handelt es sich um die Verwendung von erfindungsgemäßen genetisch modifizierten Pflanzenzellen, erfindungsgemäßen genetisch modifizierten Pflanzen, erfindungsgemäßem Vermehrungsmaterial, erfindungsgemäßen erntebaren Pflanzeteilen oder von Pflanzen, erhältlich nach einem erfindungsgemäßen Verfahren zur Herstellung einer Pflanze, zur Herstellung von Lebens- oder Futtermitteln, zur Herstellung eines Pharmazeutikums oder zur Herstellung eines kosmetischen Produktes.

### Beschreibung der Sequenzen

SEQ ID NO 1: Nucleinsäuresequenz, codierend eine Hyaluronansynthase des *Paramecium bursaria Chlorella* Virus 1.
SEQ ID NO 2: Aminosäuresequenz einer Hyaluronansynthase des *Paramecium bursaria Chlorella* Virus 1. Die dargestellte Aminosäuresequenz kann von SEQ ID NO 1 abgeleitet werden.
SEQ ID NO 3: Synthetische Nucleinsäuresequenz, codierend eine Hyaluronansynthase des *Paramecium bursaria Chlorella* Virus 1. Die Synthese der Codons der dargestellten Sequenz erfolgte in der Weise, dass sie an die Verwendung von Codons in Pflanzenzellen angepasst ist. Die dargestellte Nucleinsäuresequenz codiert ein Protein mit der unter SEQ ID No 2 dargestellten Aminosäuresequenz.
SEQ ID NO 4: Nucleinsäuresequenz, codierend ein Protein mit der Aktivität einer Chondroitinsynthase aus *Pasteurella multocida*.
SEQ ID NO 5: Aminosäuresequenz eines Proteins mit der Aktivität einer Chondroitinsynthase aus *Pasteurella multocida*. Die dargestellte Aminosäuresequenz kann von SEQ ID NO 4 abgeleitet werden.
SEQ ID NO 6: Nucleinsäuresequenz, codierend ein Protein mit der Aktivität einer Heparosansynthase aus *Pasteurella multocida*.
SEQ ID NO 7: Aminosäuresequenz eines Proteins mit der Aktivität einer Heparosansynthase aus *Pasteurella multocida*. Die dargestellte Aminosäuresequenz kann von SEQ ID NO 6 abgeleitet werden.
SEQ ID NO 8: Nucleinsäuresequenz, codierend ein Protein mit der Aktivität einer GlcN-6-P Acetyltransferase aus *Saccharomyces cerevisiae*.
SEQ ID NO 9: Aminosäuresequenz eines Proteins mit der Aktivität einer GlcN-6-P Acetyltransferase aus *Saccharomyces cerevisiae*. Die dargestellte Aminosäuresequenz kann von SEQ ID NO 8 abgeleitet werden.
SEQ ID NO 10: Nucleinsäuresequenz, codierend ein Protein mit der Aktivität einer UDP-GIcNAc Pyrophosphorylase aus *Saccharomyces cerevisiae*.
SEQ ID NO 11: Aminosäuresequenz eines Proteins mit der Aktivität einer UDP-GlcNAc Pyrophosphorylase aus *Saccharomyces cerevisiae*. Die dargestellte Aminosäuresequenz kann von SEQ ID NO 10 abgeleitet werden.
SEQ ID NO 12: Nucleinsäuresequenz einer Expressionskassette umfassend den YLCV-Promoter, Restriktionsschnittstellen, die Polyadenylierungssignalsequenz des ocs-Terminators aus *Agrobacterium* und die Polyadenylierungssignalsequenz des nos-Terminators aus *Agrobacterium.*
SEQ ID NO 13: Synthetisches Oligonucleotid zur Herstellung einer MCS ("Multiple Cloning Site").
SEQ ID NO 14: Synthetisches Oligonucleotid zur Herstellung einer MCS ("Multiple Cloning Site").
SEQ ID NO 15: Primer, der für PCR Reaktionen eingesetzt wurde.
SEQ ID NO 16: Primer, der für PCR Reaktionen eingesetzt wurde.
SEQ ID NO 17: Primer, der für PCR Reaktionen eingesetzt wurde.
SEQ ID NO 18: Primer, der für PCR Reaktionen eingesetzt wurde.
SEQ ID NO 19: Primer, der für PCR Reaktionen eingesetzt wurde.
SEQ ID NO 20: Primer, der für PCR Reaktionen eingesetzt wurde.
SEQ ID NO 21: Nucleinsäuresequenz, codierend ein Protein mit der Aktivität einer GlcN-1-P Mutase aus *Escherichia coli.*
SEQ ID NO 22: Aminosäuresequenz eines Proteins mit der Aktivität einer GlcN-1-P Mutase aus *Escherichia coli.* Die dargestellte Aminosäuresequenz kann von SEQ ID NO 21 abgeleitet werden.
SEQ ID NO 23: Synthetisches Oligonucleotid, das als PCR Primer verwendet wurde.
SEQ ID NO 24: Synthetisches Oligonucleotid, das als PCR Primer verwendet wurde.
SEQ ID NO 25: Nucleinsäuresequenz, codierend ein Protein mit den bifunktionalen Aktivitäten einer Glucosamin-1-Phosphat Acetyltransferase und einer UDP-GlcNAc Pyrophosphorylase aus *E*. *coli* (*g*/*mu*).
SEQ ID NO 26: Aminosäuresequenz eines Proteins mit den bifunktionalen Aktivitäten einer Glucosamin-1-Phosphat Acetyltransferase und einer UDP-GlcNAc Pyrophosphorylase aus *E*. *coli*. Die dargestellte Aminosäuresequenz kann von SEQ ID NO 25 abgeleitet werden.
SEQ ID NO 27: Primer, der für PCR Reaktionen eingesetzt wurde
SEQ ID NO 28: Primer, der für PCR Reaktionen eingesetzt wurde
SEQ ID NO 29: Nucleinsäuresequenz, codierend ein Protein mit der Aktivität einer Phosphoacetylglucosamine (GlcN-P) Mutase aus *Saccharomyces cerevisiae*.
SEQ ID NO: 30 Aminosäuresequenz eines Proteins mit der Aktivität einer Phosphoacetylglucosamine Mutase aus *Saccharomyces cerevisiae*. Die dargestellte Aminosäuresequenz kann von SEQ ID NO 30 abgeleitet werden.

### Allgemeine Methoden

Im Folgenden werden Methoden beschrieben, die im Zusammenhang mit der vorliegenden Erfindung verwendet werden können. Diese Methoden stellen konkrete Ausführungsformen dar, beschränken die vorliegende Erfindung jedoch nicht auf diese Methoden. Dem Fachmann ist bekannt, dass er durch Modifikation der beschriebenen Methoden und/oder durch Ersetzen einzelner Methoden bzw. Methodenteile durch alternative Methoden bzw. alternative Methodenteile die Erfindung in gleicher Weise ausführen kann.

### 1. Transformation von Kartoffelpflanzen

Kartoffelpflanzen wurden mit Hilfe von *Agrobacterium*, wie bei Rocha-Sosa et al. (EMBO J. 8, (1989), 23-29) beschrieben, transferiert.

### 2. Isolierung von Glucosaminglycan am Beispiel von Hyaluronan aus pflanzlichem Gewebe

Pflanzenmaterial wurde zum Nachweis des Vorliegens von Hyaluronan und zur Bestimmung des Hyaluronangehaltes in pflanzlichem Gewebe folgendermaßen aufgearbeitet: Zu ca. 0,3 g Pflanzenmaterial wurden 200 µl Wasser (demineralisiert, Leitfähigkeit ≥18 MΩ) zugegeben und mit einer Labor-Schwingkugelmühle (MM200, Firma Retsch, Germany) zerkleinert (30 sec. bei 30 HZ). Anschließend erfolgte eine weitere Zugabe von 800 µl Wasser (demineralisiert, Leitfähigkeit ≥18 MΩ) und eine gute Durchmischung (z.B. mit Hilfe eines Vortex). Die Zelltrümmer und unlösliche Bestandteile wurden vom Überstand durch 5 minütiges Zentrifugieren bei 16000xg abgetrennt. Um die Menge an Hyaluronan in gesamten oberirdischen Teilen von Pflanzen zu bestimmen wurden die oberirdischen Teile der Pflanzen ca. 1 cm bis 3 cm über dem Kultursubstart abgeschnitten, in kleine Teile zerteilt und anschließend mittels eines Warring Blendors wie unter Allgemeine Methoden Punkt 3 aufgeschlossen. Dem erhaltenen Zentrifugationsüberstand (siehe Allgemeine Methoden punkt 3) kann anschließend ein Aliquot zur Bestimmung des Hyaluronangehaltes entnommen werden.

### 3. Aufreinigung von Glucosaminglycan am Beispiel von Hyaluronan

Zerkleinertes Pflanzenmaterial oder der gesamte oberirdische Teil einer Pflanze wurden nach Zugabe von Wasser (ca. 100 ml Wasser, demineralisiert, Leitfähigkeit ≥18 MΩ, je 100 g Pflanzematerial) in einem Warring Blendor für ca. 30 Sekunden bei maximaler Geschwindigkeit zerkleinert. Anschließend wurden die Zelltrümmer über ein Teesieb abgetrennt. Die abgetrennten Zelltrümmer wurden nochmals mit 300 ml Wasser (demineralisiert, Leitfähigkeit ≥18 MΩ) aufgeschlemmt und erneut über ein Teesieb abgetrennt. Die beiden erhaltenen Suspensionen (100 ml + 300 ml) wurden vereinigt und für 15 Minuten bei 13000xg zentrifugiert. Dem erhaltenen Zentrifugationsüberstand wurde NaCl bis zu einer Endkonzentration von 1% zugegeben. Nachdem das NaCl gelöst war, erfolgte eine Fällung, durch Zugabe des doppelten Volumens Ethanol, anschließendem gutem Durchmischen und Inkubation über Nacht bei -20°C. Anschließend erfolgte eine Zentrifugation für 15 Minuten bei 13000xg. Der nach dieser Zentrifugation erhaltene, sedimentierte Niederschlag wurde in 100 ml Puffer (50mM TrisHCl, pH 8, 1 mM CaCl₂) gelöst, bevor Proteinase K bis zu einer Endkonzentration von 100 µg/ml zugegeben und die Lösung für 2 Stunden bei 42°C inkubiert wurde. Es folgte eine Inkubation für 10 Minuten bei 95°C. Es wurde zu dieser Lösung erneut NaCl bis zu einer Endkonzentration von 1% zugegeben. Nachdem das NaCl gelöst war, erfolgte eine erneute Fällung, durch Zugabe des doppelten Volumens Ethanol, gutem Durchmischen und Inkubation für ca. 96 Stunden bei -20°C. Anschließend erfolgte eine Zentrifugation für 15 Minuten bei 13000xg. Der nach dieser Zentrifugation erhaltene, sedimentierte Niederschlag wurde in 30 ml Wasser (demineralisiert, Leitfähigkeit ≥18 MΩ) gelöst und erneut mit NaCl bis zu einer Endkonzentration von 1% versetzt. Durch Zugabe des doppelten Volumens Ethanol, gutem Durchmischen und Inkubation über Nacht bei -20°C erfolgte eine erneute Fällung. Der nach anschließender 15 minütiger Zentrifugation bei 13000xg erhaltene Niederschlag wurde in 20 ml Wasser (demineralisiert, Leitfähigkeit ≥18 MΩ) gelöst.
Eine weitere Aufreinigung erfolgte mittels Zentrifugalfiltration. Dazu wurden jeweils 5 ml des gelösten Niederschlages über einen Membranfilter (CentriconAmicon, Porenweite 10000 NMWL, Prod. Nr. UCF8 010 96) gegeben und bei 2200xg solange zentrifugiert, bis noch ca. 3 ml der Lösung oberhalb des Filters übrig waren. Anschließend wurden noch zweimal jeweils 3 ml Wasser (demineralisiert, Leitfähigkeit ≥18 MΩ) zu der über der Membran befindlichen Lösung hinzu gegeben und jeweils erneut unter gleichen Bedingungen zentrifugiert, bis am Ende noch ca. 3 ml der Lösung oberhalb des Filters übrig waren. Die nach Zentrifugalfiltration noch über der Membran vorliegenden Lösungen werden abgenommen und die Membran noch mehrmals (drei- bis fünfmal) mit ca. 1,5 ml Wasser (demineralisiert, Leitfähigkeit ≥18 MΩ) gespült. Alle noch über der Membran vorliegenden Lösungen und die Spüllösungen werden vereinigt, mit NaCl bis zu einer Endkonzentration von 1% versetzt, nach Lösen des NaCl mit dem doppelten Volumen an Ethanol versehen, gemischt und durch Lagerung bei -20°C über Nacht gefällt. Der nach anschließender 15 minütiger Zentrifugation bei 13000xg erhaltene Niederschlag wurde in 4 ml Wasser (demineralisiert, Leitfähigkeit ≥18 MΩ) gelöst und anschließend gefriergetrocknet (24 Stunden bei einem Druck von 0,37 mbar, Gefriertrocknungsanlage Christ Alpha 1-4, Firma Christ, Osterode).

### 4. Nachweis von Hyaluronan und Bestimmung des Hyaluronangehaltes

Der Nachweis von Hyaluronan erfolgte mit Hilfe eines käuflich erwerbbaren Tests (Hyaluronic Acid (HA) Test Kit der Firma Corgenix, Inc., Colorado, USA, Prod. Nr. 029-001) nach den Angaben des Herstellers, die durch Bezugnahme hiermit als Gegenstand in die Beschreibung aufgenommen sind. Das Testprinzip beruht auf der Verfügbarkeit eines spezifisch an Hyaluronan bindenden Proteins (HABP) und erfolgt ähnlich einem ELISA, wobei eine Farbreaktion den Hyaluronangehalt in der untersuchten Probe anzeigt. Die Hyaluronanwerte werden dabei mit Hilfe einer Eichgeraden, die unter Verwendung von definierten Mengen an Hyaluronan, die dem Testkit beigefügt sind, ermittelt. Die zu vermessenden Proben sollten daher für die quantitative Bestimmung von Hyaluronan in solch einer Konzentration eingesetzt werden (z.B: verdünnen der jeweiligen Probe oder Verwendung von weniger Wasser für die Extraktion von Hyaluronan aus dem pflanzlichen Gewebe, je nachdem ob ein Grenzwert über- oder unterschritten wurde), dass sie innerhalb der angegebenen Grenzen liegen.
In parallel-Ansätzen wurden Aliquots der zu bestimmenden Proben zunächst einem Hyaluronidaseverdau unterzogen, bevor sie mit dem käuflich erwerbbaren Test (Hyaluronic Acid (HA) Test Kit der Firma Corgenix, Inc., Colorado, USA, Prod. Nr. 029-001) vermessen wurden. Der Hyaluronidaseverdau erfolgte mit 400 µl Pflanzenextrakt in Hyaluronidase Puffer (0,1 M Kalium Phosphatpuffer, pH 5,3; 150 mm NaCl) durch Zugabe von 5 µg (∼3 units) Hyaluronidase (Hyaluronidase Typ III von Sigma, Prod. Nr. H 2251) bei einer Inkubation von 30 min bei 37 °C.
Anschließend wurden alle Proben jeweils in einer Verdünnung von 1:10 für die Bestimmung des Hyaluronangehaltes eingesetzt.

### 5. Bestimmung der Aktivität einer GFAT

Die Bestimmung der Aktivität eines Proteins mit der Aktivität einer GFAT wird wie bei Rachel et al. (1996, J. Bacteriol. 178 (8), 2320-2327) beschrieben durchgeführt.
Zur Unterscheidung ob ein Protein die Aktivität einer GFAT-1 oder GFAT-2 aufweist, wird die bei Hu et al. (2004, J. Biol. Chem. 279 (29), 29988-29993) beschriebene Methode verwendet.

### Beispiele

### 1. Herstellung des pflanzlichen Expressionsvektors IR 47-71

Das Plasmid pBinAR ist ein Derivat des binären Vektorplasmids pBin19 (Bevan, 1984, Nucl Acids Res 12: 8711-8721), das folgendermaßen konstruiert wurde:
Ein 529 bp langes Fragment, das die Nukleotide 6909-7437 des 35S-Promotor des Blumenkohl-Mosaik-Virus umfasst, wurde als *EcoR* I/*Kpn* I Fragment aus dem Plasmid pDH51 (Pietrzak et al, 1986 Nucleic Acids Res. 14, 5858) isoliert und zwischen die *Eco*R I und *Kpn* I Restriktionsschnittstellen des Polylinkers von pUC18 ligiert. Dabei entstand das Plasmid pUC18-35S. Aus dem Plasmid pAGV40 (Herrera-Estrella et al, 1983 Nature, 303, 209-213) wurde mit Hilfe der Restriktionsendonucleasen *Hind* III und *Pvu* II ein 192 bp langes Fragment isoliert, das das Polyadenylierungssignal (3'-Ende) des *Octopin Synthase*-Gens (Gen 3) der T-DNA des Ti-Plasmids pTiACH5 (Gielen et al, 1984, EMBO Journal 3, .835-846) umfasst (Nucleotide 11749-11939). Nach Addition von *Sph* I Linkern an die *Pvu* II-Schnittstelle wurde das Fragment zwischen die *Sph* I- und *Hind* III-Schnittstellen von pUC18-35S ligiert. Daraus resultierte das Plasmid pA7. Hier wurde der gesamte Polylinker enthaltend den 35S-Promotor und Ocs-Terminator mit EcoR I und *Hind* III herausgeschnitten und in den entsprechend geschnittenen Vektor pBin19 ligiert. Dabei entstand der pflanzliche Expressionsvektor pBinAR (Höfgen und Willmitzer, 1990, Plant Science 66, 221-230).

Der Promotor des Patatin Gens B33 aus *Solanum tuberosum* (Rocha-Sosa et al., 1989, EMBO J. 8, 23-29) wurde als *Dra* I Fragment (Nukleotide -1512 - +14) in den mit *Sst* I geschnittenen Vektor pUC19, dessen Enden mit Hilfe der T4-DNA Polymerase geglättet worden waren, ligiert. Daraus entstand das Plasmid pUC19-B33. Aus diesem Plasmid wurde der B33-Promotor mit *EcoR* I und *Sma* I herausgeschnitten und in den entsprechend geschnittenen Vektor pBinAR ligiert. Hieraus entstand der pflanzliche Expressionsvektor pBinB33.
Um weitere Klonierungsschritte zu erleichtern, wurde die MCS (Multiple Cloning Site) erweitert. Hierfür wurden zwei komplementäre Oligonucleotide synthetisiert, für 5 Minuten auf 95°C erhitzt, auf Raumtemperatur langsam abgekühlt, um ein gutes fixieren (annealen), zu ermöglichen und in die *Sal* I und *Kpn* I Schnittstellen von pBinB33 kloniert. Die dazu verwendeten Oligonucleotide sind unter SEQ ID NO 13 und SEQ ID NO 14 dargestellt. Das erhaltene Plasmid wurde mit IR 47-71 bezeichnet.

### 2. Herstellung des pflanzlichen Expressionsvektors pBinARHyg

Das Fragment, enthaltend den 35S-Promotor, den ocs-Terminator und die gesamte Multiple Cloning Site wurde mittels der Restriktionsendonucleasen *Eco*R I und *Hind* III aus pA7 herausgeschnitten und in den mit den gleichen Restiktionsendonucleasen geschnittenen Vektor pBIBHyg Becker, 1990, Nucleic Acids Res. 18, 203) kloniert. Das erhaltene Plasmid wurde mit pBinARHyg bezeichnet.

### 3. Herstellung des Klonierungsvektors IC 317-204

Nucleinsäurefragmente, umfassend den ocs-Terminator wurden mittels der Restriktionsendonucleasen *Xho* I und *Hind* III aus dem Plasmid IR 47-71 isoliert und in den mit den gleichen Restriktionsendonucleasen geschnittenen Vektor pBlueScript KS (Firma Stratagene, Prod. Nr. 212207) kloniert. Das erhaltene Plasmid wurde mit IC 306-204 bezeichnet.
Nucleinsäurefragmente, umfassend den B33 Promoter wurden mittels der Restriktionsendonucleasen *Bam* HI und *Eco* RI aus dem Plasmid IR 47-71 isoliert und in den mit den gleichen Restriktionsendonucleasen geschnittenen Vektor pBlueScript KS (Firma Stratagene, Prod. Nr. 212207) kloniert. Das erhaltene Plasmid wurde mit IC 314-204 bezeichnet.

Aus IC 306-204 wurde der OCS-Terminator mittels der Restriktionsendonuclease *Bam* HI isoliert und in das mit der gleichen Restriktionsendonuclease geschnittene Plasmid IC 314-204 kloniert. Das erhaltene Plasmid wurde mit IC 317-204 bezeichnet.

### 4. Synthese von Nucleinsäuremolekülen

### a) Synthese von Nucleinsäuremolekülen codierend eine Hyaluronansynthase des Paramecium bursaria Chlorella Virus 1

Die Nucleinsäuresequenz, codierend eine Hyaluronansynthase aus *Paramecium bursaria Chlorella* Virus 1, wurde von der Firma Medigenomix GmbH (München, Deutschland) synthetisiert und in den Vektor pCR 2.1 der Firma Invitrogen (Prod. Nr. K2000-01) kloniert. Das erhaltene Plasmid wurde mit IC 323-215 bezeichnet. Die synthetische Nucleinsäuresequenz codierend das HAS Protein aus *Paramecium bursaria Chlorella* Virus 1, ist unter SEQ ID NO 3 dargestellt. Die korrespondierende, ursprünglich aus dem *Paramecium bursaria Chlorella* Virus 1 isolierte Nucleinsäuresequenz, ist unter SEQ ID NO 1 dargestellt.

### b) Synthese der Nucleinsäuresequenzen umfassend einen YLCV promotor sowie eine MCS, einen nos-Terminator und einen ocs-Terminator

Die Nucleinsäuresequenz, umfassend einen YLCV Promotor (Stavolone et al., Plant Molecular Biology 53: 703-713, 2003); sowie eine MCS ("Multiple Cloning Site") enthaltend die Schnittstellen *Sac* I und *Sma* I,, einen *nos*-Terminator und einen *ocs-*Terminator wurde von der Firma Entelechon GmbH synthetisiert und in den Vektor pCR4Topo der Firma Invitrogen (Prod. Nr. K4510-20) kloniert. Das erhaltene Plasmid wurde mit IC 389-337 bezeichnet. Die synthetische Nucleinsäuresequenz ist unter SEQ ID NO 12 dargestellt.

### 5. Isolierung von Nucleinsäuremolekülen

### a) Isolierung und Klonierung einer codierenden Nucleinsäuresequenz für ein Protein mit der Aktivität einer GlcN-6-P Acetyltransferase

Die Nucleinsäuresequenz, codierend ein Protein mit der Aktivität einer GlcN-6-P Acetyltransferase aus *Saccharomyces cerevisiae* (*gna1*) wurde mittels PCR isoliert und in den Vektor pCR 2.1 der Firma Invitrogen (Prod. Nr. K4510-20) kloniert. Die Reaktionsbedingungen für die PCR waren:
1. Schritt: 5 min 94 °C,
2. Schritt: 45 sec, 94 °C
3. Schritt: 45 sec, 59 °C
4. Schritt: 45 sec, 72 °C
5. Schritt: 10 min, 72 °C
6. Schritt: 4°C

Die Schritte 2 bis 3 wurden 35 mal wiederholt, bevor mit Schritt 5 fortgefahren wurde. Im 50 µl-Reaktionsansatz befanden sich Puffer (10 mM Tris-HCl, pH 9.0, 50 mM KCI und 3 mM MgSO₄, je 500 nM Amplifikationsprimer, dargestellt unter SEQ ID NO 15 und SEQ ID NO 16" 10 µl Q-Solution (enthalten in Qiagen, Prod. Nr. 206143), je 0,2 mM desoxy-Ribonucleotide, 0,5 µl Taq DNA-Polymerase (Invitrogen, Prod. Nr.: 11304-011) und 250 ng genomische Hefe DNA als Matrize (Template). Die PCR wurde mit dem Mastercycler der Firma Eppendorf (Prod. NR. 5331 000.010) durchgeführt.
Die isolierte Nucleinsäuresequenz codierend das Protein mit der Aktivität einer GlcN-6-P Acetyltransferase aus *Saccharomyces cerevisiae*, ist unter SEQ ID NO 8 dargestellt.
Nach Klonierung des erhaltenen Fragmentes in den Vektor pCR 2.1 und Bestätigung der Sequenz wurde die betreffende Nucleinsäuresequenz, codierend ein Protein mit der Aktivität einer GlcN-6-P Acetyltransferase aus *Saccharomyces cerevisiae* mittels der Restriktionsendonucleasen *Kpn* I und *Xba* I, isoliert und in den mit den gleichen Restriktionsendonucleasen geschnittenen Vektor pA7 kloniert. Das erhaltene Plasmid wurde mit IC 298-204 bezeichnet.

### b) Isolierung und Klonierung einer Nucleinsäuresequenz codierend ein Protein mit der Aktivität einer UDP-GlcNAc Pyrophosphorylase

Die Nucleinsäuresequenz, codierend ein Protein mit der Aktivität einer UDP-GIcNAc Pyrophosphorylase aus *Saccharomyces cerevisiae* (*qri*) wurde mittels PCR isoliert und in den Vektor pCR 2.1 der Firma Invitrogen (Prod. Nr. K2000-01) kloniert. Die Reaktionsbedingungen für die PCR waren:
1. Schritt: 5 min 94 °C,
2. Schritt: 45 sec, 94 °C
3. Schritt: 45 sec, 59 °C
4. Schritt: 45 sec, 72 °C
5. Schritt: 30 min, 72 °C
6. Schritt: 4°C

Die Schritte 2 bis 3 wurden 35 mal wiederholt, bevor mit Schritt 5 fortgefahren wurde. Im 50 µl-Reaktionsansatz befanden sich Puffer (10 mM Tris-HCl, pH 9.0, 50 mM KCI und 3 mM MgSO₄, je 500 nM Amplifikationsprimer, dargestellt unter SEQ ID NO 17 und SEQ ID NO 18, 10 µl Q-Solution (enthalten in Qiagen, Prod. Nr.: 206143), je 0,2 mM desoxy-Ribonucleotide, 0,5 µl Taq DNA-Polymerase (Invitrogen, Prod. Nr.: 11304-011) und 250 ng genomische Hefe. DNA (Invitrogen Prod. Nr. 40802) als Matrize (Template). Die PCR wurde mit dem Mastercycler der Firma Eppendorf (Prod. Nr. 5331 000.010) durchgeführt.
Die isolierte Nucleinsäuresequenz codierend das Protein mit der Aktivität einer UDP-GlcNAc Pyrophosphorylase aus *Saccharomyces cerevisiae*, ist unter SEQ ID NO 10 dargestellt.
Nach Klonierung des erhaltenen Fragmentes in den Vektor pCR 2.1 und Bestätigung der Sequenz wurde die Nucleinsäuresequenz codierend ein Protein mit der Aktivität einer UDP-GlcNAc Pyrophosphorylase aus *Saccharomyces cerevisiae* mittels der Restriktionsendonucleasen *Kpn* I und *Xba* I, isoliert und in den mit den gleichen Restriktionsendonucleasen geschnittenen Vektor pA7 Vektor kloniert. Das erhaltene Plasmid wurde mit IC 303-204 bezeichnet.

### c) Isolierung und Klonierung einer Nucleinsäuresequenz codierend ein Protein mit der Aktivität einer GlcNAc-P Mutase

Die Nucleinsäuresequenz, codierend ein Protein mit der Aktivität einer Phosphoacetylglucosamin Mutase aus *Saccharomyces cerevisiae* (*pcm I,* EC 5.4.2.3) wurde mittels PCR isoliert und in den Vektor pCR 2.1 der Firma Invitrogen (Prod. Nr. K2000-01) kloniert. Die Reaktionsbedingungen für die PCR waren:
1. Schritt: 5 min 94 °C,
2. Schritt: 45 sec, 94 °C
3. Schritt: 45 sec, 59 °C
4. Schritt: 45 sec, 72 °C
5. Schritt: 30 min, 72 °C
6. Schritt: 4°C

Die Schritte 2 bis 3 wurden 35 mal wiederholt, bevor mit Schritt 5 fortgefahren wurde. Im 50 µl-Reaktionsansatz befanden sich Puffer (10 mM Tris-HCl, pH 9.0, 50 mM KCI und 3 mM MgSO4, je 500 nM Amplifikationsprimer, dargestellt unter SEQ ID NO 27 und SEQ ID NO 28, 10 µl Q-Solution (enthalten in Qiagen, Prod. Nr.: 206143), je 0,2 mM desoxy-Ribonucleotide, 0,5 µl Taq DNA-Polymerase (Invitrogen, Prod. Nr.: 11304-011) und 250 ng genomische Hefe DNA (Invitrogen Prod. Nr. 40802) als Matrize (Template). Die PCR wurde mit dem Mastercycler der Firma Eppendorf (Prod. Nr. 5331 000.010) durchgeführt.
Nach Klonierung des erhaltenen Fragmentes in den Vektor pCR 2.1 und Bestätigung der Sequenz wurde die Nucleinsäuresequenz codierend ein Protein mit der Aktivität einer Phosphoacetylglucosamin Mutase aus *Saccharomyces cerevisiae* mittels der Restriktionsendonucleasen *Kpn* I und *Xba* I isoliert und in den mit den gleichen Restriktionsendonucleasen geschnittenen Vektor pA7 kloniert. Das erhaltene Plasmid wurde mit IC 304-204 bezeichnet.

### d) Isolierung und Klonierung einer Nucleinsäuresequenz codierend ein Protein mit der Aktivität einer GlcN-1-P Mutase aus Escherichia coli

Die Nucleinsäuresequenz, codierend ein Protein mit der Aktivität einer Glucosamin 1-Phosphat Mutase (GlcN-1-P Mutase) aus *E*. *coli* (*glmm*) wurde mittels PCR isoliert und in den Vektor pCR 2.1 der Firma Invitrogen (Prod. Nr. K2000-01)) kloniert. Die Reaktionsbedingungen für die PCR waren:
1. Schritt: 5 min 94 °C,
2. Schritt: 45 sec, 94 °C
3. Schritt: 45 sec, 59 °C
4. Schritt: 45 sec, 72 °C
5. Schritt: 30 min, 72 °C
6. Schritt: 4°C

Die Schritte 2 bis 3 wurden 35 mal wiederholt, bevor mit Schritt 5 fortgefahren wurde. Im 50 µl-Reaktionsansatz befanden sich Puffer (10 mM Tris-HCl, pH 9.0, 50 mM KCl und 3 mM MgSO₄, je 500 nM Amplifikationsprimer, dargestellt unter SEQ ID NO 19 und SEQ ID NO 20, 10 µl Q-Solution (enthalten in Qiagen, Prod. Nr.: 206143), je 0,2 mM desoxy-Ribonucleotide, 0,5 µl Taq DNA-Polymerase (Invitrogen, Prod. Nr.: 11304-011) und 250 ng genomische *E*. *col*i DNA als Matrize (Template). Die PCR wurde mit dem Mastercycler der Firma Eppendorf (Prod. Nr. 5331 000.010) durchgeführt.
Die isolierte Nucleinsäuresequenz codierend das Protein mit der Aktivität einer Glucosamin 1-Phosphat Mutase Protein aus *E*. *coli* (*glmm*), ist unter SEQ ID NO 21 dargestellt.
Nach Klonierung des erhaltenen Fragmentes in den Vektor pCR 2.1 und Bestätigung der Sequenz wurde die Nucleinsäuresequenz, codierend ein Protein mit der Aktivität einer GlcN-1-P Mutase aus *E*. *coli* mittels der Restriktionsendonucleasen *Kpn* I und *Xba* I isoliert und in den mit den gleichen Restriktionsendonucleasen geschnittenen Vektor pA7 Vektor kloniert. Das erhaltene Plasmid wurde mit IC 300-204 bezeichnet.

### e) Isolierung und Klonierung einer Nucleinsäuresequenz codierend ein Protein mit der bifunktionalen Aktivität einer GlcN-1-P Acetyltransferase und einer UDP-GlcNAc-1-P Pyrophosphorylase aus Escherichia coli

Die Nucleinsäuresequenz, codierend ein bifunktionales Protein mit der Aktivität einer Glucosamin-1-Phosphat Acetyltransferase und einer UDP-GlcNAc Pyrophosphorylase aus *E*. *coli* (*qlmu*) wurde mittels PCR isoliert und in den Vektor pCR2.1 der Firma Invitrogen (Prod. Nr. K2000-01)) kloniert. Die Reaktionsbedingungen für die PCR waren:
1. Schritt: 5 min 94 °C,
2. Schritt: 45 sec, 94 °C
3. Schritt: 45 sec, 59 °C
4. Schritt: 45 sec, 72 °C
5. Schritt: 30 min, 72 °C
6. Schritt: 4°C

Die Schritte 2 bis 3 wurden 35 mal wiederholt, bevor mit Schritt 5 fortgefahren wurde. Im 50 µl-Reaktionsansatz befanden sich Puffer (10 mM Tris-HCl, pH 9.0, 50 mM KCl und 3 mM MgSO₄, je 500 nM Amplifikationsprimer, dargestellt unter SEQ ID NO 23 und SEQ ID NO 24, 10 µl Q-Solution (enthalten in Qiagen, Prod. Nr.: 206143), je 0,2 mM desoxy-Ribonucleotide, 0,5 µl Taq DNA-Polymerase (Invitrogen, Prod. Nr.: 11304-011) und 250 ng genomische *E*. *coli DNA* als Matrize (Template). Die PCR wurde mit dem Mastercycler der Firma Eppendorf (Prod. Nr. 5331 000.010) durchgeführt.
Die isolierte Nucleinsäuresequenz codierend ein Protein mit den bifunktionalen Aktivitäten einer Glucosamin-1-Phosphat Acetyltransferase und einer UDP-GlcNAc Pyrophosphorylase aus *E*. *coli* (*glmu*), ist unter SEQ ID NO 25 dargestellt.
Nach Klonierung des erhaltenen Fragmentes in den Vektor pCR 21 und Bestätigung der Sequenz wurde die Nucleinsäuresequenz, codierend ein bifunktionales Protein mit der Aktivität einer GlcN-1-P Acetyltransferase und einer UDP-GlcNAc Pyrophosphorylase aus *E*. *coli* (*glmu*) mittels der Restriktionsendonucleasen *Kpn* I und *Xba* I isoliert und in den mit den gleichen Restriktionsendonucleasen geschnittenen Vektor pA7 Vektor kloniert. Das erhaltene Plasmid wurde mit IC 299-204 bezeichnet.

### 6. Herstellung des pflanzlichen Expressionsvektors IC 341-222, umfassend eine codierende Nucleinsäuresequenz für eine Hyaluronansynthase des Paramecium bursaria Chlorella Virus 1

Aus dem Plasmid IC 323-215 wurden mittels Restriktionsverdau mit *BamH* I und *Xho* I Nucleinsäuremoleküle, umfassend die codierende Sequenz der Hyaluronansynthase, isoliert und in die *BamH* I und *Xho* I Schnittstellen des Plasmides IR 47-71 kloniert. Der erhaltene pflanzliche Expressionsvektor wurde mit IC 341-222 bezeichnet.

### 7. Herstellung des pflanzlichen Expressionsvektors IC 351-222 enthaltend eine codierende Nucleinsäuresequenz für ein Protein mit der Aktivität einer GlcN-6-P Acetyltransferase aus Saccharomyces cerevisiae

Ausgangsplasmid ist der bereits beschriebene pflanzlichen Expressionsvektors pUBI bar (WO 97 44472), in dessen *Eco*R I und *Sda* I Restriktionsschnittstellen die die codierende Sequenz des *gna*-Gens aus Hefe kloniert wurde. Die kodierende Sequenz des *gna*-Gens aus Hefe wurde mittels *Eco*R I und *Sda* I Restriktionsverdau aus dem Plasmid IC 298-204 isoliert. Der erhaltene Vektor wurde mit IC 351-222 bezeichnet.

### 8. Herstellung des pflanzlichen Expressionsvektors IC 392-337 enthaltend codierende Nucleinsäuresequenzen für ein Protein mit der Aktivität einer GlcN-6-P Acetyltransferase und ein Protein mit der Aktivität einer UDP-GlcNAc Pyrophosphorylase

Ausgangsplasmid ist das weiter oben beschriebene Plasmid IC 351-222, in dessen *Eco* RI Restriktionsschnittstelle die Kassette aus YLCV Promotor sowie NOS Terminator und OCS Terminator, die mittels der Restriktionsendonuclease *Eco* RI aus dem Plasmid IC 389-337 isoliert wurde, kloniert wurde. Diese Kassette wurde mittels Restriktionsverdau [hier fehlt doch was - oder?]. Der erhaltene Vektor wurde mit IC 390-337 bezeichnet.
Aus dem bereits beschriebenen Plasmid IC 303-204 wurden die codierende Sequenz des *qri*-Gens mittels *Sac* I und *Eco* RV Restriktionsverdau isoliert und in die *Sac* I und *Sma* I Schnittstellen des Vektors IC 390-337 ligiert. Der erhaltene Vektor wurde mit IC 391-337 bezeichnet.
Um den redundanten OCS Terminator zu entfernen wurde der Vektor IC 391-337 mit *Aat* II verdaut und anschließend religiert. Der erhaltene pflanzliche Expressionsvektor wurde mit IC 392-337 bezeichnet.

### 9. Herstellung des pflanzlichen Expressionsvektors IC 360-237 enthaltend eine codierende Nucleinsäuresequenz für ein Protein mit der Aktivität einer GlcN-1-P Mutase und ein bifunktionales Protein mit der bifunktionalen Aktivität einer GlcN-1-P Acetyltransferase und einer UDP-GlcNAc Pyrophosphorylase aus Escherichia coli

Das Ausgangsplasmid für die Einführung der Nucleinsäuresequenz codierend für ein Protein mit der bifunktionalen Aktivität einer GlcN-1-P Acetyltransferase und einer UDP-GlcNAc-1-P Pyrophosphorylase aus *Escherichia coli* war das weiter oben beschriebene Plasmid IC 299-204, aus dem mittels *Eco* RI Restriktionsverdau die codierende Sequenz isoliert und in die *Eco* RI Schnittstelle des pMCS5 Vektors (MoBiTec GmbH, Prod.Nr.: pMCSS) kloniert wurde. Der erhaltene Vektor wurde mit IC 307-204 bezeichnet. Im nächsten Schritt wurde der Vektor IC 307-204 mit *Pme* I und *Sma* I Restriktionsendonucleasen verdaut und religiert. Der erhaltene Vektor wurde mit IC 311-204 bezeichnet. Anschließend wurde aus dem Plasmid IC 311-204 mittels Restriktionsverdau mit *Bam* HI und *Kpn* I die Nucleinsäuresequenz codierend für ein Protein mit der Aktivität einer GLMU isoliert und in die Restriktionsschnittstellen *Bam* HI und *Kpn* I des Vektors IC 312-204 ligiert. Der erhaltene Vektor wurde mit IC 315-204 bezeichnet. Der Vektor IC 312-204 wurde mittels gleichzeitiger Ligation von drei Fragmenten, zusammengesetzt aus einem 35S-Promotorfragment, isoliert mittels *Eco* RI und *Sal* I Restriktionsverdau aus dem Plasmid pA7. einem *ocs*-Fragment, isoliert mittels *Hind* III und *Sal* I Restriktionsverdau aus IC 309-204 und dem mit *Eco* RI Restriktionsverdau geöffnetem Vektor IC310-204, hergestellt. Das Plasmid IC310-204 bezeichnet einen pUC 18 Vektor dem durch *Hind* III und *Ecl* 13511 Restriktionsverdau und anschießender Religation ein Teil der MCS entfernt wurde. IC 309-204 wurde hergestellt indem das ocs-Fragment aus pA7 mittels *Hind* III und *Sal* I isoliert und in den pBS KS Vektor, verdaut mit *Hind* III und *Sal* I, kloniert wurde.
Aus dem Plasmid IC 315-204 wurde mittels *Eco* RI Restriktionsverdau der 35S Promotor, die Nucleinsäuresequenz codierend ein Protein mit der bifunktionalen Aktivität einer Glucosamin-1-Phosphat Acetyltransferase und einer UDP-GlcNAc Pyrophosphorylase aus *E*. *coli* (*qlmu*) sowie ocs-Terminator isoliert und in die *Eco* RI Schnittstelle des Ubi Bar Vektors (WO 97 44472) kloniert. Der erhaltene Vektor wurde mit IC 359-237 bezeichnet.
Das Ausgangsplasmid für die Einführung der Nucleinsäuresequenz codierend für ein Protein mit der Aktivität einer GlcN-1-P Mutase ist das weiter oben beschriebene Plasmid IC 299-204, aus dem mittels *Sda* I und *Sma* I Restriktionsverdau die codierende Sequenz isoliert und in die *Sda* I und *Hpa* I Schnittstellen des Ubi bar Vektors liegiert wurde. Der erhaltene Vektor wurde mit IC 355-222 bezeichnet.
Aus dem Plasmid IC 355-222 wurde mittels Spe I und *Dra* I Restriktionsverdau die codierende Sequenz des *glmm*-Gens isoliert und in die *Spe* I und *Pme* I Schnittstellen des IC 359-237 Plasmids kloniert. Der erhaltene Vektor wurde mit IC 360-237 bezeichnet.

### 10. Herstellung des pflanzlichen Expressionsvektors IC 393-337 enthaltend codierende Nucleinsäuresequenzen für ein Protein mit der Aktivität einer GlcN-6-P Acetyltransferase, ein Protein mit der Aktivität einer UDP-GlcNAc Pyrophosphorylase und ein Protein mit der Aktivität einer GlcNAc-P Mutase aus Saccharomyces cerevisiae

Das Ausgangsplasmid für die Einführung der Nucleinsäuresequenz codierend für GlcNAc-P Mutase aus *Saccharomyces cerevisiae* ist das weiter oben beschriebene Plasmid IC 304-204, aus dem mittels *Eco* RI Restriktionsverdau die codierende Sequenz isoliert und in die *Eco* RI Schnittstelle des pMCS5 Vektors (MoBiTec GmbH, Prodnr.: pMCS5) kloniert wurde. Der erhaltene Vektor wurde mit IC 313-204 bezeichnet. Im nächsten Schritt wurde die Nucleinsäuresequenz codierend für GlcNAc-P Mutase aus *Saccharomyces cerevisiae* aus dem Vektor IC 313-204 mittels *Pme* I und *Pac* I Restriktionsverdau isoliert und in den mit *Pme* I und *Pac* I verdauten Vektor IC 393-337 kloniert. Der erhaltene Vektor wurde mit IC 394-337 bezeichnet.
Ausgangsvektor für die Herstellung des Plasmids IC 393-337 ist das weiter oben beschrieben Plasmids IC 391-337 das bereits die Nucleinsäuresequenzen für ein Protein mit der Aktivität einer GlcN-6-P Acetyltransferase und ein Protein mit der Aktivität einer UDP-GlcNAc Pyrophosphorylase enthielt. Hierfür wurde der weiter oben beschriebene B33 Promoter mittels *Pac* I und *Avr* II Restriktionsverdau isoliert und in den mit *Pac* I und *Avr* II verdauten Vektor IC 391-337 kloniert. Der erhaltene pflanzliche Expressionsvektor wurde mit IC 393-337 bezeichnet.

### 11. Transformation von Kartoffelpflanzen

Kartoffelpflanzen wurden mit dem pflanzlichen Expressionsvektor IC 341-222, enthaltend eine codierende Nucleinsäuresequenz für eine Hyaluronansynthase aus *Paramecium bursaria Chlorella* Virus 1 unter der Kontrolle des Promotors des Patatin Gens B33 aus *Solanum tuberosum* (Rocha-Sosa et al., 1989, EMBO J. 8, 23-29) nach der unter Allgemeine Methoden Punkt 1 angegebnen Methode transformiert.
Die erhaltenen transgenen Kartoffelpflanzen, die mit dem Plasmid IC 341-222 transformiert waren, wurden mit 365 ES X bezeichnet, wobei X für aus der Transformation unabhängig hervorgegangene Pflanzen steht.
Die nach Transformation erhaltenen Linien mit der Bezeichnung 365 ES X wurden auf die Menge des von den betreffenden Pflanzen synthetisierten Hyaluronans hin analysiert (siehe auch WO 2006 032538). Für die weiteren im Folgenden beschriebenen Transformationen wurden die Linien 365 ES 13 und 365 ES 74 ausgewählt.
Kartoffelpflanzen der Linien 365 ES 13 bzw. 365 ES 74 wurden mit dem pflanzlichen Expressionsvektor IC 392-337 bzw. IC 360-237 bzw. IC 394-337 nach der unter Allgemeine Methoden Punkt 1 angegebnen Methode transformiert.
Die erhaltenen transgenen Kartoffelpflanzen der Linie 365 ES 13, die mit dem Plasmid IC 392-337 transformiert wurden, wurden mit 437 ES X bezeichnet, wobei X für aus der Transformation unabhängig hervorgegangene Pflanzen steht.
Die erhaltenen transgenen Kartoffelpflanzen der Linie 365 ES 74, die mit dem Plasmid IC 392-337 transformiert wurden, wurden mit 438 ES X bezeichnet, wobei X für aus der Transformation unabhängig hervorgegangene Pflanzen steht.
Die erhaltenen transgenen Kartoffelpflanzen der Linie 365 ES 13, die mit dem Plasmid IC 360-237 transformiert wurden, wurden mit 397 ES X bezeichnet, wobei X für aus der Transformation unabhängig hervorgegangene Pflanzen steht.
Die erhaltenen transgenen Kartoffelpflanzen der Linie 365 ES 74, die mit dem Plasmid IC 360-237 transformiert wurden, wurden mit 398 ES X bezeichnet, wobei X für aus der Transformation unabhängig hervorgegangene Pflanzen steht.
Die erhaltenen transgenen Kartoffelpflanzen der Linie 365 ES 13, die mit dem Plasmid IC 393-337 transformiert wurden, wurden mit 444 ES X bezeichnet, wobei X für aus der Transformation unabhängig hervorgegangene Pflanzen steht.
Die erhaltenen transgenen Kartoffelpflanzen der Linie 365 ES 74, die mit dem Plasmid IC 393-337 transformiert wurden, wurden mit 445 ES X bezeichnet, wobei X für aus der Transformation unabhängig hervorgegangene Pflanzen steht.

### 12. Analyse von Kartoffelpflanzen, die fremde Nucleinsäuremoleküle, codierend eine Hyaluronansynthase und codierend ein Protein mit der Aktivität einer GlcNAc-6-P Acetyltransferase und codierend ein Protein mit der Aktivität einer UDP-GlcNAc Pyrophosphorylase aufweisen

Einzelne Pflanzen der Linien 365 ES 13, 365 ES 74, 437 ES X, 438 ES X, 397 ES X, 398 ES X, 444 ES X und 445 ES X wurden im Gewächshaus in 6 cm Töpfen in Erde kultiviert. Von 7 bis 9 Wochen alten Pflanzen wurde der gesamte oberirdische Teil der Pflanze geerntet und nach der unter Allgemeine Methoden Punkt 3 beschriebenen Methode aufgearbeitet. Die Menge des darin in den jeweiligen Pflanzenextrakten wurde durch Bestimmung des in einem Aliquot der betreffenden Pflanzenextrakte enthaltenen Hyaluronans nach der unter Allgemeine Methoden Punkt 4. beschriebenen Methode und unter zur Hilfenahme einer Eichgerade ermittelt. Der nach Zentrifugation erhaltene Überstand wurde dabei in einer Verdünnung von 1:10 für die Bestimmung des Hyaluronangehaltes eingesetzt, Für ausgewählte Pflanzen wurden folgende Ergebnisse erhalten:

**Tabelle 1: Menge an Hyaluronan (in µg Hyaluronan pro g Frischgewicht), das von unabhängigen, ausgewählten transgenen Pflanzen der Linien 365 ES 13 bzw. 4365 ES 74, die nur ein fremdes Nucleinsäuremolekül, codierend eine Hyaluronansynthase aufweisen, in gesamten oberirdischen Teilen der jeweiligen Pflanzen produziert wurde. Die jeweilige Spalte 1 bezeichnet die Pflanze, von welcher Material geerntet wurde ("wt Desiree"" bezeichnet Wildtyp Pflanzen des Kultivars Désirée, die nicht transformiert sind). Die jeweilige Spalte 2 gibt die Menge Hyaluronan bezogen auf das eingesetzte Frischgewicht an.**

| Bezeichnung der Pflanze | HA [µg/g FG] | | Bezeichnung der Pflanze | HA [µg/g FG] |
|---|---|---|---|---|
| 365ES 74-67 | 120,9 | | 365ES 13-221 | 90,6 |
| 365ES 74-68 | 125,1 | | 365ES 13-222 | 57,9 |
| 365ES 74-71 | 129,2 | | 365ES 13-223 | 59,0 |
| 365ES 74-72 | 118,2 | | 365ES 13-224 | 95,3 |
| 365ES 74-79 | 129,0 | | 365ES 13-225 | 84,0 |
| 365ES 74-80 | 140,2 | | 365ES 13-226 | 91,7 |
| 365ES 74-81 | 92,7 | | 365ES 13-227 | 69,1 |
| 365ES 74-82 | 100,9 | | 365ES 13-228 | 76,2 |
| wt Desiree 1 | 0,2 | | 265ES 13-231 | 84,3 |
| wt Desiree 2 | -0,2 | | 365ES 13-232 | 75,5 |
| wt Desiree 3 | 2,1 | | | |

**Tabelle 2: Menge an Hyaluronan (in µg Hyaluronan pro g Frischgewicht), das von unabhängigen, ausgewählten transgenen Pflanzen der Linie 437 ES bzw. 438 ES in gesamten oberirdischen Teilen der jeweiligen Pflanzen produziert wurde. Die jeweilige Spalte 1 bezeichnet die Pflanze, von welcher Material geerntet wurde. Die jeweilige Spalte 2 gibt die Menge Hyaluronan bezogen auf das eingesetzte Frischgewicht an.**

| Bezeichnung der Pflanze | HA [µg/g FG] | | Bezeichnung der Pflanze | HA [µg/g FG] |
|---|---|---|---|---|
| 438ES 1 | 183,0 | | 437ES 2 | 105,6 |
| 438ES 5 | 192,5 | | 437ES 3 | 97,6 |
| 438ES 10 | 189,5 | | 437ES 6 | 129,4 |
| 438ES 11 | 168,9 | | 437ES 12 | 103,2 |
| 438ES 13 | 195,5 | | 437ES 13 | 144,2 |
| 438ES 14 | 184,3 | | 437ES 14 | 163,7 |
| 438ES 16 | 168,9 | | 437ES 15 | 128,2 |
| 438ES 23 | 231,2 | | 437ES 16 | 100,3 |
| 438ES 25 | 169,2 | | 437ES 17 | 186,3 |
| 438ES 32 | 178,6 | | 437ES 21 | 100,7 |
| 438ES 33 | 173,4 | | 437ES 23 | 114,5 |
| 438ES 41 | 178,7 | | 437ES 26 | 105,1 |
| 438ES 57 | 239,4 | | 437ES 31 | 102,0 |
| 438ES 62 | 172,5 | | 437ES 34 | 178,9 |
| 438ES 64 | 199,5 | | 437ES 35 | 104,4 |
| 438ES 80 | 235,4 | | 437ES 39 | 98,2 |
| 438ES 84 | 189,2 | | 437ES 40 | 116,8 |
| 438ES 85 | 168,1 | | 437ES 48 | 125,1 |
| 438ES 88 | 167,4 | | 437ES 66 | 146,8 |
| 438ES 97 | 164,4 | | 437ES 69 | 106,8 |
| 438ES 102 | 160,4 | | 437ES 70 | 115,4 |
| 438ES 108 | 209,8 | | 437ES 75 | 100,3 |
| 438ES 112 | 185,9 | | 437ES 76 | 125,5 |
| | | | 437ES 79 | 102,0 |
| | | | 437ES 80 | 125,7 |
| | | | 437ES 82 | 135,7 |
| | | | 437ES 95 | 100,3 |
| | | | 437ES 105 | 108,8 |

Die dargestellten Ergebnisse verdeutlichen, dass Pflanzen, die gleichzeitig fremde Nucleinsäuremoleküle, codierend eine Hyaluronansynthase und codierend ein Protein mit der Aktivität einer GlcN-6-P Acetyltransferase und codierend ein Protein mit der Aktivität einer UDP-GlcNAc Pyrophosphorylase aufweisen, deutlich höhere Mengen an Hyaluronan synthetisieren im Vergleich zu Pflanzen, die nur ein fremdes Nucleinsäuremolekül, codierend eine Hyaluronansynthase aufweisen.

**Tabelle 3: Menge an Hyaluronan (in µg Hyaluronan pro g Frischgewicht), das von unabhängigen, ausgewählten transgenen Pflanzen der Linie 497 ES bzw. 498 ES in gesamten oberirdischen Teilen der jeweiligen Pflanzen produziert wurde. Die jeweilige Spalte 1 bezeichnet die Pflanze, von welcher Material geerntet wurde. Die jeweilige Spalte 2 gibt die Menge Hyaluronan bezogen auf das eingesetzte Frischgewicht an.**

| Bezeichnung der Pflanze | HA [µg/g FG] | | Bezeichnung der Pflanze | HA [µg/g FG] |
|---|---|---|---|---|
| 397 ES 1 | 4,51 | | 398 ES 1 | 53,04 |
| 397 ES 2 | 22,54 | | 398 ES 2 | 43,64 |
| 397 ES 3 | 16,27 | | 398 ES 3 | 130,01 |
| 397 ES 5 | 10,13 | | 398 ES 4 | 89,26 |
| 397 ES 6 | 13,60 | | 398 ES 5 | 74,35 |
| 397 ES 7 | 33,62 | | 398 ES 6 | 55,39 |
| 397 ES 8 | 19,87 | | 398 ES 7 | 99,61 |
| 397 ES 9 | 70,37 | | 398 ES 8 | 90,82 |
| 397 ES 10 | 51,91 | | 398 ES 9 | 41,80 |
| 397 ES 11 | 78,71 | | 398 ES 10 | 79,66 |
| 397 ES 12 | 9,76 | | 398 ES 11 | 9,57 |
| 397 ES 13 | 6,62 | | 398 ES 12 | 41,24 |
| 397 ES 14 | 32,74 | | 398 ES 13 | 89,05 |
| 397 ES 16 | 21,34 | | 398 ES 14 | 77,19 |
| 397 ES 17 | 81,90 | | 398 ES 15 | 96,96 |
| 397 ES 18 | 33,16 | | 398 ES 16 | 84,24 |
| 397 ES 19 | 32,18 | | 398 ES 17 | 124,63 |
| 397 ES 20 | 26,67 | | 398 ES 18 | 76,19 |
| 397 ES 21 | 63,21 | | 398 ES 19 | 71,45 |
| 397 ES 22 | 2,66 | | 398 ES 20 | 46,17 |
| 397 ES 24 | 1,41 | | 398 ES 21 | 80,96 |
| 397 ES 25 | 37,27 | | 398 ES 22 | 31,25 |
| 397 ES 26 | 20,34 | | 398 ES 23 | 87,35 |
| 397 ES 27 | 32,89 | | 398 ES 24 | 62,31 |
| 397 ES 28 | 9,89 | | 398 ES 25 | 58,98 |
| 397 ES 29 | 8,31 | | 398 ES 26 | 71,14 |
| 397 ES 30 | 85,77 | | 398 ES 27 | 18,48 |
| 397 ES 31 | 47,44 | | 398 ES 28 | 105,85 |
| 397 ES 32 | 53,47 | | 398 ES 29 | 12,24 |
| 397 ES 33 | 5,25 | | 398 ES 30 | 95,82 |
| 397 ES 34 | 17,10 | | 398 ES 31 | 18,43 |
| 397 ES 35 | 16,80 | | 398 ES 32 | 109,41 |
| 397 ES 36 | 17,53 | | 398 ES 33 | 87,10 |
| 397 ES 37 | 25,90 | | 398 ES 34 | 88,30 |
| 397 ES 38 | 7,68 | | 398 ES 35 | 97,85 |
| 397 ES 39 | 0,49 | | 398 ES 36 | 58,51 |
| 397 ES 40 | 0,85 | | 398 ES 37 | 78,51 |
| 397 ES 41 | 14,65 | | 398 ES 38 | 99,67 |
| 397 ES 42 | 35,36 | | 398 ES 39 | 11,28 |
| 397 ES 43 | 49,96 | | 398 ES 40 | 29,12 |
| 397 ES 44 | 28,78 | | 398 ES 41 | 74,15 |
| 397 ES 45 | 18,95 | | 398 ES 42 | 22,75 |
| 397 ES 46 | 7,93 | | 398 ES 43 | 77,05 |
| 397 ES 47 | 28,28 | | 398 ES 44 | 101,34 |
| 397 ES 48 | 13,94 | | 398 ES 45 | 57,16 |
| 397 ES 49 | 60,16 | | 398 ES 46 | 54,19 |
| 397 ES 50 | 29,77 | | 398 ES 47 | 64,35 |
| | | | 398 ES 48 | 60,04 |
| | | | 398 ES 49 | 77,36 |
| | | | 398 ES 50 | 50,84 |

Die dargestellten Ergebnisse verdeutlichen, dass Pflanzen, die gleichzeitig fremde Nucleinsäuremoleküle, codierend eine Hyaluronansynthase und codierend ein Protein mit der Aktivität einer GlcN-P Mutase und ein Protein mit der bifunktionalen Aktivität einer GlcN-1-P Acetyltransferase und einer UDP-GIcNAc Pyrophosphorylase aufweisen, keine signifikant höheren Mengen an Hyaluronan synthetisieren im Vergleich zu Pflanzen, die nur ein fremdes Nucleinsäuremolekül, codierend eine Hyaluronansynthase aufweisen.

**Tabelle 4: Menge an Hyaluronan (in µg Hyaluronan pro g Frischgewicht), das von unabhängigen, ausgewählten transgenen Pflanzen der Linie 444 ES bzw. 445 ES in gesamten oberirdischen Teilen der jeweiligen Pflanzen produziert wurde. Die jeweilige Spalte 1 bezeichnet die Pflanze, von welcher Material geerntet wurde. Die jeweilige Spalte 2 gibt die Menge Hyaluronan bezogen auf das eingesetzte Frischgewicht an.**

| Bezeichnung der Pflanze | HA [µg/g FG] | | Bezeichnung der Pflanze | HA [µg/g FG] |
|---|---|---|---|---|
| 444ES 6 | 81,06 | | 445ES 1 | 102,53 |
| 444ES 12 | 95,89 | | 445ES 6 | 99,65 |
| 444ES 14 | 90,76 | | 445ES 7 | 152,66 |
| 444ES 17 | 83,20 | | 445ES 8 | 82,24 |
| 444ES 19 | 69,18 | | 445ES 9 | 119,74 |
| 444ES 23 | 59,45 | | 445ES 12 | 82,68 |
| 444ES 27 | 58,93 | | 445ES 16 | 102,24 |
| 444ES 35 | 58,45 | | 445ES 18 | 86,47 |
| 444ES 37 | 67,34 | | 445ES 19 | 103,37 |
| 444ES 41 | 63,27 | | 445ES 20 | 96,06 |
| 444ES 43 | 60,82 | | 445ES 23 | 116,64 |
| | | | 445ES 24 | 95,13 |
| | | | 445ES 34 | 87,24 |
| | | | 445ES 40 | 81,74 |
| | | | 445ES 42 | 98,72 |
| | | | 445ES 47 | 84,41 |
| | | | 445ES 60 | 86,71 |
| | | | 445ES 135 | 94,13 |

Die dargestellten Ergebnisse verdeutlichen, dass Pflanzen, die gleichzeitig fremde Nucleinsäuremoleküle, codierend eine Hyaluronansynthase und codierend ein Protein mit der Aktivität einer GlcN-P Mutase und ein Protein mit der Aktivität einer GlcN-6-P Acetyltransferase und ein Protein mit der Aktivität einer UDP-GIcNAc Pyrophosphorylase aufweisen, keine signifikant höheren Mengen an Hyaluronan synthetisieren im Vergleich zu Pflanzen, die nur ein fremdes Nucleinsäuremolekül, codierend eine Hyaluronansynthase und ein Protein mit der Aktivität einer GlcN-6-P Acetyltransferase und ein Protein mit der Aktivität einer UDP-GlcNAc Pyrophosphorylase aufweisen.

## Patentansprüche

1. Genetisch modifizierte Pflanzenzelle, die ein fremdes Nucleinsäuremolekül, codierend eine Glucosaminglycansynthase aufweist, **dadurch gekennzeichnet, dass** besagte Pflanzenzelle zusätzlich ein fremdes Nucleinsäuremolekül, codierend ein Protein mit der Aktivität einer Glucosamin-6-Phosphat-Acetyltransferase und ein fremdes Nucleinsäuremolekül, codierend ein Protein mit der Aktivität einer monofunktionalen UDP-N-acetyl-Glucosamin Pyrophosphorylase aufweist.

2. Pflanze enthaltend genetisch modifizierte Pflanzenzellen nach Anspruch 1.

3. Vermehrungsmaterial von Pflanzen nach Anspruch 2, enthaltend genetisch modifizierte Pflanzenzellen nach Anspruch 1.

4. Erntebare Pflanzenteile von Pflanzen nach Anspruch 2, enthaltend genetisch modifizierte Pflanzenzellen nach Anspruch 1.

5. Verfahren zur Herstellung einer Pflanze, worin
a) eine Pflanzenzelle, genetisch modifiziert wird, wobei die genetische Modifikation die folgenden Schritte i bis iii umfasst
i) Einführung eines Nucleinsäuremoleküls, codierend ein Protein mit der Aktivität einer Glucosaminglycansynthase in eine Pflanzenzelle
ii) Einführung eines Nucleinsäuremoleküls, codierend ein Protein mit der Aktivität einer Glucosamin-6-Phosphat-Acetyltransferase in eine Pflanzenzelle
iii) Einführung eines Nucleinsäuremoleküls, codierend ein Protein mit der monofunktionalen Aktivität einer UDP-N-acetyl-Glucosamin Pyrophosphorylase in eine Pflanzenzelle
b) aus Pflanzenzellen von Schritt a) i und/oder a) ii und/oder a)iii eine Pflanze regeneriert wird;
c) gegebenenfalls weitere Pflanzen mit Hilfe der Pflanzen nach Schritt b) erzeugt werden,
wobei die Schritte a) i bis a) iii in beliebiger Reihenfolge, einzeln oder beliebige Kombinationen der Schritte a) i bis a) iii gleichzeitig durchgeführt werden können und gegebenenfalls in Pflanzenzellen der nach den Schritten b) oder c) erhaltenen Pflanzen die noch fehlenden fremden Nucleinsäuremoleküle der Schritte a) i bis a) iii eingeführt werden.

6. Verfahren zur Herstellung von Glcosaminglycanen, umfassend den Schritt der Extraktion von Glcosaminglycanen aus genetisch modifizierten Pflanzenzellen nach Anspruch 1, aus Pflanzen nach Anspruch 2 aus Vermehrungsmaterial nach Anspruch 3 aus erntebaren Pflanzenteilen nach Anspruch 4 oder aus Pflanzen erhältlich nach einem Verfahren nach Anspruch 5.

7. Verwendung einer genetisch modifizierten Pflanzenzelle nach Anspruch 1, einer Pflanze nach Anspruch 2, Vermehrungsmaterial nach Anspruch 3, erntebaren Pflanzenteilen nach Anspruch 4 oder von Pflanzen, erhältlich nach einem Verfahren nach Anspruch 5 zur Herstellung von Glcosaminglycanen.

8. Zusammensetzung enthaltend genetisch modifizierte Pflanzenzellen nach Anspruch 1.

9. Verfahren zur Herstellung einer Zusammensetzung enthaltend Glcosaminglycane, worin genetisch modifizierte Pflanzenzellen nach Anspruch 1, Pflanzen nach Anspruch 2, Vermehrungsmaterial nach Anspruch 3, erntebare Pflanzenteile nach Anspruch 4 oder Pflanzen erhältlich nach einem Verfahren nach Anspruch 5 verwendet werden.

10. Verwendung von genetisch modifizierte Pflanzenzellen nach Anspruch 1, von Pflanzen nach Anspruch 2, von Vermehrungsmaterial nach Anspruch 3, von erntebaren Pflanzenteilen nach Anspruch 4 oder von Pflanzen, erhältlich nach einem Verfahren nach Anspruch 5, zur Herstellung einer Zusammensetzung nach Anspruch 8.


SEQUENCE LISTING

<110>  Bayer CropScience AG

<120>  Pflanzen, die erhöhte Mengen an Glucosaminglycanen synthetisieren

<130>  BCS 07-5007 EP

<160>  30

<170>  PatentIn version 3.3

<210>  1
<211>  1707
<212>  DNA
<213>  Paramecium bursaria Chlorella Virus 1

<220>
<221>  CDS
<222>  (1)..(1707)

<300>
<308>  GenBank/U4258ß
<309>  1995-12-24
<313>  (50903)..(52609)

<400>  1
atg ggt aaa aat ata atc ata atg gtt tcg tgg tac acc atc ata act       48
Met Gly Lys Asn Ile Ile Ile Met Val Ser Trp Tyr Thr Ile Ile Thr
1               5                   10                  15

tca aat cta atc gcg gtt gga gga gcc tct cta atc ttg gct ccg gca       96
Ser Asn Leu Ile Ala Val Gly Gly Ala Ser Leu Ile Leu Ala Pro Ala
            20                  25                  30

att act ggg tat gtt cta cat tgg aat att gct ctc tcg aca atc tgg      144
Ile Thr Gly Tyr Val Leu His Trp Asn Ile Ala Leu Ser Thr Ile Trp
        35                  40                  45

gga gta tca gct tat ggt att ttc gtt ttt ggg ttt ttc ctt gca caa      192
Gly Val Ser Ala Tyr Gly Ile Phe Val Phe Gly Phe Phe Leu Ala Gln
    50                  55                  60

gtt tta ttt tca gaa ctg aac agg aaa cgt ctt cgc aag tgg att tct      240
Val Leu Phe Ser Glu Leu Asn Arg Lys Arg Leu Arg Lys Trp Ile Ser
65                  70                  75                  80

ctc aga cct aag ggt tgg aat gat gtt cgt ttg gct gtg atc att gct      288

Leu Arg Pro Lys Gly Trp Asn Asp Val Arg Leu Ala Val Ile Ile Ala
                85                  90                  95

gga tat cgc gag gat cct tat atg ttc cag aag tgc ctc gag tct gta      336
Gly Tyr Arg Glu Asp Pro Tyr Met Phe Gln Lys Cys Leu Glu Ser Val
            100                 105                 110

cgt gac tct gat tat ggc aac gtt gcc cgt ctg att tgt gtg att gac      384
Arg Asp Ser Asp Tyr Gly Asn Val Ala Arg Leu Ile Cys Val Ile Asp
        115                 120                 125

ggt gat gag gac gat gat atg agg atg gct gcc gtt tac aag gcg atc      432
Gly Asp Glu Asp Asp Asp Met Arg Met Ala Ala Val Tyr Lys Ala Ile
    130                 135                 140

tac aat gat aat atc aag aag ccc gag ttt gtt ctg tgt gag tca gac      480
Tyr Asn Asp Asn Ile Lys Lys Pro Glu Phe Val Leu Cys Glu Ser Asp
145                 150                 155                 160

gac aag gaa ggt gaa cgc atc gac tct gat ttc tct cgc gac att tgt      528
Asp Lys Glu Gly Glu Arg Ile Asp Ser Asp Phe Ser Arg Asp Ile Cys
                165                 170                 175

gtc ctc cag cct cat cgt gga aaa cgg gag tgt ctt tat act ggg ttt      576
Val Leu Gln Pro His Arg Gly Lys Arg Glu Cys Leu Tyr Thr Gly Phe
            180                 185                 190

caa ctt gca aag atg gac ccc agt gtc aat gct gtc gtt ctg att gac      624
Gln Leu Ala Lys Met Asp Pro Ser Val Asn Ala Val Val Leu Ile Asp
        195                 200                 205

agc gat acc gtt ctc gag aag gat gct att ctg gaa gtt gta tac cca      672
Ser Asp Thr Val Leu Glu Lys Asp Ala Ile Leu Glu Val Val Tyr Pro
    210                 215                 220

ctt gca tgc gat ccc gag atc caa gcc gtt gca ggt gag tgt aag att      720
Leu Ala Cys Asp Pro Glu Ile Gln Ala Val Ala Gly Glu Cys Lys Ile
225                 230                 235                 240

tgg aac aca gac act ctt ttg agt ctt ctc gtc gct tgg cgg tac tat      768
Trp Asn Thr Asp Thr Leu Leu Ser Leu Leu Val Ala Trp Arg Tyr Tyr
                245                 250                 255

tct gcg ttt tgt gtg gag agg agt gcc cag tct ttt ttc agg act gtt      816
Ser Ala Phe Cys Val Glu Arg Ser Ala Gln Ser Phe Phe Arg Thr Val
            260                 265                 270

cag tgc gtt ggg ggg cca ctg ggt gcc tac aag att gat atc att aag      864
Gln Cys Val Gly Gly Pro Leu Gly Ala Tyr Lys Ile Asp Ile Ile Lys

275                 280                 285

gag att aag gac ccc tgg att tcc cag cgc ttt ctt ggt cag aag tgt       912
Glu Ile Lys Asp Pro Trp Ile Ser Gln Arg Phe Leu Gly Gln Lys Cys
    290                 295                 300

act tac ggt gac gac cgc cgg cta acc aac gag atc ttg atg cgt ggt       960
Thr Tyr Gly Asp Asp Arg Arg Leu Thr Asn Glu Ile Leu Met Arg Gly
305                 310                 315                 320

aaa aag gtt gtg ttc act cca ttt gct gtt ggt tgg tct gac agt ccg      1008
Lys Lys Val Val Phe Thr Pro Phe Ala Val Gly Trp Ser Asp Ser Pro
                325                 330                 335

acc aat gtg ttt cgg tac atc gtt cag cag acc cgc tgg agt aag tcg      1056
Thr Asn Val Phe Arg Tyr Ile Val Gln Gln Thr Arg Trp Ser Lys Ser
            340                 345                 350

tgg tgc cgc gaa att tgg tac acc ctc ttc gcc gcg tgg aag cac ggt      1104
Trp Cys Arg Glu Ile Trp Tyr Thr Leu Phe Ala Ala Trp Lys His Gly
        355                 360                 365

ttg tct gga att tgg ctg gcc ttt gaa tgt ttg tat caa att aca tac      1152
Leu Ser Gly Ile Trp Leu Ala Phe Glu Cys Leu Tyr Gln Ile Thr Tyr
    370                 375                 380

ttc ttc ctc gtg att tac ctc ttt tct cgc cta gcc gtt gag gcc gac      1200
Phe Phe Leu Val Ile Tyr Leu Phe Ser Arg Leu Ala Val Glu Ala Asp
385                 390                 395                 400

cct cgc gcc cag aca gcc acg gtg att gtg agc acc acg gtt gca ttg      1248
Pro Arg Ala Gln Thr Ala Thr Val Ile Val Ser Thr Thr Val Ala Leu
                405                 410                 415

att aag tgt ggg tat ttt tca ttc cga gcc aag gat att cgg gcg ttt      1296
Ile Lys Cys Gly Tyr Phe Ser Phe Arg Ala Lys Asp Ile Arg Ala Phe
            420                 425                 430

tac ttt gtg ctt tat aca ttt gtt tac ttt ttc tgt atg att ccg gcc      1344
Tyr Phe Val Leu Tyr Thr Phe Val Tyr Phe Phe Cys Met Ile Pro Ala
        435                 440                 445

agg att act gca atg atg acg ctt tgg gac att ggc tgg ggt act cgc      1392
Arg Ile Thr Ala Met Met Thr Leu Trp Asp Ile Gly Trp Gly Thr Arg
    450                 455                 460

ggt gga aac gag aag cct tcc gtt ggc acc cgg gtc gct ctg tgg gca      1440
Gly Gly Asn Glu Lys Pro Ser Val Gly Thr Arg Val Ala Leu Trp Ala
465                 470                 475                 480

aag caa tat ctc att gca tat atg tgg tgg gcc gcg gtt gtt ggc gct 1488
Lys Gln Tyr Leu Ile Ala Tyr Met Trp Trp Ala Ala Val Val Gly Ala
                485                 490                 495

gga gtt tac agc atc gtc cat aac tgg atg ttc gat tgg aat tct ctt 1536
Gly Val Tyr Ser Ile Val His Asn Trp Met Phe Asp Trp Asn Ser Leu
            500                 505                 510

tct tat cgt ttt gct ttg gtt ggt att tgt tct tac att gtt ttt att 1584
Ser Tyr Arg Phe Ala Leu Val Gly Ile Cys Ser Tyr Ile Val Phe Ile
        515                 520                 525

gtt att gtg ctg gtg gtt tat ttc acc ggc aaa att acg act tgg aat 1632
Val Ile Val Leu Val Val Tyr Phe Thr Gly Lys Ile Thr Thr Trp Asn
    530                 535                 540

ttc acg aag ctt cag aag gag cta atc gag gat cgc gtt ctg tac gat 1680
Phe Thr Lys Leu Gln Lys Glu Leu Ile Glu Asp Arg Val Leu Tyr Asp
545                 550                 555                 560

gca act acc aat gct cag tct gtg tga 1707
Ala Thr Thr Asn Ala Gln Ser Val
                565

<210> 2
<211> 568
<212> PRT
<213> Paramecium bursaria Chlorella Virus 1

<400> 2

Met Gly Lys Asn Ile Ile Ile Met Val Ser Trp Tyr Thr Ile Ile Thr
1               5                   10                  15

Ser Asn Leu Ile Ala Val Gly Gly Ala Ser Leu Ile Leu Ala Pro Ala
            20                  25                  30

Ile Thr Gly Tyr Val Leu His Trp Asn Ile Ala Leu Ser Thr Ile Trp
        35                  40                  45

Gly Val Ser Ala Tyr Gly Ile Phe Val Phe Gly Phe Phe Leu Ala Gln
    50                  55                  60

Val Leu Phe Ser Glu Leu Asn Arg Lys Arg Leu Arg Lys Trp Ile Ser
65                  70                  75                  80

Leu Arg Pro Lys Gly Trp Asn Asp Val Arg Leu Ala Val Ile Ile Ala
                85                  90                  95

Gly Tyr Arg Glu Asp Pro Tyr Met Phe Gln Lys Cys Leu Glu Ser Val
            100                 105                 110

Arg Asp Ser Asp Tyr Gly Asn Val Ala Arg Leu Ile Cys Val Ile Asp
        115                 120                 125

Gly Asp Glu Asp Asp Asp Met Arg Met Ala Ala Val Tyr Lys Ala Ile
    130                 135                 140

Tyr Asn Asp Asn Ile Lys Lys Pro Glu Phe Val Leu Cys Glu Ser Asp
145                 150                 155                 160

Asp Lys Glu Gly Glu Arg Ile Asp Ser Asp Phe Ser Arg Asp Ile Cys
                165                 170                 175

Val Leu Gln Pro His Arg Gly Lys Arg Glu Cys Leu Tyr Thr Gly Phe
            180                 185                 190

Gln Leu Ala Lys Met Asp Pro Ser Val Asn Ala Val Val Leu Ile Asp
        195                 200                 205

Ser Asp Thr Val Leu Glu Lys Asp Ala Ile Leu Glu Val Val Tyr Pro
    210                 215                 220

Leu Ala Cys Asp Pro Glu Ile Gln Ala Val Ala Gly Glu Cys Lys Ile
225                 230                 235                 240

Trp Asn Thr Asp Thr Leu Leu Ser Leu Leu Val Ala Trp Arg Tyr Tyr
                245                 250                 255

Ser Ala Phe Cys Val Glu Arg Ser Ala Gln Ser Phe Phe Arg Thr Val

            260                 265                 270

Gln Cys Val Gly Gly Pro Leu Gly Ala Tyr Lys Ile Asp Ile Ile Lys
        275                 280                 285

Glu Ile Lys Asp Pro Trp Ile Ser Gln Arg Phe Leu Gly Gln Lys Cys
    290                 295                 300

Thr Tyr Gly Asp Asp Arg Arg Leu Thr Asn Glu Ile Leu Met Arg Gly
305                 310                 315                 320

Lys Lys Val Val Phe Thr Pro Phe Ala Val Gly Trp Ser Asp Ser Pro
                325                 330                 335

Thr Asn Val Phe Arg Tyr Ile Val Gln Gln Thr Arg Trp Ser Lys Ser
            340                 345                 350

Trp Cys Arg Glu Ile Trp Tyr Thr Leu Phe Ala Ala Trp Lys His Gly
        355                 360                 365

Leu Ser Gly Ile Trp Leu Ala Phe Glu Cys Leu Tyr Gln Ile Thr Tyr
    370                 375                 380

Phe Phe Leu Val Ile Tyr Leu Phe Ser Arg Leu Ala Val Glu Ala Asp
385                 390                 395                 400

Pro Arg Ala Gln Thr Ala Thr Val Ile Val Ser Thr Thr Val Ala Leu
                405                 410                 415

Ile Lys Cys Gly Tyr Phe Ser Phe Arg Ala Lys Asp Ile Arg Ala Phe
            420                 425                 430

Tyr Phe Val Leu Tyr Thr Phe Val Tyr Phe Phe Cys Met Ile Pro Ala
        435                 440                 445

Arg Ile Thr Ala Met Met Thr Leu Trp Asp Ile Gly Trp Gly Thr Arg
    450                 455                 460

Gly Gly Asn Glu Lys Pro Ser Val Gly Thr Arg Val Ala Leu Trp Ala
465                 470                 475                 480

Lys Gln Tyr Leu Ile Ala Tyr Met Trp Trp Ala Ala Val Val Gly Ala
                485                 490                 495

Gly Val Tyr Ser Ile Val His Asn Trp Met Phe Asp Trp Asn Ser Leu
            500                 505                 510

Ser Tyr Arg Phe Ala Leu Val Gly Ile Cys Ser Tyr Ile Val Phe Ile
        515                 520                 525

Val Ile Val Leu Val Val Tyr Phe Thr Gly Lys Ile Thr Thr Trp Asn
    530                 535                 540

Phe Thr Lys Leu Gln Lys Glu Leu Ile Glu Asp Arg Val Leu Tyr Asp
545                 550                 555                 560

Ala Thr Thr Asn Ala Gln Ser Val
                565

<210>  3
<211>  1707
<212>  DNA
<213>  Artificial

<220>
<223>  Synthetic nucleotide sequence coding for a Paramecium bursaria
       Chlorella Virus 1 Hyaluronansynthase protein

<400>  3
atgggtaaga acattatcat tatggtgtcc tggtacacaa ttattacaag taatctcatc      60

gcagttggtg gtgcatctct tattctcgct ccagctatca ctggatatgt tcttcactgg     120

aacatcgccc tctcaactat ttggggagtt tccgcatatg gtatttttgt tttcgggttc     180

tttttggctc aggttctgtt ctcagagctc aatcgtaaga gactcaggaa gtggattagc     240

cttagaccaa aggggtggaa tgacgttcgt ctcgctgtca ttatcgctgg ctaccgtgaa     300

gatccttaca tgtttcaaaa gtgcttggaa tcagttaggg atagtgatta tggcaacgtc 360

gctagactga tctgtgtgat tgatggagat gaggacgacg atatgaggat ggcagctgtt 420

tataaggcta tctataatga taacattaag aagcctgaat ttgttctttg cgagtctgat 480

gacaaggaag gagaacggat tgattcagat ttctcacgtg atatctgcgt tctccaacct 540

catcgtggga agcgtgaatg tctttataca ggtttccaac tcgccaaaat ggacccatca 600

gtgaacgctg tggttcttat cgatagtgat actgtgctgg agaaagatgc tatcttggag 660

gttgtttacc ctcttgcctg tgatcctgaa attcaagctg tggctggaga gtgcaagatc 720

tggaacacag atactcttct ttctctgctt gtcgcatgga gatattactc cgcattctgt 780

gtggagagga gcgctcaatc ctttttccgt accgttcaat gcgttggtgg tcctttggga 840

gcttacaaaa ttgatatcat caaggagatt aaggacccat ggattagtca aaggtttctt 900

ggtcagaagt gcacttatgg cgatgatcgt agattgacta acgaaatcct tatgaggggc 960

aagaaagtcg tttttactcc atttgctgtc ggatggtctg attcacctac aaatgttttc 1020

cgttatattg tgcaacaaac acgttggagt aagagctggt gtagggagat ctggtacact 1080

ttgttcgctg cttggaagca cgggcttagc ggaatttggc ttgcttttga atgcctttac 1140

cagattacat acttttcttt ggtgatctat ttgttttcac gtcttgccgt cgaggctgac 1200

cctagagcac agactgcaac tgtgattgtt tctactacag tcgcacttat taagtgtggc 1260

tatttcagtt ttagagcaaa agatattaga gccttctatt ttgttttgta cacatttgtt 1320

tatttctttt gcatgattcc agctcgtatt accgctatga tgaccttgtg ggacatcgga 1380

tggggaacta gaggtggtaa cgaaaagcct tctgtgggaa caagggtggc cctttgggca 1440

aaacaatatc tcatcgccta catgtggtgg gccgctgtcg ttggtgccgg agtgtactca 1500

atcgttcata actggatgtt tgactggaac tctttgagct atcgtttcgc tcttgtgggt 1560

atttgttctt acattgtttt catcgtgatt gtgctcgttg tgtatttcac tggtaaaatc 1620

acaacctgga atttcactaa acttcaaaag gaattgattg aagacagggt tctgtatgat 1680

gctactacca acgcccagtc agtttaa 1707

<210>  4
<211>  2895
<212>  DNA
<213>  Pasteurella multocida

<220>
<221>  CDS
<222>  (1)..(2895)

<300>
<308>  EMBL/AF195517
<309>  2000-09-07
<313>  (61)..(2955)

<400>  4
atg aat aca tta tca caa gca ata aaa gca tat aac agc aat gac tat       48
Met Asn Thr Leu Ser Gln Ala Ile Lys Ala Tyr Asn Ser Asn Asp Tyr
1               5                   10                  15

gaa tta gca ctc aaa tta ttt gag aag tct gct gaa acc tac ggg cga       96
Glu Leu Ala Leu Lys Leu Phe Glu Lys Ser Ala Glu Thr Tyr Gly Arg
            20                  25                  30

aaa atc gtt gaa ttc caa att atc aaa tgt aaa gaa aaa ctc tcg acc      144
Lys Ile Val Glu Phe Gln Ile Ile Lys Cys Lys Glu Lys Leu Ser Thr
        35                  40                  45

aat tct tat gta agt gaa gat aaa aaa aac agt gtt tgc gat agc tca      192
Asn Ser Tyr Val Ser Glu Asp Lys Lys Asn Ser Val Cys Asp Ser Ser
    50                  55                  60

tta gat atc gca aca cag ctc tta ctt tcc aac gta aaa aaa tta act      240
Leu Asp Ile Ala Thr Gln Leu Leu Leu Ser Asn Val Lys Lys Leu Thr
65                  70                  75                  80

cta tcc gaa tca gaa aaa aac agt tta aaa aat aaa tgg aaa tct atc      288
Leu Ser Glu Ser Glu Lys Asn Ser Leu Lys Asn Lys Trp Lys Ser Ile
                85                  90                  95

act ggg aaa aaa tcg gag aac gca gaa atc aga aag gtg gaa cta gta      336
Thr Gly Lys Lys Ser Glu Asn Ala Glu Ile Arg Lys Val Glu Leu Val
            100                 105                 110

ccc aaa gat ttt cct aaa gat ctt gtt ctt gct cca ttg cca gat cat      384
Pro Lys Asp Phe Pro Lys Asp Leu Val Leu Ala Pro Leu Pro Asp His
        115                 120                 125

gtt aat gat ttt aca tgg tac aaa aat cga aaa aaa agc tta ggt ata        432
Val Asn Asp Phe Thr Trp Tyr Lys Asn Arg Lys Lys Ser Leu Gly Ile
    130                 135                 140

aag cct gta aat aag aat atc ggt ctt tct att att att cct aca ttt        480
Lys Pro Val Asn Lys Asn Ile Gly Leu Ser Ile Ile Ile Pro Thr Phe
145                 150                 155                 160

aat cgt agc cgt att tta gat ata acg tta gcc tgt ttg gtc aat cag        528
Asn Arg Ser Arg Ile Leu Asp Ile Thr Leu Ala Cys Leu Val Asn Gln
                165                 170                 175

aaa aca aac tac cca ttt gaa gtc gtt gtt gca gat gat ggt agt aag        576
Lys Thr Asn Tyr Pro Phe Glu Val Val Val Ala Asp Asp Gly Ser Lys
            180                 185                 190

gaa aac tta ctt acc att gtg caa aaa tac gaa caa aaa ctt gac ata        624
Glu Asn Leu Leu Thr Ile Val Gln Lys Tyr Glu Gln Lys Leu Asp Ile
        195                 200                 205

aag tat gta aga caa aaa gat tat gga tat caa ttg tgt gca gtc aga        672
Lys Tyr Val Arg Gln Lys Asp Tyr Gly Tyr Gln Leu Cys Ala Val Arg
    210                 215                 220

aac tta ggt tta cgt aca gca aag tat gat ttt gtc tcg att cta gac        720
Asn Leu Gly Leu Arg Thr Ala Lys Tyr Asp Phe Val Ser Ile Leu Asp
225                 230                 235                 240

tgc gat atg gca cca caa caa tta tgg gtt cat tct tat ctt aca gaa        768
Cys Asp Met Ala Pro Gln Gln Leu Trp Val His Ser Tyr Leu Thr Glu
                245                 250                 255

cta tta gaa gac aat gat att gtt tta att gga cct aga aaa tat gtg        816
Leu Leu Glu Asp Asn Asp Ile Val Leu Ile Gly Pro Arg Lys Tyr Val
            260                 265                 270

gat act cat aat att acc gca gaa caa ttc ctt aac gat cca tat tta        864
Asp Thr His Asn Ile Thr Ala Glu Gln Phe Leu Asn Asp Pro Tyr Leu
        275                 280                 285

ata gaa tca cta cct gaa acc gct aca aat aac aat cct tcg att aca        912
Ile Glu Ser Leu Pro Glu Thr Ala Thr Asn Asn Asn Pro Ser Ile Thr
    290                 295                 300

tca aaa gga aat ata tcg ttg gat tgg aga tta gaa cat ttc aaa aaa        960
Ser Lys Gly Asn Ile Ser Leu Asp Trp Arg Leu Glu His Phe Lys Lys
305                 310                 315                 320

acc gat aat cta cgt cta tgt gat tct ccg ttt cgt tat ttt agt tgc       1008

Thr Asp Asn Leu Arg Leu Cys Asp Ser Pro Phe Arg Tyr Phe Ser Cys
                325                 330                 335

ggt aat gtt gca ttt tct aaa gaa tgg cta aat aaa gta ggt tgg ttc      1056
Gly Asn Val Ala Phe Ser Lys Glu Trp Leu Asn Lys Val Gly Trp Phe
            340                 345                 350

gat gaa gaa ttt aat cat tgg ggg ggc gaa gat gta gaa ttt ggt tac      1104
Asp Glu Glu Phe Asn His Trp Gly Gly Glu Asp Val Glu Phe Gly Tyr
        355                 360                 365

aga tta ttt gcc aaa ggc tgt ttt ttc aga gta att gac ggc gga atg      1152
Arg Leu Phe Ala Lys Gly Cys Phe Phe Arg Val Ile Asp Gly Gly Met
    370                 375                 380

gca tac cat caa gaa cca cct ggt aaa gaa aat gaa aca gac cgc gaa      1200
Ala Tyr His Gln Glu Pro Pro Gly Lys Glu Asn Glu Thr Asp Arg Glu
385                 390                 395                 400

gct ggt aaa agt att acg ctt aaa att gtg aaa gaa aag gta cct tac      1248
Ala Gly Lys Ser Ile Thr Leu Lys Ile Val Lys Glu Lys Val Pro Tyr
                405                 410                 415

atc tat aga aag ctt tta cca ata gaa gat tca cat att cat aga ata      1296
Ile Tyr Arg Lys Leu Leu Pro Ile Glu Asp Ser His Ile His Arg Ile
            420                 425                 430

cct tta gtt tct att tat atc ccc gct tat aac tgt gca aat tat att      1344
Pro Leu Val Ser Ile Tyr Ile Pro Ala Tyr Asn Cys Ala Asn Tyr Ile
        435                 440                 445

caa aga tgt gta gat agt gct ctt aat caa act gtt gtc gat ctc gag      1392
Gln Arg Cys Val Asp Ser Ala Leu Asn Gln Thr Val Val Asp Leu Glu
    450                 455                 460

gtt tgt att tgt aac gat ggt tca aca gat aat acc tta gaa gtg atc      1440
Val Cys Ile Cys Asn Asp Gly Ser Thr Asp Asn Thr Leu Glu Val Ile
465                 470                 475                 480

aat aag ctt tat ggt aat aat cct agg gta cgc atc atg tct aaa cca      1488
Asn Lys Leu Tyr Gly Asn Asn Pro Arg Val Arg Ile Met Ser Lys Pro
                485                 490                 495

aat ggc gga ata gcc tca gca tca aat gca gcc gtt tct ttt gct aaa      1536
Asn Gly Gly Ile Ala Ser Ala Ser Asn Ala Ala Val Ser Phe Ala Lys
            500                 505                 510

ggt tat tac att ggg cag tta gat tca gat gat tat ctt gag cct gat      1584
Gly Tyr Tyr Ile Gly Gln Leu Asp Ser Asp Asp Tyr Leu Glu Pro Asp

515                 520                 525

gca gtt gaa ctg tgt tta aaa gaa ttt tta aaa gat aaa acg cta gct      1632
Ala Val Glu Leu Cys Leu Lys Glu Phe Leu Lys Asp Lys Thr Leu Ala
    530                 535                 540

tgt gtt tat acc act aat aga aac gtc aat ccg gat ggt agc tta atc      1680
Cys Val Tyr Thr Thr Asn Arg Asn Val Asn Pro Asp Gly Ser Leu Ile
545                 550                 555                 560

gct aat ggt tac aat tgg cca gaa ttt tca cga gaa aaa ctc aca acg      1728
Ala Asn Gly Tyr Asn Trp Pro Glu Phe Ser Arg Glu Lys Leu Thr Thr
                565                 570                 575

gct atg att gct cac cat ttt aga atg ttt acg att aga gct tgg cat      1776
Ala Met Ile Ala His His Phe Arg Met Phe Thr Ile Arg Ala Trp His
            580                 585                 590

tta acg gat gga ttt aac gaa aat att gaa aac gcc gtg gat tat gac      1824
Leu Thr Asp Gly Phe Asn Glu Asn Ile Glu Asn Ala Val Asp Tyr Asp
        595                 600                 605

atg ttc ctt aaa ctc agt gaa gtt gga aaa ttt aaa cat ctt aat aaa      1872
Met Phe Leu Lys Leu Ser Glu Val Gly Lys Phe Lys His Leu Asn Lys
    610                 615                 620

atc tgc tat aac cgc gta tta cat ggt gat aac aca tcc att aag aaa      1920
Ile Cys Tyr Asn Arg Val Leu His Gly Asp Asn Thr Ser Ile Lys Lys
625                 630                 635                 640

ctc ggc att caa aag aaa aac cat ttt gtt gta gtc aat cag tca tta      1968
Leu Gly Ile Gln Lys Lys Asn His Phe Val Val Val Asn Gln Ser Leu
                645                 650                 655

aat aga caa ggc atc aat tat tat aat tat gac aaa ttt gat gat tta      2016
Asn Arg Gln Gly Ile Asn Tyr Tyr Asn Tyr Asp Lys Phe Asp Asp Leu
            660                 665                 670

gat gaa agt aga aag tat atc ttc aat aaa acc gct gaa tat caa gaa      2064
Asp Glu Ser Arg Lys Tyr Ile Phe Asn Lys Thr Ala Glu Tyr Gln Glu
        675                 680                 685

gaa atg gat att tta aaa gat ctt aaa ctc att caa aat aaa gat gcc      2112
Glu Met Asp Ile Leu Lys Asp Leu Lys Leu Ile Gln Asn Lys Asp Ala
    690                 695                 700

aaa atc gca gtc agt att ttc tat ccc aat aca tta aac ggc tta gtg      2160
Lys Ile Ala Val Ser Ile Phe Tyr Pro Asn Thr Leu Asn Gly Leu Val
705                 710                 715                 720

aaa aaa cta aac aat att att gaa tat aat aaa aat ata ttc gtt att     2208
Lys Lys Leu Asn Asn Ile Ile Glu Tyr Asn Lys Asn Ile Phe Val Ile
                725                 730                 735

att cta cat gtt gat aag aat cat ctt aca cca gac atc aaa aaa gaa     2256
Ile Leu His Val Asp Lys Asn His Leu Thr Pro Asp Ile Lys Lys Glu
            740                 745                 750

ata ttg gct ttc tat cat aag cac caa gtg aat att tta cta aat aat     2304
Ile Leu Ala Phe Tyr His Lys His Gln Val Asn Ile Leu Leu Asn Asn
        755                 760                 765

gac atc tca tat tac acg agt aat aga cta ata aaa act gag gca cat     2352
Asp Ile Ser Tyr Tyr Thr Ser Asn Arg Leu Ile Lys Thr Glu Ala His
    770                 775                 780

tta agt aat att aat aaa tta agt cag tta aat cta aat tgt gaa tac     2400
Leu Ser Asn Ile Asn Lys Leu Ser Gln Leu Asn Leu Asn Cys Glu Tyr
785                 790                 795                 800

atc att ttt gat aat cat gac agc cta ttc gtt aaa aat gac agc tat     2448
Ile Ile Phe Asp Asn His Asp Ser Leu Phe Val Lys Asn Asp Ser Tyr
                805                 810                 815

gct tat atg aaa aaa tat gat gtc ggc atg aat ttc tca gca tta aca     2496
Ala Tyr Met Lys Lys Tyr Asp Val Gly Met Asn Phe Ser Ala Leu Thr
            820                 825                 830

cat gat tgg atc gag aaa atc aat gcg cat cca cca ttt aaa aag ctg     2544
His Asp Trp Ile Glu Lys Ile Asn Ala His Pro Pro Phe Lys Lys Leu
        835                 840                 845

att aaa acc tat ttt aat gac aat gac tta aga agt atg aat gtg aaa     2592
Ile Lys Thr Tyr Phe Asn Asp Asn Asp Leu Arg Ser Met Asn Val Lys
    850                 855                 860

ggg gca tca caa ggt atg ttt atg aag tat gcg cta ccg cat gag ctt     2640
Gly Ala Ser Gln Gly Met Phe Met Lys Tyr Ala Leu Pro His Glu Leu
865                 870                 875                 880

ctg acg att att aaa gaa gtc atc aca tcc tgc caa tca att gat agt     2688
Leu Thr Ile Ile Lys Glu Val Ile Thr Ser Cys Gln Ser Ile Asp Ser
                885                 890                 895

gtg cca gaa tat aac act gag gat att tgg ttc caa ttt gca ctt tta     2736
Val Pro Glu Tyr Asn Thr Glu Asp Ile Trp Phe Gln Phe Ala Leu Leu
            900                 905                 910

atc tta gaa aag aaa acc ggc cat gta ttt aat aaa aca tcg acc ctg      2784
Ile Leu Glu Lys Lys Thr Gly His Val Phe Asn Lys Thr Ser Thr Leu
        915                 920                 925

act tat atg cct tgg gaa cga aaa tta caa tgg aca aat gaa caa att      2832
Thr Tyr Met Pro Trp Glu Arg Lys Leu Gln Trp Thr Asn Glu Gln Ile
    930                 935                 940

caa agt gca aaa aaa ggc gaa aat atc ccc gtt aac aag ttc att att      2880
Gln Ser Ala Lys Lys Gly Glu Asn Ile Pro Val Asn Lys Phe Ile Ile
945                 950                 955                 960

aat agt ata acg cta                                                  2895
Asn Ser Ile Thr Leu
                965

<210>  5
<211>  965
<212>  PRT
<213>  Pasteurella multocida

<400>  5

Met Asn Thr Leu Ser Gln Ala Ile Lys Ala Tyr Asn Ser Asn Asp Tyr
1               5                   10                  15

Glu Leu Ala Leu Lys Leu Phe Glu Lys Ser Ala Glu Thr Tyr Gly Arg
            20                  25                  30

Lys Ile Val Glu Phe Gln Ile Ile Lys Cys Lys Glu Lys Leu Ser Thr
        35                  40                  45

Asn Ser Tyr Val Ser Glu Asp Lys Lys Asn Ser Val Cys Asp Ser Ser
    50                  55                  60

Leu Asp Ile Ala Thr Gln Leu Leu Leu Ser Asn Val Lys Lys Leu Thr
65                  70                  75                  80

Leu Ser Glu Ser Glu Lys Asn Ser Leu Lys Asn Lys Trp Lys Ser Ile
                85                  90                  95

Thr Gly Lys Lys Ser Glu Asn Ala Glu Ile Arg Lys Val Glu Leu Val

            100                 105                 110

Pro Lys Asp Phe Pro Lys Asp Leu Val Leu Ala Pro Leu Pro Asp His
        115                 120                 125

Val Asn Asp Phe Thr Trp Tyr Lys Asn Arg Lys Lys Ser Leu Gly Ile
    130                 135                 140

Lys Pro Val Asn Lys Asn Ile Gly Leu Ser Ile Ile Ile Pro Thr Phe
145                 150                 155                 160

Asn Arg Ser Arg Ile Leu Asp Ile Thr Leu Ala Cys Leu Val Asn Gln
                165                 170                 175

Lys Thr Asn Tyr Pro Phe Glu Val Val Val Ala Asp Asp Gly Ser Lys
            180                 185                 190

Glu Asn Leu Leu Thr Ile Val Gln Lys Tyr Glu Gln Lys Leu Asp Ile
        195                 200                 205

Lys Tyr Val Arg Gln Lys Asp Tyr Gly Tyr Gln Leu Cys Ala Val Arg
    210                 215                 220

Asn Leu Gly Leu Arg Thr Ala Lys Tyr Asp Phe Val Ser Ile Leu Asp
225                 230                 235                 240

Cys Asp Met Ala Pro Gln Gln Leu Trp Val His Ser Tyr Leu Thr Glu
                245                 250                 255

Leu Leu Glu Asp Asn Asp Ile Val Leu Ile Gly Pro Arg Lys Tyr Val
            260                 265                 270

Asp Thr His Asn Ile Thr Ala Glu Gln Phe Leu Asn Asp Pro Tyr Leu
        275                 280                 285

Ile Glu Ser Leu Pro Glu Thr Ala Thr Asn Asn Asn Pro Ser Ile Thr
    290                 295                 300

Ser Lys Gly Asn Ile Ser Leu Asp Trp Arg Leu Glu His Phe Lys Lys
305                 310                 315                 320

Thr Asp Asn Leu Arg Leu Cys Asp Ser Pro Phe Arg Tyr Phe Ser Cys
                325                 330                 335

Gly Asn Val Ala Phe Ser Lys Glu Trp Leu Asn Lys Val Gly Trp Phe
            340                 345                 350

Asp Glu Glu Phe Asn His Trp Gly Gly Glu Asp Val Glu Phe Gly Tyr
        355                 360                 365

Arg Leu Phe Ala Lys Gly Cys Phe Phe Arg Val Ile Asp Gly Gly Met
    370                 375                 380

Ala Tyr His Gln Glu Pro Pro Gly Lys Glu Asn Glu Thr Asp Arg Glu
385                 390                 395                 400

Ala Gly Lys Ser Ile Thr Leu Lys Ile Val Lys Glu Lys Val Pro Tyr
                405                 410                 415

Ile Tyr Arg Lys Leu Leu Pro Ile Glu Asp Ser His Ile His Arg Ile
            420                 425                 430

Pro Leu Val Ser Ile Tyr Ile Pro Ala Tyr Asn Cys Ala Asn Tyr Ile
        435                 440                 445

Gln Arg Cys Val Asp Ser Ala Leu Asn Gln Thr Val Val Asp Leu Glu
    450                 455                 460

Val Cys Ile Cys Asn Asp Gly Ser Thr Asp Asn Thr Leu Glu Val Ile
465                 470                 475                 480

Asn Lys Leu Tyr Gly Asn Asn Pro Arg Val Arg Ile Met Ser Lys Pro
                485                 490                 495

Asn Gly Gly Ile Ala Ser Ala Ser Asn Ala Ala Val Ser Phe Ala Lys
            500                 505                 510

Gly Tyr Tyr Ile Gly Gln Leu Asp Ser Asp Asp Tyr Leu Glu Pro Asp
        515                 520                 525

Ala Val Glu Leu Cys Leu Lys Glu Phe Leu Lys Asp Lys Thr Leu Ala
    530                 535                 540

Cys Val Tyr Thr Thr Asn Arg Asn Val Asn Pro Asp Gly Ser Leu Ile
545                 550                 555                 560

Ala Asn Gly Tyr Asn Trp Pro Glu Phe Ser Arg Glu Lys Leu Thr Thr
                565                 570                 575

Ala Met Ile Ala His His Phe Arg Met Phe Thr Ile Arg Ala Trp His
            580                 585                 590

Leu Thr Asp Gly Phe Asn Glu Asn Ile Glu Asn Ala Val Asp Tyr Asp
        595                 600                 605

Met Phe Leu Lys Leu Ser Glu Val Gly Lys Phe Lys His Leu Asn Lys
    610                 615                 620

Ile Cys Tyr Asn Arg Val Leu His Gly Asp Asn Thr Ser Ile Lys Lys
625                 630                 635                 640

Leu Gly Ile Gln Lys Lys Asn His Phe Val Val Val Asn Gln Ser Leu
                645                 650                 655

Asn Arg Gln Gly Ile Asn Tyr Tyr Asn Tyr Asp Lys Phe Asp Asp Leu
            660                 665                 670

Asp Glu Ser Arg Lys Tyr Ile Phe Asn Lys Thr Ala Glu Tyr Gln Glu
        675                 680                 685

Glu Met Asp Ile Leu Lys Asp Leu Lys Leu Ile Gln Asn Lys Asp Ala
    690                 695                 700

Lys Ile Ala Val Ser Ile Phe Tyr Pro Asn Thr Leu Asn Gly Leu Val
705                 710                 715                 720

Lys Lys Leu Asn Asn Ile Ile Glu Tyr Asn Lys Asn Ile Phe Val Ile
                725                 730                 735

Ile Leu His Val Asp Lys Asn His Leu Thr Pro Asp Ile Lys Lys Glu
            740                 745                 750

Ile Leu Ala Phe Tyr His Lys His Gln Val Asn Ile Leu Leu Asn Asn
        755                 760                 765

Asp Ile Ser Tyr Tyr Thr Ser Asn Arg Leu Ile Lys Thr Glu Ala His
    770                 775                 780

Leu Ser Asn Ile Asn Lys Leu Ser Gln Leu Asn Leu Asn Cys Glu Tyr
785                 790                 795                 800

Ile Ile Phe Asp Asn His Asp Ser Leu Phe Val Lys Asn Asp Ser Tyr
                805                 810                 815

Ala Tyr Met Lys Lys Tyr Asp Val Gly Met Asn Phe Ser Ala Leu Thr
            820                 825                 830

His Asp Trp Ile Glu Lys Ile Asn Ala His Pro Pro Phe Lys Lys Leu
        835                 840                 845

Ile Lys Thr Tyr Phe Asn Asp Asn Asp Leu Arg Ser Met Asn Val Lys
    850                 855                 860

Gly Ala Ser Gln Gly Met Phe Met Lys Tyr Ala Leu Pro His Glu Leu
865                 870                 875                 880

Leu Thr Ile Ile Lys Glu Val Ile Thr Ser Cys Gln Ser Ile Asp Ser

885                 890                 895

Val Pro Glu Tyr Asn Thr Glu Asp Ile Trp Phe Gln Phe Ala Leu Leu
            900                 905                 910

Ile Leu Glu Lys Lys Thr Gly His Val Phe Asn Lys Thr Ser Thr Leu
        915                 920                 925

Thr Tyr Met Pro Trp Glu Arg Lys Leu Gln Trp Thr Asn Glu Gln Ile
    930                 935                 940

Gln Ser Ala Lys Lys Gly Glu Asn Ile Pro Val Asn Lys Phe Ile Ile
945                 950                 955                 960

Asn Ser Ile Thr Leu
                965

<210>  6
<211>  1953
<212>  DNA
<213>  Pasteurella multocida

<220>
<221>  CDS
<222>  (1)..(1953)

<300>
<308>  EMBL/AY292199
<309>  2004-11-25
<313>  (1)..(1953)

<400>  6
atg aag gga aaa aaa gag atg act caa att caa ata gct aaa aat cca       48
Met Lys Gly Lys Lys Glu Met Thr Gln Ile Gln Ile Ala Lys Asn Pro
1               5                   10                  15

ccc caa cat gaa aaa gaa aat gaa ctc aac acc ttt caa aat aaa att       96
Pro Gln His Glu Lys Glu Asn Glu Leu Asn Thr Phe Gln Asn Lys Ile
            20                  25                  30

gat agt cta aaa aca act tta aac aaa gac att att tct caa caa act      144
Asp Ser Leu Lys Thr Thr Leu Asn Lys Asp Ile Ile Ser Gln Gln Thr

35                  40                  45

cta ttg gca aaa cag gac agt aaa cat ccg cta tcc gca tcc ctt gaa        192
Leu Leu Ala Lys Gln Asp Ser Lys His Pro Leu Ser Ala Ser Leu Glu
    50                  55                  60

aac gaa aat aaa ctt tta tta aaa caa ctc caa ttg gtt ctg caa gaa        240
Asn Glu Asn Lys Leu Leu Leu Lys Gln Leu Gln Leu Val Leu Gln Glu
65                  70                  75                  80

ttt aaa aaa ata tat acc tat aat caa gca tta gaa gca aag cta gaa        288
Phe Lys Lys Ile Tyr Thr Tyr Asn Gln Ala Leu Glu Ala Lys Leu Glu
                85                  90                  95

aaa gat aag caa aca aca tca ata aca gat tta tat aat gaa gtc gct        336
Lys Asp Lys Gln Thr Thr Ser Ile Thr Asp Leu Tyr Asn Glu Val Ala
            100                 105                 110

aaa agt gat tta ggg tta gtc aaa gaa acc aac agc gca aat cca tta        384
Lys Ser Asp Leu Gly Leu Val Lys Glu Thr Asn Ser Ala Asn Pro Leu
        115                 120                 125

gtc agt att atc atg aca tct cac aat aca gcg caa ttt atc gaa gct        432
Val Ser Ile Ile Met Thr Ser His Asn Thr Ala Gln Phe Ile Glu Ala
    130                 135                 140

tct att aat tca tta ttg tta caa aca tat aaa aac ata gaa att att        480
Ser Ile Asn Ser Leu Leu Leu Gln Thr Tyr Lys Asn Ile Glu Ile Ile
145                 150                 155                 160

att gta gat gat gat agc tcg gat aat aca ttt gaa att gcc tcg aga        528
Ile Val Asp Asp Asp Ser Ser Asp Asn Thr Phe Glu Ile Ala Ser Arg
                165                 170                 175

ata gcg aat aca aca agc aaa gtc aga gta ttt aga tta aat tca aac        576
Ile Ala Asn Thr Thr Ser Lys Val Arg Val Phe Arg Leu Asn Ser Asn
            180                 185                 190

cta gga act tac ttt gcg aaa aat aca ggc ata tta aaa tct aaa ggt        624
Leu Gly Thr Tyr Phe Ala Lys Asn Thr Gly Ile Leu Lys Ser Lys Gly
        195                 200                 205

gac att att ttc ttt caa gat agt gat gat gta tgt cat cat gaa aga        672
Asp Ile Ile Phe Phe Gln Asp Ser Asp Asp Val Cys His His Glu Arg
    210                 215                 220

ata gaa aga tgt gta aat ata tta tta gct aat aaa gaa act att gct        720
Ile Glu Arg Cys Val Asn Ile Leu Leu Ala Asn Lys Glu Thr Ile Ala
225                 230                 235                 240

gtt cgt tgt gca tac tca aga cta gca cca gaa aca cag cat atc att     768
Val Arg Cys Ala Tyr Ser Arg Leu Ala Pro Glu Thr Gln His Ile Ile
                245                 250                 255

aaa gtc aat aat atg gat tat aga tta ggt ttt ata acc ttg ggt atg     816
Lys Val Asn Asn Met Asp Tyr Arg Leu Gly Phe Ile Thr Leu Gly Met
            260                 265                 270

cac aaa aaa gta ttt caa gaa att ggt ttc ttc aat tgt acg act aaa     864
His Lys Lys Val Phe Gln Glu Ile Gly Phe Phe Asn Cys Thr Thr Lys
        275                 280                 285

ggc tca gat gat gag ttt ttt cat aga att gcg aaa tat tat gga aaa     912
Gly Ser Asp Asp Glu Phe Phe His Arg Ile Ala Lys Tyr Tyr Gly Lys
    290                 295                 300

gaa aaa ata aaa aat tta ctc ttg ccg tta tac tac aac aca atg aga     960
Glu Lys Ile Lys Asn Leu Leu Leu Pro Leu Tyr Tyr Asn Thr Met Arg
305                 310                 315                 320

gaa aac tct tta ttt act gat atg gtt gaa tgg ata gac aat cat aac     1008
Glu Asn Ser Leu Phe Thr Asp Met Val Glu Trp Ile Asp Asn His Asn
                325                 330                 335

ata ata cag aaa atg tct gat acc aga caa cat tat gca acc ctg ttt     1056
Ile Ile Gln Lys Met Ser Asp Thr Arg Gln His Tyr Ala Thr Leu Phe
            340                 345                 350

caa gcg atg cat aac gaa act gcc tca cat gat ttc aaa aat ctt ttt     1104
Gln Ala Met His Asn Glu Thr Ala Ser His Asp Phe Lys Asn Leu Phe
        355                 360                 365

caa ttc cct cgt att tac gac gcc tta cca gta cca caa gaa atg agt     1152
Gln Phe Pro Arg Ile Tyr Asp Ala Leu Pro Val Pro Gln Glu Met Ser
    370                 375                 380

aag ttg tcc aat cct aag att cct gtt tat atc aat att tgt tct att     1200
Lys Leu Ser Asn Pro Lys Ile Pro Val Tyr Ile Asn Ile Cys Ser Ile
385                 390                 395                 400

ccc tca aga ata gcg caa tta caa cgt att atc ggc ata cta aaa aat     1248
Pro Ser Arg Ile Ala Gln Leu Gln Arg Ile Ile Gly Ile Leu Lys Asn
                405                 410                 415

caa tgt gat cat ttt cat att tat ctt gat ggc tat gta gaa atc cct     1296
Gln Cys Asp His Phe His Ile Tyr Leu Asp Gly Tyr Val Glu Ile Pro
            420                 425                 430

gac ttc ata aaa aat tta ggt aat aaa gca acc gtt gtt cat tgc aaa      1344
Asp Phe Ile Lys Asn Leu Gly Asn Lys Ala Thr Val Val His Cys Lys
        435                 440                 445

gat aaa gat aac tcc att aga gat aat ggc aaa ttc att tta ctg gaa      1392
Asp Lys Asp Asn Ser Ile Arg Asp Asn Gly Lys Phe Ile Leu Leu Glu
    450                 455                 460

gag ttg att gaa aaa aat caa gat gga tat tat ata acc tgt gat gat      1440
Glu Leu Ile Glu Lys Asn Gln Asp Gly Tyr Tyr Ile Thr Cys Asp Asp
465                 470                 475                 480

gac att atc tat cca agc gat tac atc aat acg atg atc aag aag ctg      1488
Asp Ile Ile Tyr Pro Ser Asp Tyr Ile Asn Thr Met Ile Lys Lys Leu
                485                 490                 495

aat gaa tac gat gat aaa gcg gtt att ggt tta cac ggc att ctc ttt      1536
Asn Glu Tyr Asp Asp Lys Ala Val Ile Gly Leu His Gly Ile Leu Phe
            500                 505                 510

cca agt aga atg acc aaa tat ttt tcg gcg gat aga ctg gta tat agc      1584
Pro Ser Arg Met Thr Lys Tyr Phe Ser Ala Asp Arg Leu Val Tyr Ser
        515                 520                 525

ttc tat aaa cct ctg gaa aaa gac aaa gcg gtc aat gta tta ggt aca      1632
Phe Tyr Lys Pro Leu Glu Lys Asp Lys Ala Val Asn Val Leu Gly Thr
    530                 535                 540

gga act gtt agc ttt aga gtc agt ctc ttt aat caa ttt tct ctt tct      1680
Gly Thr Val Ser Phe Arg Val Ser Leu Phe Asn Gln Phe Ser Leu Ser
545                 550                 555                 560

gac ttt acc cat tca ggc atg gct gat atc tat ttc tct ctc ttg tgt      1728
Asp Phe Thr His Ser Gly Met Ala Asp Ile Tyr Phe Ser Leu Leu Cys
                565                 570                 575

aag aaa aat aat att ctt cag att tgt att tca aga cca gca aac tgg      1776
Lys Lys Asn Asn Ile Leu Gln Ile Cys Ile Ser Arg Pro Ala Asn Trp
            580                 585                 590

cta acg gaa gat aat aga gac agc gaa aca ctc tat cat caa tat cga      1824
Leu Thr Glu Asp Asn Arg Asp Ser Glu Thr Leu Tyr His Gln Tyr Arg
        595                 600                 605

gac aat gat gag caa caa act cag ctg atc atg gaa aac ggt cca tgg      1872
Asp Asn Asp Glu Gln Gln Thr Gln Leu Ile Met Glu Asn Gly Pro Trp
    610                 615                 620

gga tat tca agt att tat cca tta gtc aaa aat cat cct aaa ttt act      1920

Gly Tyr Ser Ser Ile Tyr Pro Leu Val Lys Asn His Pro Lys Phe Thr
625                 630                 635                 640

gac ctt atc ccc tgt tta cct ttt tat ttt tta                         1953
Asp Leu Ile Pro Cys Leu Pro Phe Tyr Phe Leu
                645                 650

<210>  7
<211>  651
<212>  PRT
<213>  Pasteurella multocida

<400>  7

Met Lys Gly Lys Lys Glu Met Thr Gln Ile Gln Ile Ala Lys Asn Pro
1               5                   10                  15

Pro Gln His Glu Lys Glu Asn Glu Leu Asn Thr Phe Gln Asn Lys Ile
            20                  25                  30

Asp Ser Leu Lys Thr Thr Leu Asn Lys Asp Ile Ile Ser Gln Gln Thr
        35                  40                  45

Leu Leu Ala Lys Gln Asp Ser Lys His Pro Leu Ser Ala Ser Leu Glu
    50                  55                  60

Asn Glu Asn Lys Leu Leu Leu Lys Gln Leu Gln Leu Val Leu Gln Glu
65                  70                  75                  80

Phe Lys Lys Ile Tyr Thr Tyr Asn Gln Ala Leu Glu Ala Lys Leu Glu
                85                  90                  95

Lys Asp Lys Gln Thr Thr Ser Ile Thr Asp Leu Tyr Asn Glu Val Ala
            100                 105                 110

Lys Ser Asp Leu Gly Leu Val Lys Glu Thr Asn Ser Ala Asn Pro Leu
        115                 120                 125

Val Ser Ile Ile Met Thr Ser His Asn Thr Ala Gln Phe Ile Glu Ala
    130                 135                 140

Ser Ile Asn Ser Leu Leu Leu Gln Thr Tyr Lys Asn Ile Glu Ile Ile
145                 150                 155                 160

Ile Val Asp Asp Asp Ser Ser Asp Asn Thr Phe Glu Ile Ala Ser Arg
                165                 170                 175

Ile Ala Asn Thr Thr Ser Lys Val Arg Val Phe Arg Leu Asn Ser Asn
            180                 185                 190

Leu Gly Thr Tyr Phe Ala Lys Asn Thr Gly Ile Leu Lys Ser Lys Gly
        195                 200                 205

Asp Ile Ile Phe Phe Gln Asp Ser Asp Asp Val Cys His His Glu Arg
    210                 215                 220

Ile Glu Arg Cys Val Asn Ile Leu Leu Ala Asn Lys Glu Thr Ile Ala
225                 230                 235                 240

Val Arg Cys Ala Tyr Ser Arg Leu Ala Pro Glu Thr Gln His Ile Ile
                245                 250                 255

Lys Val Asn Asn Met Asp Tyr Arg Leu Gly Phe Ile Thr Leu Gly Met
            260                 265                 270

His Lys Lys Val Phe Gln Glu Ile Gly Phe Phe Asn Cys Thr Thr Lys
        275                 280                 285

Gly Ser Asp Asp Glu Phe Phe His Arg Ile Ala Lys Tyr Tyr Gly Lys
    290                 295                 300

Glu Lys Ile Lys Asn Leu Leu Leu Pro Leu Tyr Tyr Asn Thr Met Arg
305                 310                 315                 320

Glu Asn Ser Leu Phe Thr Asp Met Val Glu Trp Ile Asp Asn His Asn
                325                 330                 335

Ile Ile Gln Lys Met Ser Asp Thr Arg Gln His Tyr Ala Thr Leu Phe
            340                 345                 350

Gln Ala Met His Asn Glu Thr Ala Ser His Asp Phe Lys Asn Leu Phe
        355                 360                 365

Gln Phe Pro Arg Ile Tyr Asp Ala Leu Pro Val Pro Gln Glu Met Ser
    370                 375                 380

Lys Leu Ser Asn Pro Lys Ile Pro Val Tyr Ile Asn Ile Cys Ser Ile
385                 390                 395                 400

Pro Ser Arg Ile Ala Gln Leu Gln Arg Ile Ile Gly Ile Leu Lys Asn
                405                 410                 415

Gln Cys Asp His Phe His Ile Tyr Leu Asp Gly Tyr Val Glu Ile Pro
            420                 425                 430

Asp Phe Ile Lys Asn Leu Gly Asn Lys Ala Thr Val Val His Cys Lys
        435                 440                 445

Asp Lys Asp Asn Ser Ile Arg Asp Asn Gly Lys Phe Ile Leu Leu Glu
    450                 455                 460

Glu Leu Ile Glu Lys Asn Gln Asp Gly Tyr Tyr Ile Thr Cys Asp Asp
465                 470                 475                 480

Asp Ile Ile Tyr Pro Ser Asp Tyr Ile Asn Thr Met Ile Lys Lys Leu
                485                 490                 495

Asn Glu Tyr Asp Asp Lys Ala Val Ile Gly Leu His Gly Ile Leu Phe
            500                 505                 510

Pro Ser Arg Met Thr Lys Tyr Phe Ser Ala Asp Arg Leu Val Tyr Ser
        515                 520                 525

Phe Tyr Lys Pro Leu Glu Lys Asp Lys Ala Val Asn Val Leu Gly Thr
    530                 535                 540

Gly Thr Val Ser Phe Arg Val Ser Leu Phe Asn Gln Phe Ser Leu Ser
545                 550                 555                 560

Asp Phe Thr His Ser Gly Met Ala Asp Ile Tyr Phe Ser Leu Leu Cys
                565                 570                 575

Lys Lys Asn Asn Ile Leu Gln Ile Cys Ile Ser Arg Pro Ala Asn Trp
            580                 585                 590

Leu Thr Glu Asp Asn Arg Asp Ser Glu Thr Leu Tyr His Gln Tyr Arg
        595                 600                 605

Asp Asn Asp Glu Gln Gln Thr Gln Leu Ile Met Glu Asn Gly Pro Trp
    610                 615                 620

Gly Tyr Ser Ser Ile Tyr Pro Leu Val Lys Asn His Pro Lys Phe Thr
625                 630                 635                 640

Asp Leu Ile Pro Cys Leu Pro Phe Tyr Phe Leu
                645                 650

<210>  8
<211>  480
<212>  DNA
<213>  Saccharomyces cerevisiae

<220>
<221>  CDS
<222>  (1)..(477)

<300>
<308>  GenBanh/AY558564
<309>  2007-04-24
<313>  (1)..(477)

<400>  8
atg agc tta ccc gat gga ttt tat ata agg cga atg gaa gag ggg gat        48

Met Ser Leu Pro Asp Gly Phe Tyr Ile Arg Arg Met Glu Glu Gly Asp
1               5                   10                  15

ttg gaa cag gtc act gag acg cta aag gtt ttg acc acc gtg ggc act      96
Leu Glu Gln Val Thr Glu Thr Leu Lys Val Leu Thr Thr Val Gly Thr
            20                  25                  30

att acc ccc gaa tcc ttc agc aaa ctc ata aaa tac tgg aat gaa gcc      144
Ile Thr Pro Glu Ser Phe Ser Lys Leu Ile Lys Tyr Trp Asn Glu Ala
        35                  40                  45

aca gta tgg aat gat aac gaa gat aaa aaa ata atg caa tat aac ccc      192
Thr Val Trp Asn Asp Asn Glu Asp Lys Lys Ile Met Gln Tyr Asn Pro
    50                  55                  60

atg gtg att gtg gac aag cgc acc gag acg gtt gcc gct acg ggg aat      240
Met Val Ile Val Asp Lys Arg Thr Glu Thr Val Ala Ala Thr Gly Asn
65                  70                  75                  80

atc atc atc gaa aga aag atc att cat gaa ctg ggg cta tgt ggc cac      288
Ile Ile Ile Glu Arg Lys Ile Ile His Glu Leu Gly Leu Cys Gly His
                85                  90                  95

atc gag gac att gca gta aac tcc aag tat cag ggc caa ggt ttg ggc      336
Ile Glu Asp Ile Ala Val Asn Ser Lys Tyr Gln Gly Gln Gly Leu Gly
            100                 105                 110

aag ctc ttg att gat caa ttg gta act atc ggc ttt gac tac ggt tgt      384
Lys Leu Leu Ile Asp Gln Leu Val Thr Ile Gly Phe Asp Tyr Gly Cys
        115                 120                 125

tat aag att att tta gat tgc gat gag aaa aat gtc aaa ttc tat gaa      432
Tyr Lys Ile Ile Leu Asp Cys Asp Glu Lys Asn Val Lys Phe Tyr Glu
    130                 135                 140

aaa tgt ggg ttt agc aac gca ggc gtg gaa atg caa att aga aaa tag      480
Lys Cys Gly Phe Ser Asn Ala Gly Val Glu Met Gln Ile Arg Lys
145                 150                 155


<210>  9
<211>  159
<212>  PRT
<213>  Saccharomyces cerevisiae

<400>  9

Met Ser Leu Pro Asp Gly Phe Tyr Ile Arg Arg Met Glu Glu Gly Asp
1               5                   10                  15

Leu Glu Gln Val Thr Glu Thr Leu Lys Val Leu Thr Thr Val Gly Thr
            20                  25                  30

Ile Thr Pro Glu Ser Phe Ser Lys Leu Ile Lys Tyr Trp Asn Glu Ala
        35                  40                  45

Thr Val Trp Asn Asp Asn Glu Asp Lys Lys Ile Met Gln Tyr Asn Pro
    50                  55                  60

Met Val Ile Val Asp Lys Arg Thr Glu Thr Val Ala Ala Thr Gly Asn
65                  70                  75                  80

Ile Ile Ile Glu Arg Lys Ile Ile His Glu Leu Gly Leu Cys Gly His
                85                  90                  95

Ile Glu Asp Ile Ala Val Asn Ser Lys Tyr Gln Gly Gln Gly Leu Gly
            100                 105                 110

Lys Leu Leu Ile Asp Gln Leu Val Thr Ile Gly Phe Asp Tyr Gly Cys
        115                 120                 125

Tyr Lys Ile Ile Leu Asp Cys Asp Glu Lys Asn Val Lys Phe Tyr Glu
    130                 135                 140

Lys Cys Gly Phe Ser Asn Ala Gly Val Glu Met Gln Ile Arg Lys
145                 150                 155

<210>  10
<211>  1434
<212>  DNA
<213>  Saccharomyces cerevisiae

<220>
<221>  CDS
<222>  (1)..(1431)

<300>

<308>  GenBank/X79380
<309>  2005-04-18
<313>  (3261)..(4694)

<400>  10
atg act gac aca aaa cag cta ttc att gaa gcc gga caa agt caa ctt        48
Met Thr Asp Thr Lys Gln Leu Phe Ile Glu Ala Gly Gln Ser Gln Leu
1               5                   10                  15

ttc cac aat tgg gaa agc ttg tct cgc aaa gac caa gaa gaa ttg ctt        96
Phe His Asn Trp Glu Ser Leu Ser Arg Lys Asp Gln Glu Glu Leu Leu
            20                  25                  30

tca aac ctg gag caa ata tct tcc aag agg tcc cct gca aaa cta ctg       144
Ser Asn Leu Glu Gln Ile Ser Ser Lys Arg Ser Pro Ala Lys Leu Leu
        35                  40                  45

gaa gac tgt caa aat gct att aaa ttc tca cta gct aac tct tct aag       192
Glu Asp Cys Gln Asn Ala Ile Lys Phe Ser Leu Ala Asn Ser Ser Lys
    50                  55                  60

gat act ggc gtc gaa att tca cca ttg ccc cct act tcg tac gag tcg       240
Asp Thr Gly Val Glu Ile Ser Pro Leu Pro Pro Thr Ser Tyr Glu Ser
65                  70                  75                  80

ctt att ggc aac agt aag aaa gaa aat gaa tac tgg cgt tta ggc ctt       288
Leu Ile Gly Asn Ser Lys Lys Glu Asn Glu Tyr Trp Arg Leu Gly Leu
                85                  90                  95

gaa gct att ggc aag ggt gaa gtc gca gtg att tta atg gct ggc gga       336
Glu Ala Ile Gly Lys Gly Glu Val Ala Val Ile Leu Met Ala Gly Gly
            100                 105                 110

caa ggt acg cgg tta gga tcc tct caa cca aag ggc tgt tac gac att       384
Gln Gly Thr Arg Leu Gly Ser Ser Gln Pro Lys Gly Cys Tyr Asp Ile
        115                 120                 125

gga ttg cct tct aag aaa tct ctt ttt caa att caa gct gaa aag ttg       432
Gly Leu Pro Ser Lys Lys Ser Leu Phe Gln Ile Gln Ala Glu Lys Leu
    130                 135                 140

atc agg ttg caa gat atg gta aag gac aaa aag gta gaa att cct tgg       480
Ile Arg Leu Gln Asp Met Val Lys Asp Lys Lys Val Glu Ile Pro Trp
145                 150                 155                 160

tat att atg aca tca ggc ccc act aga gcg gct act gag gca tac ttt       528
Tyr Ile Met Thr Ser Gly Pro Thr Arg Ala Ala Thr Glu Ala Tyr Phe
                165                 170                 175

caa gaa cac aat tat ttt ggc ttg aat aaa gaa caa att acg ttc ttc 576
Gln Glu His Asn Tyr Phe Gly Leu Asn Lys Glu Gln Ile Thr Phe Phe
            180                 185                 190

aac cag gga acc ctg cct gcc ttt gat tta acc ggg aag cat ttc cta 624
Asn Gln Gly Thr Leu Pro Ala Phe Asp Leu Thr Gly Lys His Phe Leu
        195                 200                 205

atg aaa gac cca gta aac cta tct caa tca cca gat gga aat ggt gga 672
Met Lys Asp Pro Val Asn Leu Ser Gln Ser Pro Asp Gly Asn Gly Gly
    210                 215                 220

ctc tac cgt gcc atc aag gaa aac aag ttg aac gaa gac ttt gat agg 720
Leu Tyr Arg Ala Ile Lys Glu Asn Lys Leu Asn Glu Asp Phe Asp Arg
225                 230                 235                 240

aga gga atc aag cat gtt tac atg tac tgt gtc gat aat gtc cta tct 768
Arg Gly Ile Lys His Val Tyr Met Tyr Cys Val Asp Asn Val Leu Ser
                245                 250                 255

aaa atc gca gac cct gta ttt att ggt ttt gcc atc aag cat ggc ttc 816
Lys Ile Ala Asp Pro Val Phe Ile Gly Phe Ala Ile Lys His Gly Phe
            260                 265                 270

gaa ctg gcc acc aaa gcc gtt aga aag aga gat gcg cat gaa tca gtt 864
Glu Leu Ala Thr Lys Ala Val Arg Lys Arg Asp Ala His Glu Ser Val
        275                 280                 285

ggg tta att gct act aaa aac gag aaa cca tgt gtc ata gaa tat tct 912
Gly Leu Ile Ala Thr Lys Asn Glu Lys Pro Cys Val Ile Glu Tyr Ser
    290                 295                 300

gaa att tcc aat gaa ttg gct gaa gca aag gat aaa gat ggc tta tta 960
Glu Ile Ser Asn Glu Leu Ala Glu Ala Lys Asp Lys Asp Gly Leu Leu
305                 310                 315                 320

aaa cta cgc gca ggc aac att gta aat cat tat tac cta gtg gat tta 1008
Lys Leu Arg Ala Gly Asn Ile Val Asn His Tyr Tyr Leu Val Asp Leu
                325                 330                 335

cta aaa cgt gat ttg gat cag tgg tgt gag aat atg cca tat cac att 1056
Leu Lys Arg Asp Leu Asp Gln Trp Cys Glu Asn Met Pro Tyr His Ile
            340                 345                 350

gcg aag aag aaa att cca gct tat gat agt gtt acc ggc aag tac act 1104
Ala Lys Lys Lys Ile Pro Ala Tyr Asp Ser Val Thr Gly Lys Tyr Thr
        355                 360                 365

aag cct acc gaa cca aac ggt ata aaa tta gag caa ttc ata ttt gat 1152

Lys Pro Thr Glu Pro Asn Gly Ile Lys Leu Glu Gln Phe Ile Phe Asp
    370                 375                 380

gtc ttt gac act gta cca ctg aac aag ttt ggg tgc tta gaa gta gat      1200
Val Phe Asp Thr Val Pro Leu Asn Lys Phe Gly Cys Leu Glu Val Asp
385                 390                 395                 400

aga tgc aaa gaa ttt tca cct tta aaa aac ggt cct ggt tct aag aac      1248
Arg Cys Lys Glu Phe Ser Pro Leu Lys Asn Gly Pro Gly Ser Lys Asn
                405                 410                 415

gat aat cct gag acc agc aga cta gca tat ttg aaa cta gga acc tcg      1296
Asp Asn Pro Glu Thr Ser Arg Leu Ala Tyr Leu Lys Leu Gly Thr Ser
            420                 425                 430

tgg ttg gaa gat gca ggc gct att gta aaa gat ggg gta cta gtc gaa      1344
Trp Leu Glu Asp Ala Gly Ala Ile Val Lys Asp Gly Val Leu Val Glu
        435                 440                 445

gtt tcc agc aaa ttg agt tat gca ggt gaa aat cta tcc cag ttc aaa      1392
Val Ser Ser Lys Leu Ser Tyr Ala Gly Glu Asn Leu Ser Gln Phe Lys
    450                 455                 460

ggt aaa gtc ttt gac aga agt ggt ata gta tta gaa aaa taa              1434
Gly Lys Val Phe Asp Arg Ser Gly Ile Val Leu Glu Lys
465                 470                 475

<210>  11
<211>  477
<212>  PRT
<213>  Saccharomyces cerevisiae

<400>  11

Met Thr Asp Thr Lys Gln Leu Phe Ile Glu Ala Gly Gln Ser Gln Leu
1               5                   10                  15

Phe His Asn Trp Glu Ser Leu Ser Arg Lys Asp Gln Glu Glu Leu Leu
            20                  25                  30

Ser Asn Leu Glu Gln Ile Ser Ser Lys Arg Ser Pro Ala Lys Leu Leu
        35                  40                  45

Glu Asp Cys Gln Asn Ala Ile Lys Phe Ser Leu Ala Asn Ser Ser Lys
    50                  55                  60

Asp Thr Gly Val Glu Ile Ser Pro Leu Pro Pro Thr Ser Tyr Glu Ser
65                  70                  75                  80

Leu Ile Gly Asn Ser Lys Lys Glu Asn Glu Tyr Trp Arg Leu Gly Leu
                85                  90                  95

Glu Ala Ile Gly Lys Gly Glu Val Ala Val Ile Leu Met Ala Gly Gly
            100                 105                 110

Gln Gly Thr Arg Leu Gly Ser Ser Gln Pro Lys Gly Cys Tyr Asp Ile
        115                 120                 125

Gly Leu Pro Ser Lys Lys Ser Leu Phe Gln Ile Gln Ala Glu Lys Leu
    130                 135                 140

Ile Arg Leu Gln Asp Met Val Lys Asp Lys Lys Val Glu Ile Pro Trp
145                 150                 155                 160

Tyr Ile Met Thr Ser Gly Pro Thr Arg Ala Ala Thr Glu Ala Tyr Phe
                165                 170                 175

Gln Glu His Asn Tyr Phe Gly Leu Asn Lys Glu Gln Ile Thr Phe Phe
            180                 185                 190

Asn Gln Gly Thr Leu Pro Ala Phe Asp Leu Thr Gly Lys His Phe Leu
        195                 200                 205

Met Lys Asp Pro Val Asn Leu Ser Gln Ser Pro Asp Gly Asn Gly Gly
    210                 215                 220

Leu Tyr Arg Ala Ile Lys Glu Asn Lys Leu Asn Glu Asp Phe Asp Arg
225                 230                 235                 240

Arg Gly Ile Lys His Val Tyr Met Tyr Cys Val Asp Asn Val Leu Ser
                245                 250                 255

Lys Ile Ala Asp Pro Val Phe Ile Gly Phe Ala Ile Lys His Gly Phe
            260                 265                 270

Glu Leu Ala Thr Lys Ala Val Arg Lys Arg Asp Ala His Glu Ser Val
        275                 280                 285

Gly Leu Ile Ala Thr Lys Asn Glu Lys Pro Cys Val Ile Glu Tyr Ser
    290                 295                 300

Glu Ile Ser Asn Glu Leu Ala Glu Ala Lys Asp Lys Asp Gly Leu Leu
305                 310                 315                 320

Lys Leu Arg Ala Gly Asn Ile Val Asn His Tyr Tyr Leu Val Asp Leu
                325                 330                 335

Leu Lys Arg Asp Leu Asp Gln Trp Cys Glu Asn Met Pro Tyr His Ile
            340                 345                 350

Ala Lys Lys Lys Ile Pro Ala Tyr Asp Ser Val Thr Gly Lys Tyr Thr
        355                 360                 365

Lys Pro Thr Glu Pro Asn Gly Ile Lys Leu Glu Gln Phe Ile Phe Asp
    370                 375                 380

Val Phe Asp Thr Val Pro Leu Asn Lys Phe Gly Cys Leu Glu Val Asp
385                 390                 395                 400

Arg Cys Lys Glu Phe Ser Pro Leu Lys Asn Gly Pro Gly Ser Lys Asn
                405                 410                 415

Asp Asn Pro Glu Thr Ser Arg Leu Ala Tyr Leu Lys Leu Gly Thr Ser
            420                 425                 430

Trp Leu Glu Asp Ala Gly Ala Ile Val Lys Asp Gly Val Leu Val Glu
        435                 440                 445

Val Ser Ser Lys Leu Ser Tyr Ala Gly Glu Asn Leu Ser Gln Phe Lys
    450                 455                 460

Gly Lys Val Phe Asp Arg Ser Gly Ile Val Leu Glu Lys
465                 470                 475

<210>  12
<211>  919
<212>  DNA
<213>  Artificial

<220>
<223>  Recombinant nucleic acid sequence comprising sequnces for an YLCV
       promoter, a cloning site, an ocs-termination signal and a
       nos-termination signal.

<220>
<221>  promoter
<222>  (8)..(352)
<223>  Sequence of the YLCV promoter

<220>
<221>  polyA_signal
<222>  (366)..(663)
<223>  polyA-signal of the ocs gene from Agrobacterium tumefaciens

<220>
<221>  polyA_signal
<222>  (664)..(872)
<223>  polyA-signal of the nos gene from Agrobacterium tumefaciens

<400>  12
gaattcctgg cagacaaagt ggcagacata ctgtcccaca aatgaagatg gaatctgtaa      60

aagaaaacgc gtgaaataat gcgtctgaca aaggttaggt cggctgcctt taatcaatac     120

caaagtggtc cctaccacga tggaaaaact gtgcagtcgg tttggctttt tctgacgaac     180

aaataagatt cgtggccgac aggtgggggt ccaccatgtg aaggcatctt cagactccaa     240

taatggagca atgacgtaag ggcttacgaa ataagtaagg gtagtttggg aaatgtccac     300

tcacccgtca gtctataaat acttagcccc tccctcattg ttaagggagc aagagctcgc     360

ccgggatctc gaatcacgcg ttctaggatc cgaagcagat cgttcaaaca tttggcaata     420

aagtttctta agattgaatc ctgttgccgg tcttgcgatg attatcatat aatttctgtt    480

gaattacgtt aagcatgtaa taattaacat gtaatgcatg acgttattta tgagatgggt    540

ttttatgatt agagtcccgc aattatacat ttaatacgcg atagaaaaca aaatatagcg    600

cgcaaactag gataaattat cgcgcgcggt gtcatctatg ttactagatc gggaagatct    660

cgagacgtcg ggacaatcag taaattgaac ggagaatatt attcataaaa atacgatagt    720

aacgggtgat atattcatta gaatgaaccg aaaccggcgg taaggatctg agctacacat    780

gctcaggttt tttacaacgt gcacaacaga attgaaagca aatatcatgc gatcataggc    840

gtctcgcata tctcattaaa gcagggcatg cctgtttaaa cattaattaa acctaggtga    900

cgtctaaaag ggcgaattc                                                  919


<210>  13
<211>  48
<212>  DNA
<213>  Artificial

<220>
<223>  Synthetic oligonucleotide

<400>  13
tcgacaggcc tggatcctta attaaactag tctcgaggag ctcggtac                  48


<210>  14
<211>  40
<212>  DNA
<213>  Artificial

<220>
<223>  Synthetic oligonusleotide

<400>  14
cgagctcctc gagactagtt taattaagga tccaggcctg                          40


<210>  15
<211>  29
<212>  DNA
<213>  Artificial

<220>

<223>  Synthetic oligonucleotide

<400>  15
cttaattaat agttgacgaa cggaagctg                                          29


<210>  16
<211>  25
<212>  DNA
<213>  Artificial

<220>
<223>  Synthetic oligonucleotide

<400>  16
agcatgcttg cagaccgtca ttagg                                              25


<210>  17
<211>  26
<212>  DNA
<213>  Artificial

<220>
<223>  Synthetic oligonucleotide

<400>  17
aaagtgcttc ataagtagct caaaca                                             26


<210>  18
<211>  20
<212>  DNA
<213>  Artificial

<220>
<223>  Synthetic oligonucleotide

<400>  18
aacaccagat cgaactgcaa                                                    20


<210>  19
<211>  21
<212>  DNA
<213>  Artificial

<220>
<223>  Synthetic oligonucleotide

<400>  19
ccgccttttt agccagttat c                                                21

<210>  20
<211>  20
<212>  DNA
<213>  Artificial

<220>
<223>  Synthetic oligonucleotide

<400>  20
ccactctgtc tgcaaaggaa                                                  20

<210>  21
<211>  1338
<212>  DNA
<213>  Escherichia coli

<220>
<221>  CDS
<222>  (1)..(1338)

<300>
<308>  GenBank / AY616604
<309>  2006-08-18
<313>  (1)..(1338)

<400>  21
atg agt aat cgt aaa tat ttc ggt acc gat ggg att cgt ggt cgt gta        48
Met Ser Asn Arg Lys Tyr Phe Gly Thr Asp Gly Ile Arg Gly Arg Val
1               5                   10                  15

ggg gat gcg ccg atc aca cct gat ttt gtg ctt aag ctg ggt tgg gcc        96
Gly Asp Ala Pro Ile Thr Pro Asp Phe Val Leu Lys Leu Gly Trp Ala
            20                  25                  30

gcg ggt aaa gtg ctg gcg cgc cac ggc tcc cgt aag att att att ggt       144
Ala Gly Lys Val Leu Ala Arg His Gly Ser Arg Lys Ile Ile Ile Gly
        35                  40                  45

aaa gac acg cgt att tct ggc tat atg ctg gag tca gca ctg gaa gcg       192
Lys Asp Thr Arg Ile Ser Gly Tyr Met Leu Glu Ser Ala Leu Glu Ala
    50                  55                  60

ggt ctg gcg gca gcg ggc ctt tcc gca ctc ttc act ggc ccg atg cca 240
Gly Leu Ala Ala Ala Gly Leu Ser Ala Leu Phe Thr Gly Pro Met Pro
65                  70                  75                  80

aca ccg gcc gtg gct tat ctg acg cgt acc ttc cgc gca gag gcc gga 288
Thr Pro Ala Val Ala Tyr Leu Thr Arg Thr Phe Arg Ala Glu Ala Gly
                85                  90                  95

att gtg ata tct gca tcg cat aac ccg ttc tac gat aat ggc att aaa 336
Ile Val Ile Ser Ala Ser His Asn Pro Phe Tyr Asp Asn Gly Ile Lys
            100                 105                 110

ttc ttc tct atc gac ggc acc aaa ctg ccg gat gcg gta gaa gag gcc 384
Phe Phe Ser Ile Asp Gly Thr Lys Leu Pro Asp Ala Val Glu Glu Ala
        115                 120                 125

atc gaa gcg gaa atg gaa aag gag atc agc tgc gtt gat tcg gca gaa 432
Ile Glu Ala Glu Met Glu Lys Glu Ile Ser Cys Val Asp Ser Ala Glu
    130                 135                 140

ctg ggt aaa gcc agc cgt atc gtt gat gcc gcg ggt cgc tat atc gag 480
Leu Gly Lys Ala Ser Arg Ile Val Asp Ala Ala Gly Arg Tyr Ile Glu
145                 150                 155                 160

ttt tgc aaa gcc acg ttc ccg aac gaa ctt agc ctc agt gaa ctg aag 528
Phe Cys Lys Ala Thr Phe Pro Asn Glu Leu Ser Leu Ser Glu Leu Lys
                165                 170                 175

att gtg gtg gat tgt gca aac ggt gcg act tat cac atc gcg ccg aac 576
Ile Val Val Asp Cys Ala Asn Gly Ala Thr Tyr His Ile Ala Pro Asn
            180                 185                 190

gtg ctg cgc gaa ctg ggg gcg aac gtt atc gct atc ggt tgt gag cca 624
Val Leu Arg Glu Leu Gly Ala Asn Val Ile Ala Ile Gly Cys Glu Pro
        195                 200                 205

aac ggt gta aac atc aat gcc gaa gtg ggg gct acc gac gtt cgc gcg 672
Asn Gly Val Asn Ile Asn Ala Glu Val Gly Ala Thr Asp Val Arg Ala
    210                 215                 220

ctc cag gct cgt gtg ctg gct gaa aaa gcg gat ctc ggt att gcc ttc 720
Leu Gln Ala Arg Val Leu Ala Glu Lys Ala Asp Leu Gly Ile Ala Phe
225                 230                 235                 240

gac ggc gat ggc gat cgc gtg att atg gtt gac cat gaa ggc aat aaa 768
Asp Gly Asp Gly Asp Arg Val Ile Met Val Asp His Glu Gly Asn Lys
                245                 250                 255

gtc gat ggc gat cag atc atg tat atc atc gcg cgt gaa ggt ctt cgt 816

Val Asp Gly Asp Gln Ile Met Tyr Ile Ile Ala Arg Glu Gly Leu Arg
            260                 265                 270

cag ggc cag ctg cgt ggt ggc gct gtg ggt aca ttg atg agc aac atg      864
Gln Gly Gln Leu Arg Gly Gly Ala Val Gly Thr Leu Met Ser Asn Met
        275                 280                 285

ggg ctt gaa ctg gcg ctg aaa cag tta gga att cca ttt gcg cgc gcg      912
Gly Leu Glu Leu Ala Leu Lys Gln Leu Gly Ile Pro Phe Ala Arg Ala
    290                 295                 300

aaa gtg ggt gac cgc tac gta ctg gaa aaa atg cag gag aaa ggc tgg      960
Lys Val Gly Asp Arg Tyr Val Leu Glu Lys Met Gln Glu Lys Gly Trp
305                 310                 315                 320

cgt atc ggt gca gag aat tcc ggt cat gtg atc ctg ctg gat aaa act     1008
Arg Ile Gly Ala Glu Asn Ser Gly His Val Ile Leu Leu Asp Lys Thr
                325                 330                 335

act acc ggt gac ggc atc gtt gct ggc ttg cag gtg ctg gcg gcg atg     1056
Thr Thr Gly Asp Gly Ile Val Ala Gly Leu Gln Val Leu Ala Ala Met
            340                 345                 350

gca cgt aac cat atg agc ctg cac gac ctt tgc agc ggc atg aaa atg     1104
Ala Arg Asn His Met Ser Leu His Asp Leu Cys Ser Gly Met Lys Met
        355                 360                 365

ttc ccg cag att ctg gtt aac gta cgt tac acc gca ggt agc ggc gat     1152
Phe Pro Gln Ile Leu Val Asn Val Arg Tyr Thr Ala Gly Ser Gly Asp
    370                 375                 380

cca ctt gag cat gag tca gtt aaa gcc gtg acc gca gag gtt gaa gct     1200
Pro Leu Glu His Glu Ser Val Lys Ala Val Thr Ala Glu Val Glu Ala
385                 390                 395                 400

gcg ctg ggc aac cgt gga cgc gtg ttg ctg cgt aaa tcc ggc acc gaa     1248
Ala Leu Gly Asn Arg Gly Arg Val Leu Leu Arg Lys Ser Gly Thr Glu
                405                 410                 415

ccg tta att cgc gtg atg gtg gaa ggc gaa gac gaa gcg cag gtg act     1296
Pro Leu Ile Arg Val Met Val Glu Gly Glu Asp Glu Ala Gln Val Thr
            420                 425                 430

gaa ttt gca cac cgc atc gcc gat gca gta aaa gcc gtt taa             1338
Glu Phe Ala His Arg Ile Ala Asp Ala Val Lys Ala Val
        435                 440                 445

<210>  22

<211>  445
<212>  PRT
<213>  Escherichia coli

<400>  22

Met Ser Asn Arg Lys Tyr Phe Gly Thr Asp Gly Ile Arg Gly Arg Val
1               5                   10                  15

Gly Asp Ala Pro Ile Thr Pro Asp Phe Val Leu Lys Leu Gly Trp Ala
            20                  25                  30

Ala Gly Lys Val Leu Ala Arg His Gly Ser Arg Lys Ile Ile Ile Gly
        35                  40                  45

Lys Asp Thr Arg Ile Ser Gly Tyr Met Leu Glu Ser Ala Leu Glu Ala
    50                  55                  60

Gly Leu Ala Ala Ala Gly Leu Ser Ala Leu Phe Thr Gly Pro Met Pro
65                  70                  75                  80

Thr Pro Ala Val Ala Tyr Leu Thr Arg Thr Phe Arg Ala Glu Ala Gly
                85                  90                  95

Ile Val Ile Ser Ala Ser His Asn Pro Phe Tyr Asp Asn Gly Ile Lys
            100                 105                 110

Phe Phe Ser Ile Asp Gly Thr Lys Leu Pro Asp Ala Val Glu Glu Ala
        115                 120                 125

Ile Glu Ala Glu Met Glu Lys Glu Ile Ser Cys Val Asp Ser Ala Glu
    130                 135                 140

Leu Gly Lys Ala Ser Arg Ile Val Asp Ala Ala Gly Arg Tyr Ile Glu
145                 150                 155                 160

Phe Cys Lys Ala Thr Phe Pro Asn Glu Leu Ser Leu Ser Glu Leu Lys
                165                 170                 175

Ile Val Val Asp Cys Ala Asn Gly Ala Thr Tyr His Ile Ala Pro Asn
            180                 185                 190

Val Leu Arg Glu Leu Gly Ala Asn Val Ile Ala Ile Gly Cys Glu Pro
        195                 200                 205

Asn Gly Val Asn Ile Asn Ala Glu Val Gly Ala Thr Asp Val Arg Ala
    210                 215                 220

Leu Gln Ala Arg Val Leu Ala Glu Lys Ala Asp Leu Gly Ile Ala Phe
225                 230                 235                 240

Asp Gly Asp Gly Asp Arg Val Ile Met Val Asp His Glu Gly Asn Lys
                245                 250                 255

Val Asp Gly Asp Gln Ile Met Tyr Ile Ile Ala Arg Glu Gly Leu Arg
            260                 265                 270

Gln Gly Gln Leu Arg Gly Gly Ala Val Gly Thr Leu Met Ser Asn Met
        275                 280                 285

Gly Leu Glu Leu Ala Leu Lys Gln Leu Gly Ile Pro Phe Ala Arg Ala
    290                 295                 300

Lys Val Gly Asp Arg Tyr Val Leu Glu Lys Met Gln Glu Lys Gly Trp
305                 310                 315                 320

Arg Ile Gly Ala Glu Asn Ser Gly His Val Ile Leu Leu Asp Lys Thr
                325                 330                 335

Thr Thr Gly Asp Gly Ile Val Ala Gly Leu Gln Val Leu Ala Ala Met
            340                 345                 350

Ala Arg Asn His Met Ser Leu His Asp Leu Cys Ser Gly Met Lys Met
        355                 360                 365

Phe Pro Gln Ile Leu Val Asn Val Arg Tyr Thr Ala Gly Ser Gly Asp
    370                 375                 380

Pro Leu Glu His Glu Ser Val Lys Ala Val Thr Ala Glu Val Glu Ala
385                 390                 395                 400

Ala Leu Gly Asn Arg Gly Arg Val Leu Leu Arg Lys Ser Gly Thr Glu
                405                 410                 415

Pro Leu Ile Arg Val Met Val Glu Gly Glu Asp Glu Ala Gln Val Thr
            420                 425                 430

Glu Phe Ala His Arg Ile Ala Asp Ala Val Lys Ala Val
        435                 440                 445

<210>  23
<211>  20
<212>  DNA
<213>  Artificial

<220>
<223>  Synthetic oligonucleotide

<400>  23
attacccggc cagaatcact                                                   20

<210>  24
<211>  20
<212>  DNA
<213>  Artificial

<220>
<223>  Synthetic oligonucleotide

<400>  24
gtcaggacgc gtatgttgaa                                                   20

<210>  25
<211>  1371
<212>  DNA
<213>  Escherichia coli

<220>
<221>  CDS
<222>  (1)..(1368)

<300>
<308>  GenBank / AP009048.1
<309>  2007-05-19
<313>  (3721481)..(3722851)

<400>  25
atg ttg aat aat gct atg agc gta gtg atc ctt gcc gca ggc aaa ggc       48
Met Leu Asn Asn Ala Met Ser Val Val Ile Leu Ala Ala Gly Lys Gly
1               5                   10                  15

acg cgc atg tat tcc gat ctt ccg aaa gtg ctg cat acc ctt gcc ggg       96
Thr Arg Met Tyr Ser Asp Leu Pro Lys Val Leu His Thr Leu Ala Gly
            20                  25                  30

aaa gcg atg gtt cag cat gtc att gat gct gcg aat gaa tta ggc gca      144
Lys Ala Met Val Gln His Val Ile Asp Ala Ala Asn Glu Leu Gly Ala
        35                  40                  45

gcg cac gtt cac ctg gtg tac ggt cac ggc ggc gat ctg cta aaa cag      192
Ala His Val His Leu Val Tyr Gly His Gly Gly Asp Leu Leu Lys Gln
    50                  55                  60

gcg ctg aaa gac gac aac ctt aac tgg gtg ctt cag gca gag cag ctg      240
Ala Leu Lys Asp Asp Asn Leu Asn Trp Val Leu Gln Ala Glu Gln Leu
65                  70                  75                  80

ggt acg ggt cat gca atg cag cag gcc gca cct ttc ttt gcc gat gat      288
Gly Thr Gly His Ala Met Gln Gln Ala Ala Pro Phe Phe Ala Asp Asp
                85                  90                  95

gaa gac att tta atg ctc tac ggc gac gtg ccg ctg atc tct gtc gaa      336
Glu Asp Ile Leu Met Leu Tyr Gly Asp Val Pro Leu Ile Ser Val Glu
            100                 105                 110

aca ctc cag cgt ctg cgt gat gct aaa ccg cag ggt ggc att ggt ctg      384
Thr Leu Gln Arg Leu Arg Asp Ala Lys Pro Gln Gly Gly Ile Gly Leu
        115                 120                 125

ctg acg gtg aaa ctg gat gat ccg acc ggt tat gga cgt atc acc cgt      432
Leu Thr Val Lys Leu Asp Asp Pro Thr Gly Tyr Gly Arg Ile Thr Arg
    130                 135                 140

gaa aac ggc aaa gtt acc ggc att gtt gag cac aaa gat gcc acc ggc      480
Glu Asn Gly Lys Val Thr Gly Ile Val Glu His Lys Asp Ala Thr Gly

145                 150                 155                 160

gag cag cgt cag att cag gag atc aac acc ggc att ctg att gcc aac      528
Glu Gln Arg Gln Ile Gln Glu Ile Asn Thr Gly Ile Leu Ile Ala Asn
                165                 170                 175

ggc gca gat atg aaa cgc tgg ctg gcg aag ctg acc aac aat aat gct      576
Gly Ala Asp Met Lys Arg Trp Leu Ala Lys Leu Thr Asn Asn Asn Ala
            180                 185                 190

cag ggc gaa tac tac atc acc gac att att gcg ctg gcg tat cag gaa      624
Gln Gly Glu Tyr Tyr Ile Thr Asp Ile Ile Ala Leu Ala Tyr Gln Glu
        195                 200                 205

ggg cgt gaa atc gtc gcc gtt cat ccg caa cgt tta agc gaa gta gaa      672
Gly Arg Glu Ile Val Ala Val His Pro Gln Arg Leu Ser Glu Val Glu
    210                 215                 220

ggc gtg aat aac cgc ctg caa ctc tcc cgt ctg gag cgt gtt tat cag      720
Gly Val Asn Asn Arg Leu Gln Leu Ser Arg Leu Glu Arg Val Tyr Gln
225                 230                 235                 240

tcc gaa cag gct gaa aaa ctg ctg tta gca ggc gtt atg ctg cgc gat      768
Ser Glu Gln Ala Glu Lys Leu Leu Leu Ala Gly Val Met Leu Arg Asp
                245                 250                 255

cca gcg cgt ttt gat ctg cgt ggt acg cta act cac ggg cgc gat gtt      816
Pro Ala Arg Phe Asp Leu Arg Gly Thr Leu Thr His Gly Arg Asp Val
            260                 265                 270

gaa att gat act aac gtt atc atc gag ggc aac gtg act ctc ggt cat      864
Glu Ile Asp Thr Asn Val Ile Ile Glu Gly Asn Val Thr Leu Gly His
        275                 280                 285

cgc gtg aaa att ggc acc ggt tgc gtg att aaa aac agc gtg att ggc      912
Arg Val Lys Ile Gly Thr Gly Cys Val Ile Lys Asn Ser Val Ile Gly
    290                 295                 300

gat gat tgc gaa atc agt ccg tat acc gtt gtg gaa gat gcg aat ctg      960
Asp Asp Cys Glu Ile Ser Pro Tyr Thr Val Val Glu Asp Ala Asn Leu
305                 310                 315                 320

gca gcg gcc tgt acc att ggc ccg ttt gcc cgt ttg cgt cct ggt gct     1008
Ala Ala Ala Cys Thr Ile Gly Pro Phe Ala Arg Leu Arg Pro Gly Ala
                325                 330                 335

gag ttg ctg gaa ggt gct cac gtc ggt aac ttc gtt gag atg aaa aaa     1056
Glu Leu Leu Glu Gly Ala His Val Gly Asn Phe Val Glu Met Lys Lys
            340                 345                 350

gcg cgt ctg ggt aaa ggc tcg aaa gct ggt cat ctg act tac ctg ggc      1104
Ala Arg Leu Gly Lys Gly Ser Lys Ala Gly His Leu Thr Tyr Leu Gly
        355                 360                 365

gat gcg gaa att ggc gat aac gtt aac atc ggc gcg gga acc att acc      1152
Asp Ala Glu Ile Gly Asp Asn Val Asn Ile Gly Ala Gly Thr Ile Thr
    370                 375                 380

tgc aac tac gat ggt gcg aat aaa ttt aag acc att atc ggc gac gat      1200
Cys Asn Tyr Asp Gly Ala Asn Lys Phe Lys Thr Ile Ile Gly Asp Asp
385                 390                 395                 400

gtg ttt gtt ggt tcc gac act cag ctg gtg gcc ccg gta aca gta ggc      1248
Val Phe Val Gly Ser Asp Thr Gln Leu Val Ala Pro Val Thr Val Gly
                405                 410                 415

aaa ggc gcg acc att gct gcg ggt aca act gtg acg cgt aat gtc ggc      1296
Lys Gly Ala Thr Ile Ala Ala Gly Thr Thr Val Thr Arg Asn Val Gly
            420                 425                 430

gaa aat gca tta gct atc agc cgt gtg ccg cag act cag aaa gaa ggc      1344
Glu Asn Ala Leu Ala Ile Ser Arg Val Pro Gln Thr Gln Lys Glu Gly
        435                 440                 445

tgg cgt cgt ccg gta aag aaa aag tga                                  1371
Trp Arg Arg Pro Val Lys Lys Lys
    450                 455

<210>  26
<211>  456
<212>  PRT
<213>  Escherichia coli

<400>  26

Met Leu Asn Asn Ala Met Ser Val Val Ile Leu Ala Ala Gly Lys Gly
1               5                   10                  15

Thr Arg Met Tyr Ser Asp Leu Pro Lys Val Leu His Thr Leu Ala Gly
            20                  25                  30

Lys Ala Met Val Gln His Val Ile Asp Ala Ala Asn Glu Leu Gly Ala
        35                  40                  45

Ala His Val His Leu Val Tyr Gly His Gly Gly Asp Leu Leu Lys Gln
    50                  55                  60

Ala Leu Lys Asp Asp Asn Leu Asn Trp Val Leu Gln Ala Glu Gln Leu
65                  70                  75                  80

Gly Thr Gly His Ala Met Gln Gln Ala Ala Pro Phe Phe Ala Asp Asp
                85                  90                  95

Glu Asp Ile Leu Met Leu Tyr Gly Asp Val Pro Leu Ile Ser Val Glu
            100                 105                 110

Thr Leu Gln Arg Leu Arg Asp Ala Lys Pro Gln Gly Gly Ile Gly Leu
        115                 120                 125

Leu Thr Val Lys Leu Asp Asp Pro Thr Gly Tyr Gly Arg Ile Thr Arg
    130                 135                 140

Glu Asn Gly Lys Val Thr Gly Ile Val Glu His Lys Asp Ala Thr Gly
145                 150                 155                 160

Glu Gln Arg Gln Ile Gln Glu Ile Asn Thr Gly Ile Leu Ile Ala Asn
                165                 170                 175

Gly Ala Asp Met Lys Arg Trp Leu Ala Lys Leu Thr Asn Asn Asn Ala
            180                 185                 190

Gln Gly Glu Tyr Tyr Ile Thr Asp Ile Ile Ala Leu Ala Tyr Gln Glu
        195                 200                 205

Gly Arg Glu Ile Val Ala Val His Pro Gln Arg Leu Ser Glu Val Glu
    210                 215                 220

Gly Val Asn Asn Arg Leu Gln Leu Ser Arg Leu Glu Arg Val Tyr Gln
225                 230                 235                 240

Ser Glu Gln Ala Glu Lys Leu Leu Leu Ala Gly Val Met Leu Arg Asp

245                 250                 255

Pro Ala Arg Phe Asp Leu Arg Gly Thr Leu Thr His Gly Arg Asp Val
            260                 265                 270

Glu Ile Asp Thr Asn Val Ile Ile Glu Gly Asn Val Thr Leu Gly His
        275                 280                 285

Arg Val Lys Ile Gly Thr Gly Cys Val Ile Lys Asn Ser Val Ile Gly
    290                 295                 300

Asp Asp Cys Glu Ile Ser Pro Tyr Thr Val Val Glu Asp Ala Asn Leu
305                 310                 315                 320

Ala Ala Ala Cys Thr Ile Gly Pro Phe Ala Arg Leu Arg Pro Gly Ala
                325                 330                 335

Glu Leu Leu Glu Gly Ala His Val Gly Asn Phe Val Glu Met Lys Lys
            340                 345                 350

Ala Arg Leu Gly Lys Gly Ser Lys Ala Gly His Leu Thr Tyr Leu Gly
        355                 360                 365

Asp Ala Glu Ile Gly Asp Asn Val Asn Ile Gly Ala Gly Thr Ile Thr
    370                 375                 380

Cys Asn Tyr Asp Gly Ala Asn Lys Phe Lys Thr Ile Ile Gly Asp Asp
385                 390                 395                 400

Val Phe Val Gly Ser Asp Thr Gln Leu Val Ala Pro Val Thr Val Gly
                405                 410                 415

Lys Gly Ala Thr Ile Ala Ala Gly Thr Thr Val Thr Arg Asn Val Gly
            420                 425                 430

Glu Asn Ala Leu Ala Ile Ser Arg Val Pro Gln Thr Gln Lys Glu Gly
        435                 440                 445

Trp Arg Arg Pro Val Lys Lys Lys
    450                 455

<210>  27
<211>  20
<212>  DNA
<213>  Artificial

<220>
<223>  Synthetic oligonucleotide

<400>  27
ggcaaaggtc agcagtaagc                                                    20

<210>  28
<211>  21
<212>  DNA
<213>  Artificial

<220>
<223>  Synthetic oligonucleotide

<400>  28
tcaagcagat gccttaacgt g                                                  21

<210>  29
<211>  1674
<212>  DNA
<213>  Saccharomyces cerevisiae

<220>
<221>  CDS
<222>  (1)..(1671)

<400>  29
atg aag gtt gat tac gag caa ttg tgc aaa ctc tac gat gac acg tgc       48
Met Lys Val Asp Tyr Glu Gln Leu Cys Lys Leu Tyr Asp Asp Thr Cys
1               5                   10                  15

cgc aca aag aat gtg cag ttc agt tac ggt acg gcc gga ttc aga acg       96
Arg Thr Lys Asn Val Gln Phe Ser Tyr Gly Thr Ala Gly Phe Arg Thr
            20                  25                  30

ctg gcc aag aat ttg gat acg gtg atg ttc agt act ggt ata ctg gcg 144
Leu Ala Lys Asn Leu Asp Thr Val Met Phe Ser Thr Gly Ile Leu Ala
        35                  40                  45

gtt ctc agg tcg ctg aag ctt cag ggt cag tat gtg ggg gtg atg atc 192
Val Leu Arg Ser Leu Lys Leu Gln Gly Gln Tyr Val Gly Val Met Ile
    50                  55                  60

acg gcg tcg cac aac cca tac cag gac aac ggg gtc aag atc gtg gaa 240
Thr Ala Ser His Asn Pro Tyr Gln Asp Asn Gly Val Lys Ile Val Glu
65                  70                  75                  80

cca gac gga tcg atg ctt ttg gcc aca tgg gag cca tat gcc atg cag 288
Pro Asp Gly Ser Met Leu Leu Ala Thr Trp Glu Pro Tyr Ala Met Gln
                85                  90                  95

ttg gcc aat gcg gcc tct ttt gcc act aat ttt gaa gaa ttt cgt gtt 336
Leu Ala Asn Ala Ala Ser Phe Ala Thr Asn Phe Glu Glu Phe Arg Val
            100                 105                 110

gag ttg gcc aag ctg att gaa cac gaa aag att gat ttg aat aca acc 384
Glu Leu Ala Lys Leu Ile Glu His Glu Lys Ile Asp Leu Asn Thr Thr
        115                 120                 125

gtc gtg cct cac atc gtg gtt ggg aga gac tct agg gaa agt agt cca 432
Val Val Pro His Ile Val Val Gly Arg Asp Ser Arg Glu Ser Ser Pro
    130                 135                 140

tac ttg ctg cgc tgc ttg act tcc tcc atg gcc agc gtc ttc cac gcg 480
Tyr Leu Leu Arg Cys Leu Thr Ser Ser Met Ala Ser Val Phe His Ala
145                 150                 155                 160

caa gtt ttg gac cta ggc tgt gtc act acg cct caa ttg cat tac att 528
Gln Val Leu Asp Leu Gly Cys Val Thr Thr Pro Gln Leu His Tyr Ile
                165                 170                 175

act gat ttg tcc aac agg cgg aaa ctg gaa gga gac aca gcg cca gtt 576
Thr Asp Leu Ser Asn Arg Arg Lys Leu Glu Gly Asp Thr Ala Pro Val
            180                 185                 190

gcc aca gaa cag gac tac tat tcg ttc ttt ata gga gcc ttc aac gag 624
Ala Thr Glu Gln Asp Tyr Tyr Ser Phe Phe Ile Gly Ala Phe Asn Glu
        195                 200                 205

ctc ttc gcc acg tat cag ctg gag aag agg ctg tct gtc cca aaa ttg 672
Leu Phe Ala Thr Tyr Gln Leu Glu Lys Arg Leu Ser Val Pro Lys Leu
    210                 215                 220

ttc ata gac aca gcc aat ggt atc ggt ggt cca cag ttg aaa aaa cta 720

Phe Ile Asp Thr Ala Asn Gly Ile Gly Gly Pro Gln Leu Lys Lys Leu
225                 230                 235                 240

ctg gcc tcc gaa gat tgg gac gtg cca gcg gag caa gtt gag gta atc      768
Leu Ala Ser Glu Asp Trp Asp Val Pro Ala Glu Gln Val Glu Val Ile
                245                 250                 255

aac gac agg tcc gat gtt cca gaa ctg ttg aat ttt gaa tgc ggt gcg      816
Asn Asp Arg Ser Asp Val Pro Glu Leu Leu Asn Phe Glu Cys Gly Ala
            260                 265                 270

gat tat gtg aag act aac cag aga tta ccc aag ggt ctt tct cca tcc      864
Asp Tyr Val Lys Thr Asn Gln Arg Leu Pro Lys Gly Leu Ser Pro Ser
        275                 280                 285

tcg ttt gat tcg cta tat tgc tcc ttt gat ggt gac gca gac agg gtt      912
Ser Phe Asp Ser Leu Tyr Cys Ser Phe Asp Gly Asp Ala Asp Arg Val
    290                 295                 300

gtg ttc tac tat gtc gac tca gga tca aaa ttt cat ttg ttg gat ggt      960
Val Phe Tyr Tyr Val Asp Ser Gly Ser Lys Phe His Leu Leu Asp Gly
305                 310                 315                 320

gac aaa att tcc act ttg ttt gca aag ttc ttg tct aaa caa cta gaa     1008
Asp Lys Ile Ser Thr Leu Phe Ala Lys Phe Leu Ser Lys Gln Leu Glu
                325                 330                 335

ttg gca cac cta gaa cat tct ttg aag att ggt gtt gtg caa act gcc     1056
Leu Ala His Leu Glu His Ser Leu Lys Ile Gly Val Val Gln Thr Ala
            340                 345                 350

tat gca aac ggc agt tcc acc gct tac ata aaa aat acg ttg cac tgt     1104
Tyr Ala Asn Gly Ser Ser Thr Ala Tyr Ile Lys Asn Thr Leu His Cys
        355                 360                 365

ccc gtg tct tgc act aag aca ggt gtt aaa cac ttg cat cat gaa gct     1152
Pro Val Ser Cys Thr Lys Thr Gly Val Lys His Leu His His Glu Ala
    370                 375                 380

gcc act cag tac gat att ggc att tat ttc gaa gca aat gga cat ggt     1200
Ala Thr Gln Tyr Asp Ile Gly Ile Tyr Phe Glu Ala Asn Gly His Gly
385                 390                 395                 400

acg att ata ttc agc gaa aaa ttt cat cga act atc aaa tct gaa tta     1248
Thr Ile Ile Phe Ser Glu Lys Phe His Arg Thr Ile Lys Ser Glu Leu
                405                 410                 415

tcc aag tcc aag tta aat ggt gat acg tta gct ttg aga act ttg aag     1296
Ser Lys Ser Lys Leu Asn Gly Asp Thr Leu Ala Leu Arg Thr Leu Lys

420                 425                 430

tgt ttc tct gaa ttg att aat cag acc gtg gga gat gct att tca gac      1344
Cys Phe Ser Glu Leu Ile Asn Gln Thr Val Gly Asp Ala Ile Ser Asp
        435                 440                 445

atg ctt gct gtc ctt gct act ttg gcg att ttg aaa atg tcg cca atg      1392
Met Leu Ala Val Leu Ala Thr Leu Ala Ile Leu Lys Met Ser Pro Met
    450                 455                 460

gat tgg gat gaa gag tat act gat ttg ccc aac aag ctg gtt aag tgc      1440
Asp Trp Asp Glu Glu Tyr Thr Asp Leu Pro Asn Lys Leu Val Lys Cys
465                 470                 475                 480

atc gtt cct gat agg tca att ttc caa acc acg gac cag gaa aga aaa      1488
Ile Val Pro Asp Arg Ser Ile Phe Gln Thr Thr Asp Gln Glu Arg Lys
                485                 490                 495

ttg ctc aat cca gtg ggg ttg caa gac aag ata gat ctt gtg gta gcc      1536
Leu Leu Asn Pro Val Gly Leu Gln Asp Lys Ile Asp Leu Val Val Ala
            500                 505                 510

aag tat ccc atg gga aga agc ttt gtc aga gcc agt ggt acg gag gat      1584
Lys Tyr Pro Met Gly Arg Ser Phe Val Arg Ala Ser Gly Thr Glu Asp
        515                 520                 525

gcg gtg agg gtt tat gcg gaa tgt aag gac tcc tct aag tta ggt caa      1632
Ala Val Arg Val Tyr Ala Glu Cys Lys Asp Ser Ser Lys Leu Gly Gln
    530                 535                 540

ttt tgt gac gaa gtg gtg gag cac gtt aag gca tct gct tga              1674
Phe Cys Asp Glu Val Val Glu His Val Lys Ala Ser Ala
545                 550                 555

<210>  30
<211>  557
<212>  PRT
<213>  Saccharomyces cerevisiae

<400>  30

Met Lys Val Asp Tyr Glu Gln Leu Cys Lys Leu Tyr Asp Asp Thr Cys
1               5                   10                  15

Arg Thr Lys Asn Val Gln Phe Ser Tyr Gly Thr Ala Gly Phe Arg Thr
            20                  25                  30

Leu Ala Lys Asn Leu Asp Thr Val Met Phe Ser Thr Gly Ile Leu Ala
        35                  40                  45

Val Leu Arg Ser Leu Lys Leu Gln Gly Gln Tyr Val Gly Val Met Ile
    50                  55                  60

Thr Ala Ser His Asn Pro Tyr Gln Asp Asn Gly Val Lys Ile Val Glu
65                  70                  75                  80

Pro Asp Gly Ser Met Leu Leu Ala Thr Trp Glu Pro Tyr Ala Met Gln
                85                  90                  95

Leu Ala Asn Ala Ala Ser Phe Ala Thr Asn Phe Glu Glu Phe Arg Val
            100                 105                 110

Glu Leu Ala Lys Leu Ile Glu His Glu Lys Ile Asp Leu Asn Thr Thr
        115                 120                 125

Val Val Pro His Ile Val Val Gly Arg Asp Ser Arg Glu Ser Ser Pro
    130                 135                 140

Tyr Leu Leu Arg Cys Leu Thr Ser Ser Met Ala Ser Val Phe His Ala
145                 150                 155                 160

Gln Val Leu Asp Leu Gly Cys Val Thr Thr Pro Gln Leu His Tyr Ile
                165                 170                 175

Thr Asp Leu Ser Asn Arg Arg Lys Leu Glu Gly Asp Thr Ala Pro Val
            180                 185                 190

Ala Thr Glu Gln Asp Tyr Tyr Ser Phe Phe Ile Gly Ala Phe Asn Glu
        195                 200                 205

Leu Phe Ala Thr Tyr Gln Leu Glu Lys Arg Leu Ser Val Pro Lys Leu
    210                 215                 220

Phe Ile Asp Thr Ala Asn Gly Ile Gly Gly Pro Gln Leu Lys Lys Leu
225                 230                 235                 240

Leu Ala Ser Glu Asp Trp Asp Val Pro Ala Glu Gln Val Glu Val Ile
                245                 250                 255

Asn Asp Arg Ser Asp Val Pro Glu Leu Leu Asn Phe Glu Cys Gly Ala
            260                 265                 270

Asp Tyr Val Lys Thr Asn Gln Arg Leu Pro Lys Gly Leu Ser Pro Ser
        275                 280                 285

Ser Phe Asp Ser Leu Tyr Cys Ser Phe Asp Gly Asp Ala Asp Arg Val
    290                 295                 300

Val Phe Tyr Tyr Val Asp Ser Gly Ser Lys Phe His Leu Leu Asp Gly
305                 310                 315                 320

Asp Lys Ile Ser Thr Leu Phe Ala Lys Phe Leu Ser Lys Gln Leu Glu
                325                 330                 335

Leu Ala His Leu Glu His Ser Leu Lys Ile Gly Val Val Gln Thr Ala
            340                 345                 350

Tyr Ala Asn Gly Ser Ser Thr Ala Tyr Ile Lys Asn Thr Leu His Cys
        355                 360                 365

Pro Val Ser Cys Thr Lys Thr Gly Val Lys His Leu His His Glu Ala
    370                 375                 380

Ala Thr Gln Tyr Asp Ile Gly Ile Tyr Phe Glu Ala Asn Gly His Gly
385                 390                 395                 400

Thr Ile Ile Phe Ser Glu Lys Phe His Arg Thr Ile Lys Ser Glu Leu
                405                 410                 415

Ser Lys Ser Lys Leu Asn Gly Asp Thr Leu Ala Leu Arg Thr Leu Lys

420                 425                 430

Cys Phe Ser Glu Leu Ile Asn Gln Thr Val Gly Asp Ala Ile Ser Asp
        435                 440                 445

Met Leu Ala Val Leu Ala Thr Leu Ala Ile Leu Lys Met Ser Pro Met
    450                 455                 460

Asp Trp Asp Glu Glu Tyr Thr Asp Leu Pro Asn Lys Leu Val Lys Cys
465                 470                 475                 480

Ile Val Pro Asp Arg Ser Ile Phe Gln Thr Thr Asp Gln Glu Arg Lys
                485                 490                 495

Leu Leu Asn Pro Val Gly Leu Gln Asp Lys Ile Asp Leu Val Val Ala
            500                 505                 510

Lys Tyr Pro Met Gly Arg Ser Phe Val Arg Ala Ser Gly Thr Glu Asp
        515                 520                 525

Ala Val Arg Val Tyr Ala Glu Cys Lys Asp Ser Ser Lys Leu Gly Gln
    530                 535                 540

Phe Cys Asp Glu Val Val Glu His Val Lys Ala Ser Ala
545                 550                 555